# EUROPEAN PATENT APPLICATION

(11) **EP 3 582 279 A1**
(43) Date of publication of application: **18.12.2019**
(21) Application number: 18751071.4
(22) Date of filing: 05.02.2018
(51) Int. Cl.: H01L 51/50, C07C 381/12, C07D 333/76, C07D 335/02, C07F 5/02, H05B 33/10

(54) **CHARGE TRANSPORTING VARNISH**

(30) Priority: 07.02.2017 JP 2017020436; 06.09.2017 JP 2017170729
(71) Applicant: Nissan Chemical Corporation, Tokyo 103-6119 (JP)
(72) Inventor: NAKAIE Naoki, Funabashi-shi Chiba 274-0052 (JP); MAEDA Daisuke, Funabashi-shi Chiba 274-0052 (JP)
(74) Representative: Mewburn Ellis LLP
(86) International application number: PCT/JP2018/003722
(87) International publication number: WO 2018/147204

(57) **Abstract**

Provided is a charge transporting varnish comprising: a charge transporting substance such as an aniline derivative and a thiophene derivative; an onium borate salt such as a compound represented by the formula; and an organic solvent.

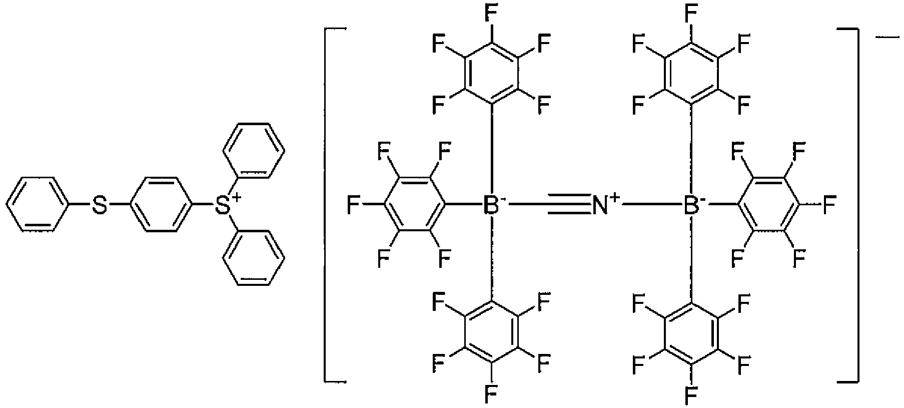

## Description

### TECHNICAL FIELD

The present invention relates to a charge-transporting varnish.

### BACKGROUND ART

In an organic electroluminescent (hereinafter, referred to as organic EL) device, a charge-transporting thin film made of an organic compound is used as a light-emitting layer and a charge injection layer. Particularly, a hole injection layer is responsible for a transfer of charges between a positive electrode and a hole injection layer or a light-emitting layer, and has an important function for achieving low-voltage driving and high brightness of an organic EL device.

A method of forming the hole injection layer is largely divided into a dry process represented by a vapor deposition method and a wet process represented by a spin coating method, and when these processes are compared, a thin film having a large area and high flatness can be efficiently produced by the method of the wet process. Therefore, as the area of the organic EL display is increased, a hole injection layer which can be formed by the wet process is currently desired.

In view of such circumstances, the present inventors have developed charge-transporting materials which are applicable to various wet processes and also if applied to a hole injection layer of an organic EL device, provide a thin film capable of realizing excellent EL device characteristics, and a compound having a good solubility in an organic solvent to be used (see for example, Patent Documents 1 to 4).

However, improvements have always been demanded for the wet process materials for the hole injection layer, and in particular, wet process materials which provide a thin film having excellent charge transportability have been demanded.

### PRIOR ART DOCUMENTS

### PATENT DOCUMENTS

Patent Document 1: WO 2008/032616
Patent Document 2: WO 2008/129947
Patent Document 3: WO 2006/025342
Patent Document 4: WO 2010/058777
Patent Document 5: JP-A 2014-205624
Patent Document 6: JP-A 2005-314682
Patent Document 7: JP-A 2006-233162
Patent Document 8: JP-A 2011-026325

### SUMMARY OF INVENTION

### TECHNICAL PROBLEM

An object of the invention, which has been made under the above circumstances, is to provide a charge-transporting varnish which provides a charge-transporting thin film having excellent charge transportability, flatness, and uniformity with good reproducibility, and a compound which is a material of the charge-transporting varnish.

### SOLUTION TO PROBLEM

As a result of making extensive investigations to attain the above object, the present inventors have found that a charge-transporting thin film having excellent charge transportability, flatness, and uniformity can be obtained with good reproducibility from a charge-transporting material and a varnish obtained by dissolving a predetermined onium borate salt in an organic solvent, and an organic EL device having an excellent brightness characteristic can be obtained by using the thin film as a hole injection layer, and completed the present invention.

That is, the present invention provides the following:
1. A charge-transporting varnish including: a charge-transporting material, an onium borate salt, and an organic solvent,
   wherein the onium borate salt includes an onium borate salt including a monovalent or divalent anion represented by formula (a1) and a counter cation represented by formulae (c1) to (c5) (provided that the salt is an electrically neutral salt): 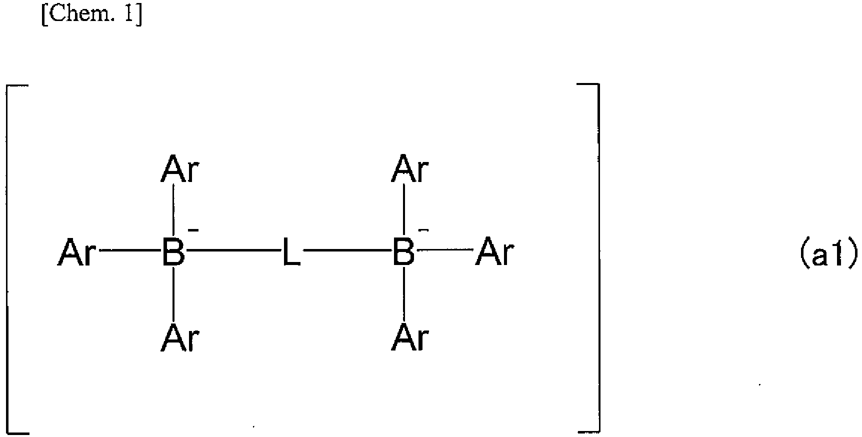 wherein Ar's, independently of each other, represent an aryl group which may have a substituent or a heteroaryl group which may have a substituent, and L is an alkylene group, -NH-, an oxygen atom, a sulfur atom, or -CN⁺, 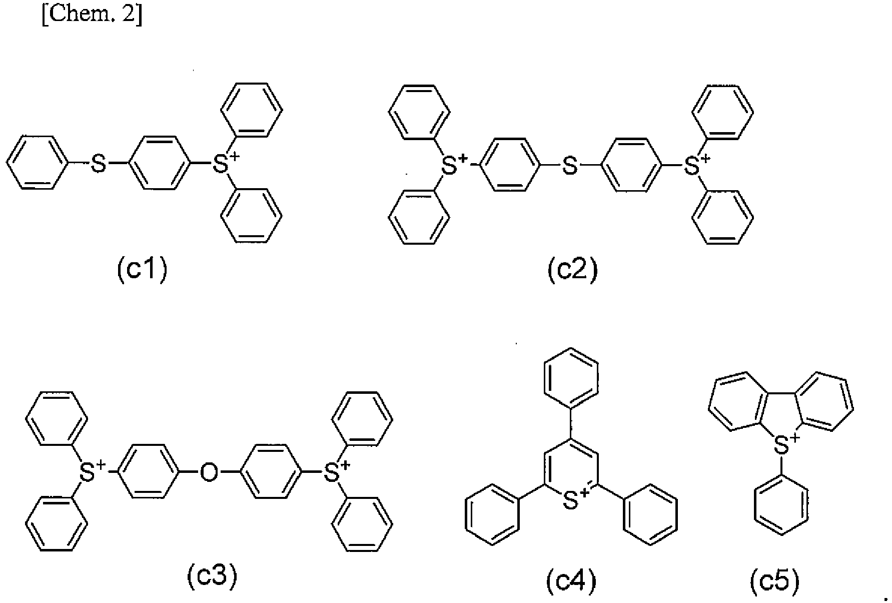
2. The charge-transporting varnish of 1, wherein Ar is an aryl group having one or two or more electron-withdrawing substituents.
3. The charge-transporting varnish of 2, wherein the electron-withdrawing substituent is a halogen atom.
4. The charge-transporting varnish of any one of 1 to 3, wherein the anion is represented by formula (a2): 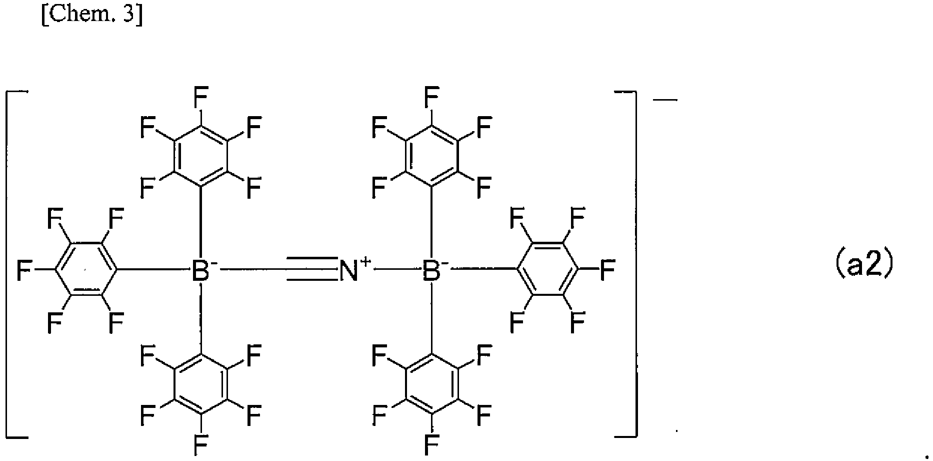
5. The charge-transporting varnish of 4, wherein the onium borate salt is represented by the following formula: 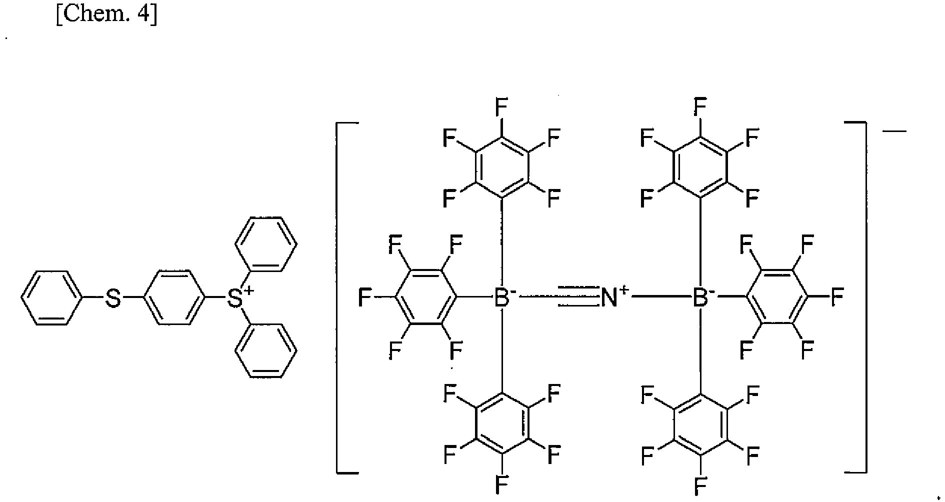
6. The charge-transporting varnish of any one of 1 to 5, wherein the charge-transporting material is at least one selected from an aniline derivative and a thiophene derivative.
7. The charge-transporting varnish of 6, wherein the charge-transporting material is the aniline derivative.
8. A charge-transporting thin film, obtained from the charge-transporting varnish of any one of 1 to 7.
9. An organic electroluminescent device, including the charge-transporting thin film of 8.
10. A method of producing a charge-transporting thin film, including applying the charge-transporting varnish of any one of 1 to 7 on a substrate, and evaporating a solvent.
11. An onium borate salt, included in any one of a hole injection layer, a hole transport layer, and a hole injection/transport layer in an organic electroluminescent device, the onium borate salt including a monovalent or divalent anion represented by formula (a1) and a counter cation represented by formulae (c1) to (c5) (provided that the salt is an electrically neutral salt): 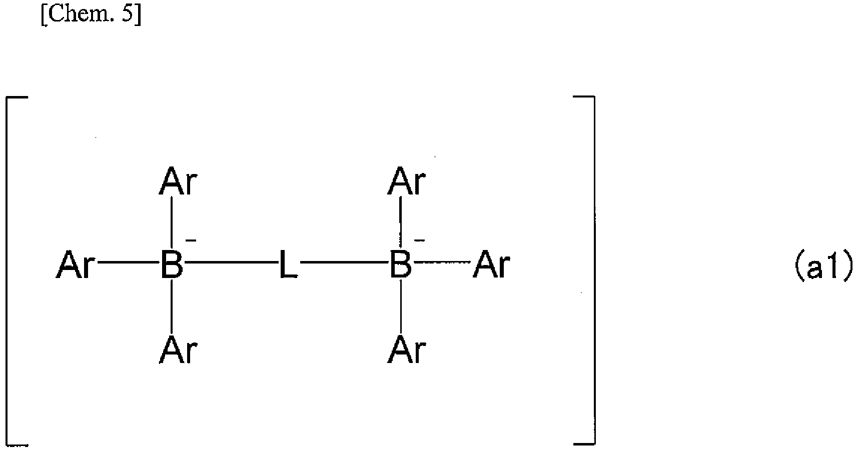 wherein Ar's, independently of each other, represent an aryl group which may have a substituent or a heteroaryl group which may have a substituent, and L is an alkylene group, -NH-, an oxygen atom, a sulfur atom, or -CN⁺, 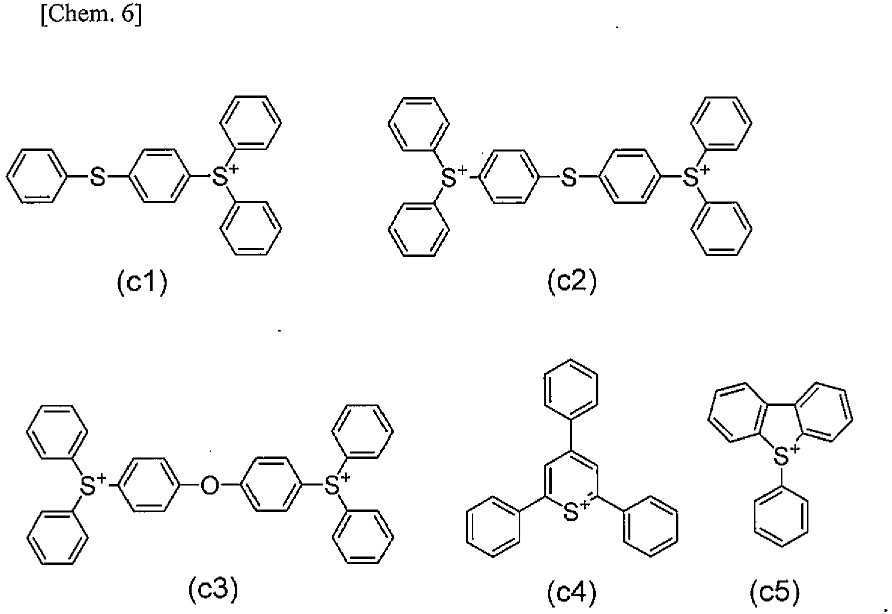
12. The onium borate salt of 11, wherein Ar is an aryl group having one or two or more electron-withdrawing substituents.
13. The onium borate salt of 12, wherein the electron-withdrawing substituent is a halogen atom.
14. The onium borate salt of any one of 11 to 13, wherein the anion is represented by formula (a2): 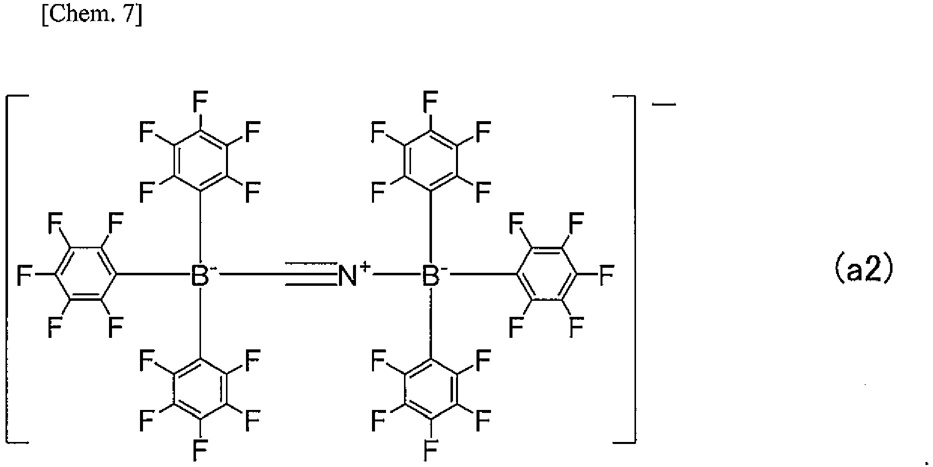
15. The onium borate salt of 14, wherein the onium borate salt is represented by the following formula:

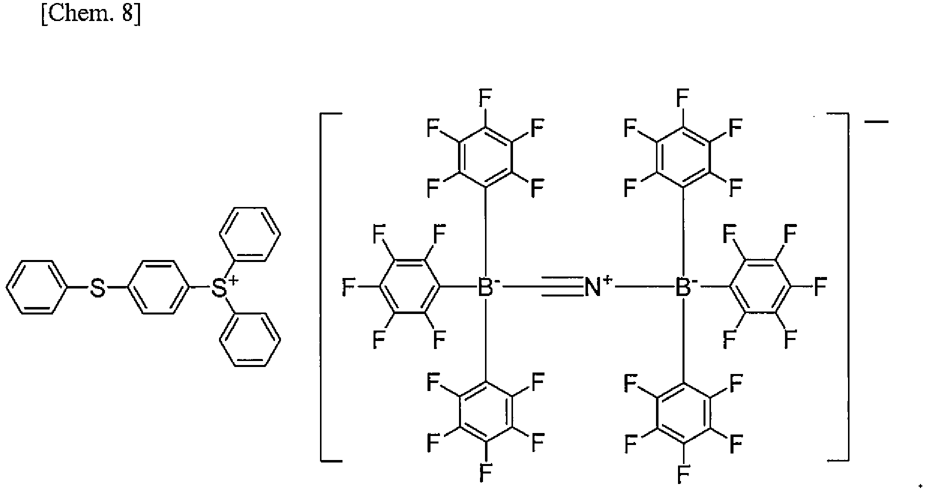

### ADVANTAGEOUS EFFECTS OF INVENTION

By using the charge-transporting varnish of the present invention, a charge-transporting thin film having excellent charge transportability, flatness, and uniformity can be obtained.

Further, the charge-transporting thin film having such characteristics can be suitably used as a thin film for an electronic device including an organic EL device. In particular, by applying the thin film to a hole injection layer of an organic EL device, an organic EL device with a low driving voltage can be obtained.

In addition, the charge-transporting varnish of the present invention can produce a thin film having excellent charge transportability with good reproducibility even when various wet processes allowing film formation on a large area, such as a spin coating method or a slit coating method, are used, and thus, can sufficiently respond to even the recent progress in the field of organic EL devices.

Further, since the thin film obtained from the charge-transporting varnish of the present invention has excellent charge transportability, and thus, it is expected to use the thin film as an antistatic film, a positive electrode buffer layer of an organic thin film solar cell, and the like.

### DESCRIPTION OF EMBODIMENTS

Hereinafter, the present invention is described in detail.

The charge-transporting varnish according to the present invention includes a charge-transporting material, an onium borate salt, and an organic solvent, wherein the onium borate salt includes an onium borate salt including a monovalent or divalent anion represented by formula (a1) and a counter cation represented by formulae (c1) to (c5) (provided that the salt is an electrically neutral salt).

In addition, charge transportability is synonymous with conductivity and is also synonymous with hole transportability. Further, the charge-transporting varnish of the present invention may have charge transportability by itself or may have charge transportability in a solid film obtained by using the varnish.

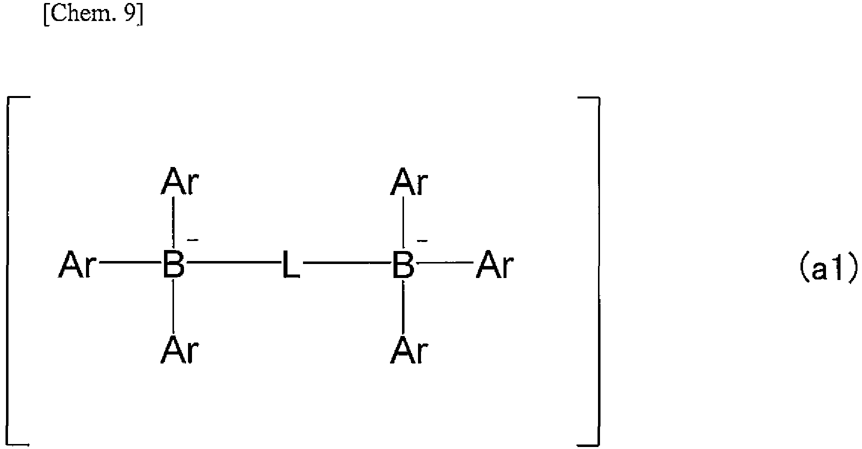

In the above formula, in the above formula, Ar's, independently of each other, represent an aryl group which may have a substituent or a heteroaryl group which may have a substituent, and L represents an alkylene group, -NH-, an oxygen atom, a sulfur atom, or -CN⁺-.

Examples of the aryl group include an aryl group having 6 to 20 carbon atoms and the like. More specific examples thereof include a phenyl group, a tolyl group, a 1-naphthyl group, a 2-naphthyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group, and the like, and a phenyl group, a tolyl group, and a naphthyl group are preferred.

Examples of the substituent include a halogen atom, a nitro group, a cyano group, an alkyl group having 1 to 20 carbon atoms, an alkenyl group having 2 to 20 carbon atoms, an alkynyl group having 2 to 20 carbon atoms, and the like.

Examples of the halogen atom include a fluorine atom, a chlorine atom, a bromine atom, an iodine atom, and the like, and a fluorine atom is preferred.

The alkyl group having 1 to 20 carbon atoms may be linear, branched, or cyclic, and specific examples thereof include a methyl group, an ethyl group, an n-propyl group, an isopropyl group, an n-butyl group, an isobutyl group, a s-butyl group, a t-butyl group, an n-pentyl group, an n-hexyl group, an n-heptyl group, an n-octyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, an n-nonadecyl group, an n-eicosanyl group, and the like, and an alkyl group having 1 to 18 carbon atoms is preferred and an alkyl group having 1 to 8 carbon atoms is more preferred.

Specific examples of the alkenyl group having 2 to 20 carbon atoms include an ethenyl group, an n-1-propenyl group, an n-2-propenyl group, a 1-methylethenyl group, an n-1-butenyl group, an n-2-butenyl group, an n-3-butenyl group, a 2-methyl-1-propenyl group, a 2-methyl-2-propenyl group, a 1-ethylethenyl group, a 1-methyl-1-propenyl group, a 1-methyl-2-propenyl group, an n-1-pentenyl group, an n-1-decenyl group, an n-1-eicosenyl group, and the like.

Specific examples of the alkynyl group having 2 to 20 carbon atoms include an ethynyl group, an n-1-propynyl group, an n-2-propynyl group, an n-1-butynyl group, an n-2-butynyl group, an n-3-butynyl group, a 1-methyl-2-propynyl group, an n-1-pentynyl group, an n-2-pentynyl group, an n-3-pentynyl group, an n-4-pentynyl group, a 1-methyl-n-butynyl group, a 2-methyl-n-butynyl group, a 3-methyl-n-butynyl group, a 1,1-dimethyl-n-propynyl group, an n-1-hexynyl group, an n-1-decynyl group, an n-1-pentadecynyl group, an n-1-eicosynyl group, and the like.

Further, it is preferred that the aryl group has one or two or more electron-withdrawing groups, among the above-described substituents. Examples of the electron-withdrawing group include a halogen atom, a nitro group, a cyano group, and the like, and a halogen atom is preferred, and a fluorine atom is particularly preferred.

Examples of the heteroaryl group include a heteroaryl group having preferably 2 to 20 carbon atoms. Specific examples thereof include an oxygen-containing heteroaryl group such as a 2-thienyl group, a 3-thienyl group, a 2-furanyl group, a 3-furanyl group, a 2-oxazolyl group, a 4-oxazolyl group, a 5-oxazolyl group, a 3-isoxazolyl group, a 4-isoxazolyl group, and a 5-isoxazolyl group; a sulfur-containing heteroaryl group such as a 2-thiazolyl group, a 4-thiazolyl group, a 5-thiazolyl group, a 3-isothiazolyl group, a 4-isothiazolyl group, and a 5-isothiazolyl group; a nitrogen-containing heteroaryl group such as a 2-imidazolyl group, a 4-imidazolyl group, a 2-pyridyl group, a 3-pyridyl group, a 4-pyridyl group, a 2-pyrazyl group, a 3-pyrazyl group, a 5-pyrazyl group, a 6-pyrazyl group, a 2-pyrimidyl group, a 4-pyrimidyl group, a 5-pyrimidyl group, a 6-pyrimidyl group, a 3-pyridazyl group, a 4-pyridazyl group, a 5-pyridazyl group, a 6-pyridazyl group, a 1,2,3-triazin-4-yl group, a 1,2,3-triazin-5-yl group, a 1,2,4-triazin-3-yl group, a 1,2,4-triazin-5-yl group, a 1,2,4-triazin-6-yl group, a 1,3,5-triazin-2-yl group, a 1,2,4,5-tetrazin-3-yl group, a 1,2,3,4-tetrazin-5-yl group, a 2-quinolinyl group, a 3-quinolinyl group, a 4-quinolinyl group, a 5-quinolinyl group, a 6-quinolinyl group, a 7-quinolinyl group, a 8-quinolinyl group, a 1-isoquinolinyl group, a 3-isoquinolinyl group, a 4-isoquinolinyl group, a 5-isoquinolinyl group, a 6-isoquinolinyl group, a 7-isoquinolinyl group, a 8-isoquinolinyl group, a 2-quinoxanyl group, a 5-quinoxanyl group, a 6-quinoxanyl group, a 2-quinazolinyl group, a 4-quinazolinyl group, a 5-quinazolinyl group, a 6-quinazolinyl group, a 7-quinazolinyl group, a 8-quinazolinyl group, a 3-cinnolinyl group, a 4-cinnolinyl group, a 5-cinnolinyl group, a 6-cinnolinyl group, a 7-cinnolinyl group, and a 8-cinnolinyl group; and the like.

Examples of the substituent of the heteroaryl group include the substituents as exemplified for the aryl group.

L represents an alkylene group, -NH-, an oxygen atom, a sulfur atom, or -CN⁺-, but -CN⁺- is preferred.

The alkylene group may be linear, branched, or cyclic, and examples thereof include an alkylene group having 1 to 20 carbon atoms, and preferably 1 to 10 carbon atoms. Specific examples thereof include a methylene group, a methyl methylene group, a dimethyl methylene group, an ethylene group, a trimethylene group, a propylene group, a tetramethylene group, a pentamethylene group, a hexamethylene group, and the like.

Examples of the anion of the above formula (a1) which can be suitably used in the present invention include the anion represented by formula (a2), but are not limited thereto.

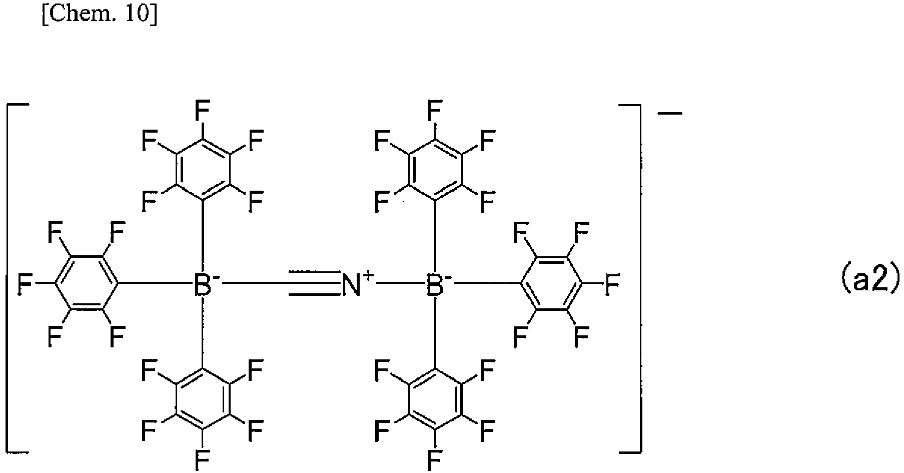

Meanwhile, examples of the counter cation include those represented by formulae (c1) to (c5).

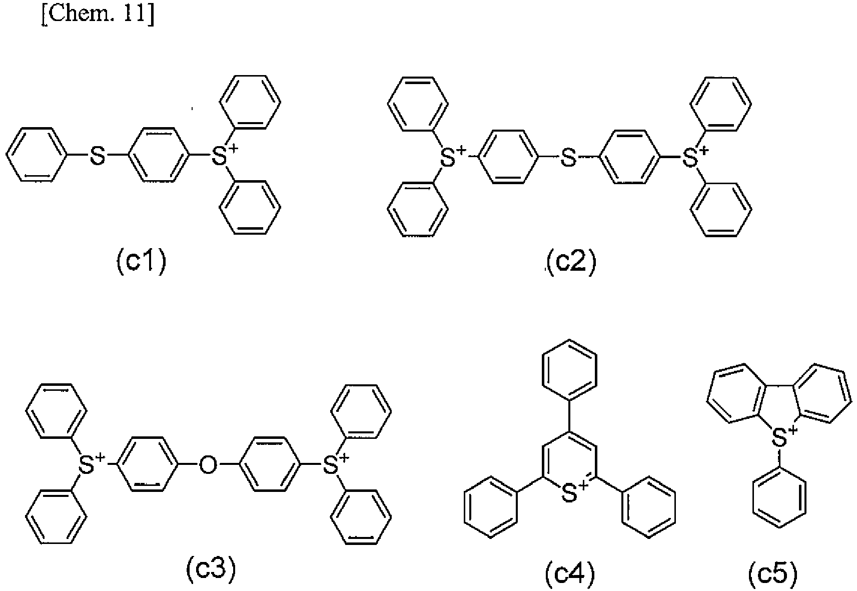

In the present invention, the onium borate salt may be used alone or in combination of two or more.

Further, if necessary, other known onium borate salts may be used in combination.

In addition, the onium borate salt can be synthesized, for example, with reference to the known method described in JP-A 2005-314682, or the like.

The onium borate salt may be previously dissolved in an organic solvent, for facilitating dissolution in the charge-transporting varnish.

Examples of such an organic solvent include carbonates such as propylene carbonate, ethylene carbonate, 1,2-butylene carbonate, dimethyl carbonate, and diethyl carbonate; ketones such as acetone, methyl ethyl ketone, cyclohexanone, methyl isoamyl ketone, and 2-heptanone; polyvalent alcohols and derivatives thereof such as monomethyl ether, monoethyl ether, monopropyl ether, monobutyl ether, or monophenyl ether of ethylene glycol, ethylene glycol monoacetate, diethylene glycol, diethylene glycol monoacetate, propylene glycol, propylene glycol monoacetate, dipropylene glycol, or dipropylene glycol monoacetate; cyclic ethers such as dioxane; esters such as ethyl formate, methyl lactate, ethyl lactate, methyl acetate, ethyl acetate, butyl acetate, methyl pyruvate, methyl acetoacetate, ethyl acetoacetate, ethyl pyruvate, ethyl ethoxyacetate, methyl methoxypropionate, ethyl ethoxypropionate, methyl 2-hydroxypropionate, ethyl 2-hydroxypropionate, ethyl 2-hydroxy-2-methylpropionate, methyl 2-hydroxy-3-methylbutanate, 3-methoxybutyl acetate, and 3-methyl-3-methoxybutyl acetate; aromatic hydrocarbons such as toluene, xylene, 3-phenoxytolulene, 4-methoxytoluene, methyl benzoate, cyclohexyl benzene, tetraline, and isophorone; and the like, and these solvents may be used alone or in combination of two or more.

If the organic solvent is used, the use ratio is preferably 15 to 1,000 parts by weight, and more preferably 30 to 500 parts by weight, per 100 parts by weight of the onium borate salt.

The charge-transporting material used in the present invention is not particularly limited, but can be suitably selected from those conventionally known in the fields of organic EL and the like and used.

Specific examples thereof include various hole-transporting materials, for example, arylamine derivatives such as an oligoaniline derivative, an N,N'-diarylbenzidine derivative, and an N,N,N',N'-tetraaryl benzidine derivative; thiophene derivatives such as an oligothiophene derivative, a thienothiophene derivative, and a thienobenzothiophene derivative; pyrrole derivatives such as oligopyrrole, and among them, an arylamine derivative and a thiophene derivative are preferred, an arylamine derivative is more preferred, and the aniline derivative represented by formula (1) or (2) is still more preferred.

Further, a molecular weight of the charge-transporting material is not particularly limited, but from a viewpoint of preparing a uniform varnish providing a thin film having high flatness, the molecular weight is preferably 200 to 9,000, from a viewpoint of obtaining charge transportability with high solvent resistance, the molecular weight is more preferably 300 or more, still more preferably 400 or more, and from a viewpoint of preparing a uniform varnish providing a thin film having high flatness with good reproducibility, the molecular weight is more preferably 8,000 or less, still more preferably 7,000 or less, further preferably 6,000 or less, and most preferably 5,000 or less.

In addition, from a viewpoint of preventing the charge-transporting material from being separated when formed into a thin film, it is preferred that the charge-transporting material has no molecular weight distribution (dispersion degree 1) (that is, a single molecular weight is preferred).

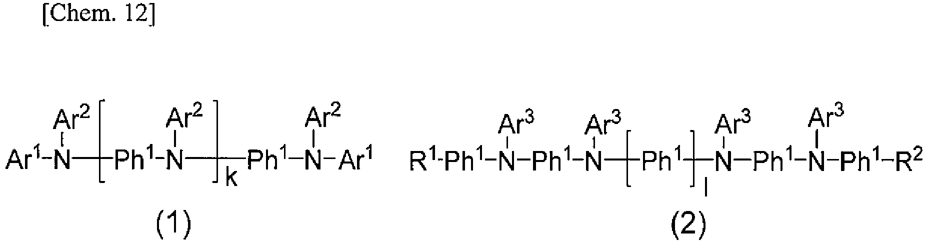

In the above formula (2), R¹ and R², independently of each other, represent a hydrogen atom, a halogen atom, a nitro group, a cyano group, or an alkyl group having 1 to 20 carbon atoms, an alkenyl group having 2 to 20 carbon atoms, an alkynyl group having 2 to 20 carbon atoms, an aryl group having 6 to 20 carbon atoms, or a heteroaryl group having 2 to 20 carbon atoms, which may be substituted by a halogen atom, and specific examples thereof include those as described for the above formula (c1).

Among them, R¹ and R² are preferably a hydrogen atom, a fluorine atom, a cyano group, an alkyl group having 1 to 20 carbon atoms which may be substituted by a halogen atom, an aryl group having 6 to 20 carbon atoms which may be substituted by a halogen atom, or a heteroaryl group having 2 to 20 carbon atoms which may be substituted by a halogen atom, more preferably a hydrogen atom, a fluorine atom, a cyano group, an alkyl group having 1 to 10 carbon atoms which may be substituted by a halogen atom, or a phenyl group which may be substituted by a halogen atom, still more preferably a hydrogen atom, a fluorine atom, a methyl group, or a trifluoromethyl group, and most preferably a hydrogen atom.

Ph¹ in the above formulae (1) and (2) represents a group represented by formula (P1).

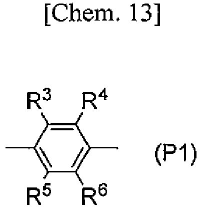

Wherein R³ to R⁶, independently of one another, represent a hydrogen atom, a halogen atom, a nitro group, a cyano group, or an alkyl group having 1 to 20 carbon atoms, an alkenyl group having 2 to 20 carbon atoms, an alkynyl group having 2 to 20 carbon atoms, an aryl group having 6 to 20 carbon atoms, or a heteroaryl group having 2 to 20 carbon atoms, which may be substituted by halogen atom, and specific examples thereof include those as described for the above formula (c1).

In particular, R³ to R⁶ are preferably a hydrogen atom, a fluorine atom, a cyano group, an alkyl group having 1 to 20 carbon atoms which may be substituted by a halogen atom, an aryl group having 6 to 20 carbon atoms which may be substituted by a halogen atom, or a heteroaryl group having 2 to 20 carbon atoms which may be substituted by a halogen atom, more preferably a hydrogen atom, a fluorine atom, a cyano group, an alkyl group having 1 to 10 carbon atoms which may be substituted by a halogen atom, or a phenyl group which may be substituted by a halogen atom, still more preferably a hydrogen atom, a fluorine atom, a methyl group, or a trifluoromethyl group, and most preferably a hydrogen atom.

Hereinafter, specific examples of the group suitable as Ph¹ include the following, but are not necessarily limited thereto.

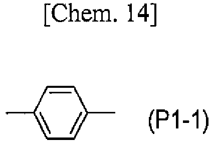

In the above formula (1), Ar¹'s, independently of each other, represent a group represented by any one of formulae (B1) to (B11), and in particular, a group represented by any one of formulae (B1') to (B11') is preferred.

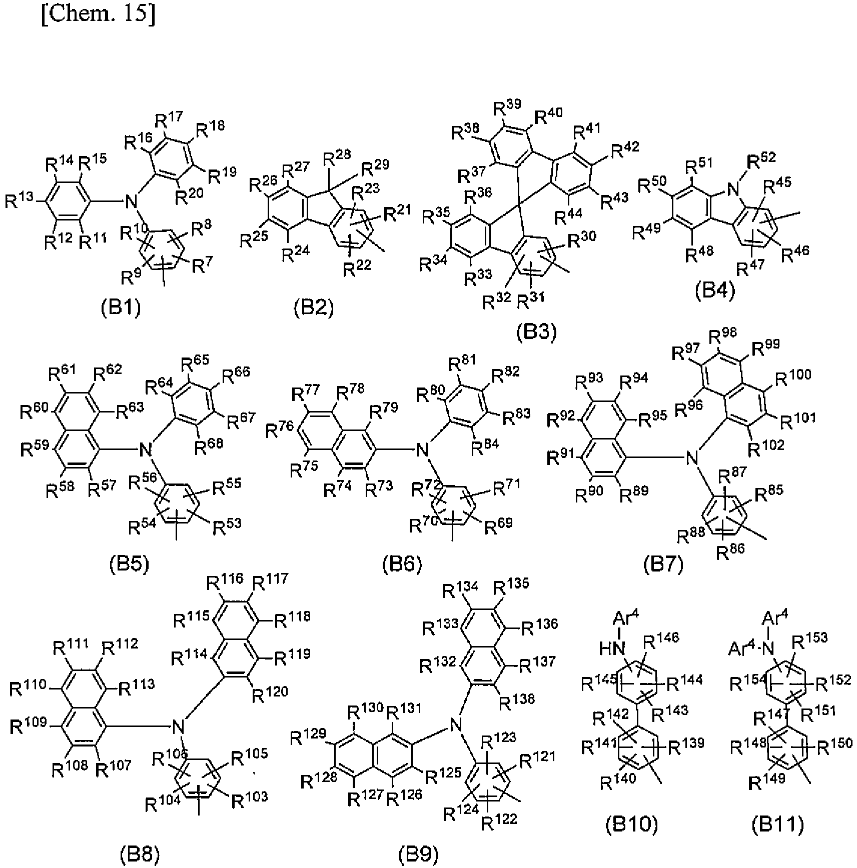

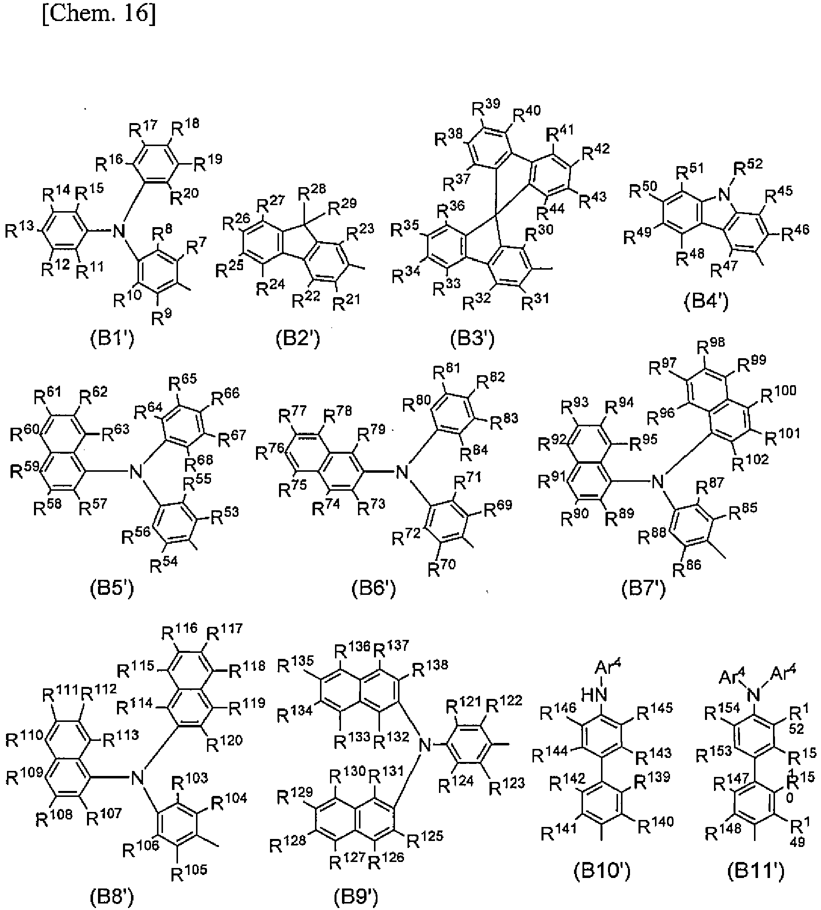

Wherein R⁷ to R²⁷, R³⁰ to R⁵¹, and R⁵³ to R¹⁵⁴, independently of one another, represent a hydrogen atom, a halogen atom, a nitro group, a cyano group, or a diphenylamino group, an alkyl group having 1 to 20 carbon atoms, an alkenyl group having 2 to 20 carbon atoms, an alkynyl group having 2 to 20 carbon atoms, an aryl group having 6 to 20 carbon atoms, or a heteroaryl group having 2 to 20 carbon atoms, which may be substituted by a halogen atom, R²⁸ and R²⁹, independently of each other, represent an aryl group having 6 to 20 carbon atoms or a heteroaryl group having 2 to 20 carbon atoms, which may be substituted by Z¹; R⁵² represents a hydrogen atom, or an alkyl group having 1 to 20 carbon atoms, an alkenyl group having 2 to 20 carbon atoms, or an alkynyl group having 2 to 20 carbon atoms, which may be substituted by Z⁴, or an aryl group having 6 to 20 carbon atoms or a heteroaryl group having 2 to 20 carbon atoms, which may be substituted by Z¹; Z¹ represents a halogen atom, a nitro group, a cyano group, or an alkyl group having 1 to 20 carbon atoms, an alkenyl group having 2 to 20 carbon atoms, or an alkynyl group having 2 to 20 carbon atoms, which may be substituted by Z²; Z² represents a halogen atom, a nitro group, a cyano group, or an aryl group having 6 to 20 carbon atoms or a heteroaryl group having 2 to 20 carbon atoms, which may be substituted by Z³; Z³ represents a halogen atom, a nitro group, or a cyano group; Z⁴ represents a halogen atom, a nitro group, a cyano group, or an aryl group having 6 to 20 carbon atoms or a heteroaryl group having 2 to 20 carbon atoms, which may be substituted by Z⁵; Z⁵ represents a halogen atom, a nitro group, a cyano group, or an alkyl group having 1 to 20 carbon atoms, an alkenyl group having 2 to 20 carbon atoms, or an alkynyl group having 2 to 20 carbon atoms, which may be substituted by Z³; and specific examples of these halogen atom, alkyl group having 1 to 20 carbon atoms, alkenyl group having 2 to 20 carbon atoms, alkynyl group having 2 to 20 carbon atoms, aryl group having 6 to 20 carbon atoms, and heteroaryl group having 2 to 20 carbon atoms include those as described in the above formula (a1).

In particular, R⁷ to R²⁷, R³⁰ to R⁵¹, and R⁵³ to R¹⁵⁴ are preferably a hydrogen atom, a fluorine atom, a cyano group, a diphenylamino group which may be substituted by a halogen atom, an alkyl group having 1 to 20 carbon atoms which may be substituted by a halogen atom, an aryl group having 6 to 20 carbon atoms which may be substituted by a halogen atom, or a heteroaryl group having 2 to 20 carbon atoms which may be substituted by a halogen atom, more preferably a hydrogen atom, a fluorine atom, a cyano group, an alkyl group having 1 to 10 carbon atoms which may be substituted by a halogen atom, or a phenyl group which may be substituted by a halogen atom, still more preferably a hydrogen atom, a fluorine atom, a methyl group, or a trifluoromethyl group, and most preferably a hydrogen atom.

In addition, R²⁸ and R²⁹ are preferably an aryl group having 6 to 14 carbon atoms which may be substituted by Z¹ or a heteroaryl group having 2 to 14 carbon atoms which may be substituted by Z¹, more preferably an aryl group having 6 to 14 carbon atoms which may be substituted by Z¹, and still more preferably a phenyl group which may be substituted by Z¹, a 1-naphthyl group which may be substituted by Z¹, or a 2-naphthyl group which may be substituted by Z¹.

In addition, R⁵² is preferably a hydrogen atom, an aryl group having 6 to 20 carbon atoms which may be substituted by Z¹, a heteroaryl group having 2 to 20 carbon atoms which may be substituted by Z¹, or an alkyl group having 1 to 20 carbon atoms which may be substituted by Z⁴, more preferably a hydrogen atom, an aryl group having 6 to 14 carbon atoms which may be substituted by Z¹, a heteroaryl group having 2 to 14 carbon atoms which may be substituted by Z¹, or an alkyl group having 1 to 10 carbon atoms which may be substituted by Z⁴, still more preferably a hydrogen atom, an aryl group having 6 to 14 carbon atoms which may be substituted by Z¹, a heteroaryl group having 2 to 14 carbon atoms which may be substituted by Z¹, or an alkyl group having 1 to 10 carbon atoms which may be substituted by Z⁴, and further preferably a hydrogen atom, a phenyl group which may be substituted by Z¹, a 1-naphthyl group which may be substituted by Z¹, a 2-naphthyl group which may be substituted by Z¹, a 2-pyridyl group which may be substituted by Z¹, a 3-pyridyl group which may be substituted by Z¹, a 4-pyridyl group which may be substituted by Z¹, or a methyl group which may be substituted by Z⁴,

In addition, Ar⁴'s, independently of each other, represent an aryl group having 6 to 20 carbon atoms which may be substituted by a arylamino group having 6 to 20 carbon atoms.

Specific examples of the aryl group having 6 to 20 carbon atoms and the arylamino group having 6 to 20 carbon atoms include those as described in formula (c1).

Ar⁴ is preferably a phenyl group, a 1-naphthyl group, a 2-naphthyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group, a p-(diphenylamino)phenyl group, a p-(1-naphthylphenylamino)phenyl group, a p-(di(1-naphthyl)amino)phenyl group, a p-(1-naphthyl-2-naphthylamino)phenyl group, a p-(di(2-naphthyl)amino)phenyl group, and more preferably a p-(diphenylamino)phenyl group.

Hereinafter, specific examples of the group suitable as Ar¹ include the following, but are not necessarily limited thereto. 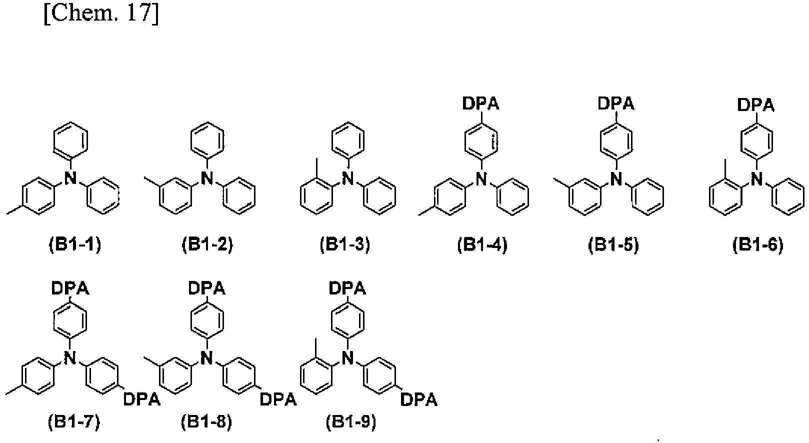 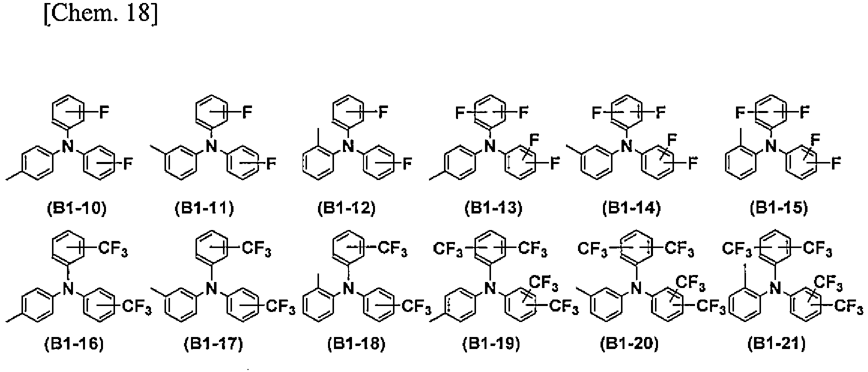 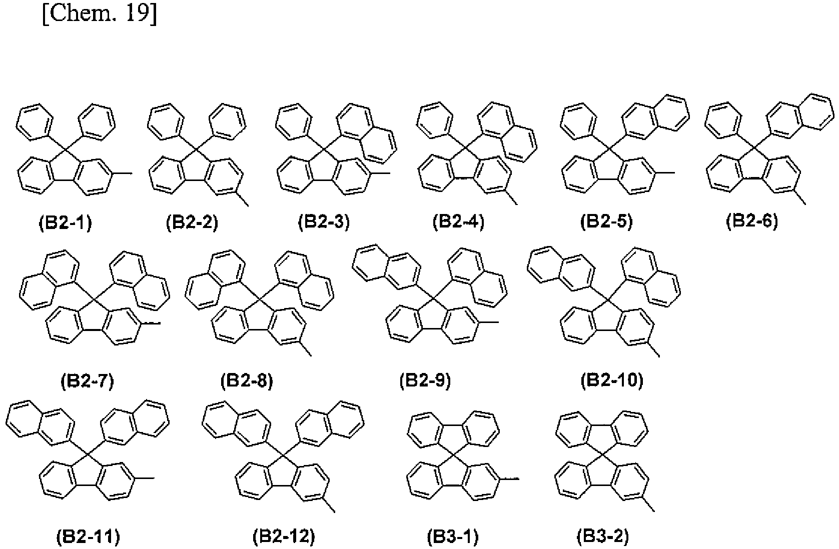 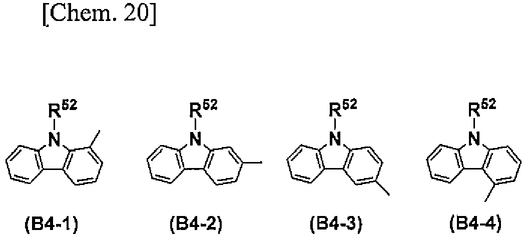 wherein R⁵² represents the same meaning as described above.

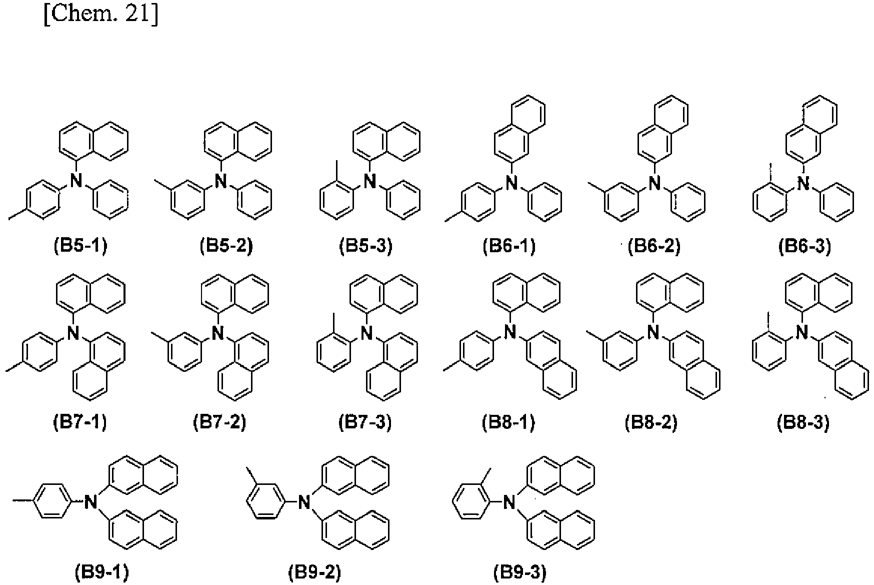

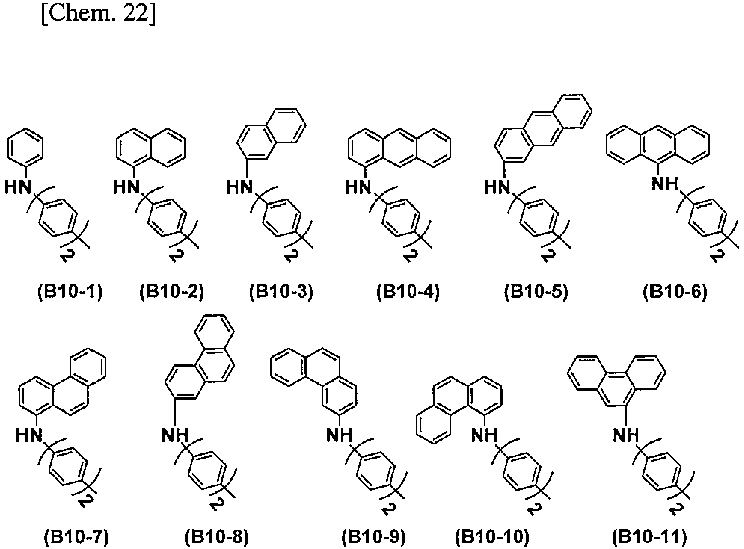

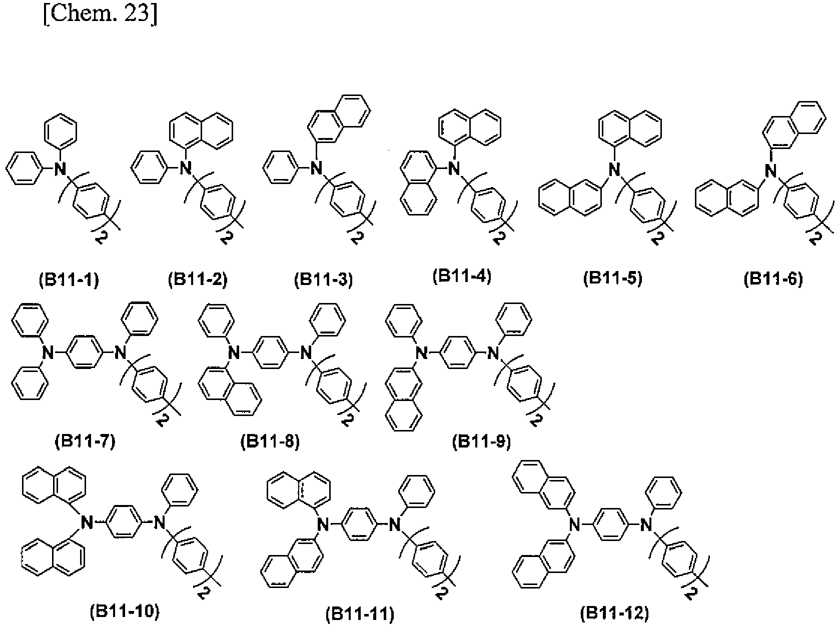

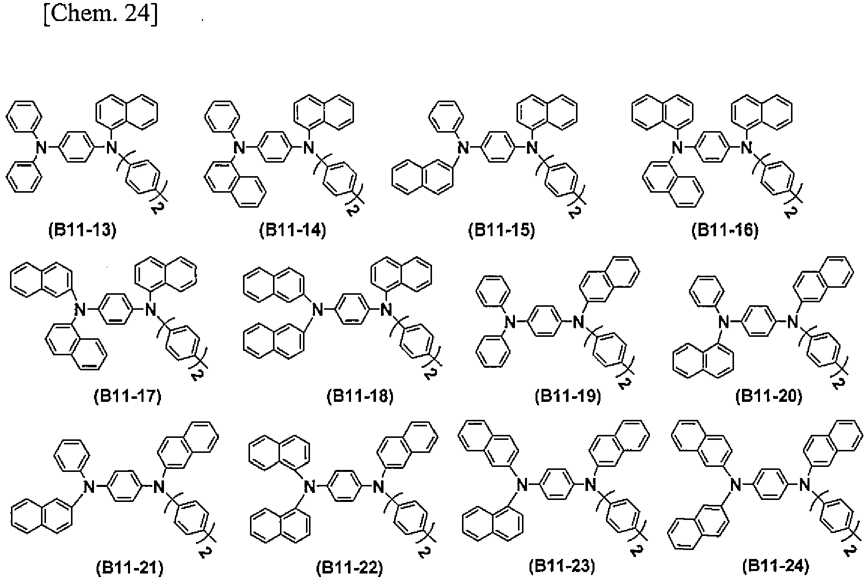

In the above formula (1), Ar²'s, independently of each other, represent a group represented by any one of formulae (A1) to (A18).

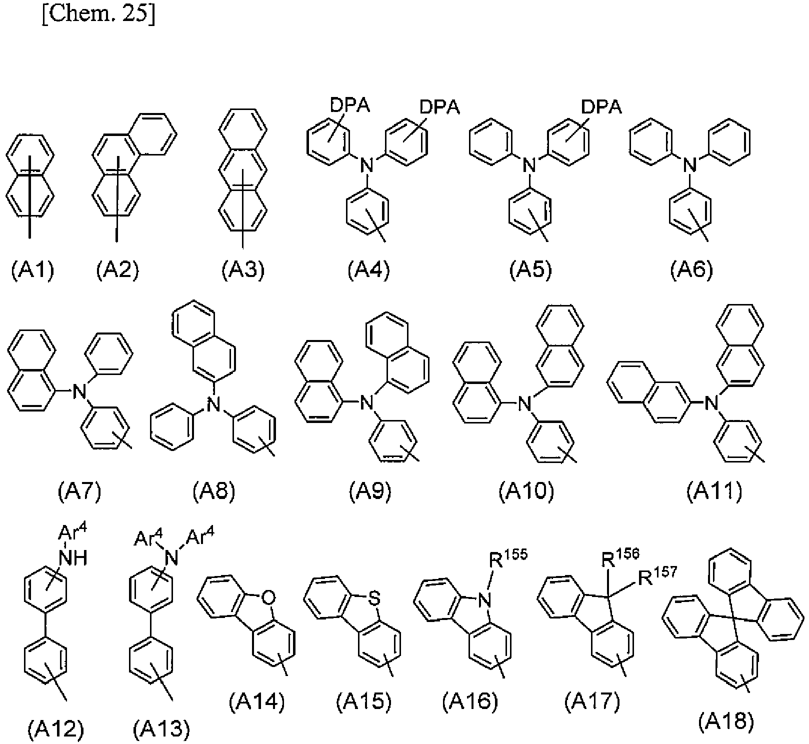

Here, in the formula, R¹⁵⁵ represents a hydrogen atom, or an alkyl group having 1 to 20 carbon atoms, an alkenyl group having 2 to 20 carbon atoms, or an alkynyl group having 2 to 20 carbon atoms, which may be substituted by Z⁴, or an aryl group having 6 to 20 carbon atoms or a heteroaryl group having 2 to 20 carbon atoms, which may be substituted by Z¹, R¹⁵⁶ and R¹⁵⁷, independently of each other, represent an aryl group having 6 to 20 carbon atoms or a heteroaryl group having 2 to 20 carbon atoms which may be substituted by Z¹, DPA represents a diphenylamino group, and Ar⁴, Z¹, and Z⁴ represent the same meaning as described above. Specific examples of these halogen atom, alkyl group having 1 to 20 carbon atoms, alkenyl group having 2 to 20 carbon atoms, alkynyl group having 2 to 20 carbon atoms, aryl group having 6 to 20 carbon atoms, and heteroaryl group having 2 to 20 carbon atoms include those as described for the above formula (c1).

In particular, R¹⁵⁵ is preferably a hydrogen atom, an aryl group having 6 to 20 carbon atoms which may be substituted by Z¹, a heteroaryl group having 2 to 20 carbon atoms which may be substituted by Z¹, or an alkyl group having 1 to 20 carbon atoms which may be substituted by Z⁴, more preferably a hydrogen atom, an aryl group having 6 to 14 carbon atoms which may be substituted by Z¹, a heteroaryl group having 2 to 14 carbon atoms which may be substituted by Z¹, or an alkyl group having 1 to 10 carbon atoms which may be substituted by Z⁴, still more preferably a hydrogen atom, an aryl group having 6 to 14 carbon atoms which may be substituted by Z¹, a heteroaryl group having 2 to 14 carbon atoms which may be substituted by Z¹, or an alkyl group having 1 to 10 carbon atoms which may be substituted by Z⁴, and further preferably a hydrogen atom, a phenyl group which may be substituted by Z¹, a 1-naphthyl group which may be substituted by Z¹, a 2-naphthyl group which may be substituted by Z¹, a 2-pyridyl group which may be substituted by Z¹, a 3-pyridyl group which may be substituted by Z¹, a 4-pyridyl group which may be substituted by Z¹, or a methyl group which may be substituted by Z⁴.

In addition, R¹⁵⁶ and R¹⁵⁷ are preferably an aryl group having 6 to 14 carbon atoms which may be substituted by Z¹ or a heteroaryl group having 2 to 14 carbon atoms which may be substituted by Z¹, more preferably an aryl group having 6 to 14 carbon atoms which may be substituted by Z¹, and still more preferably a phenyl group which may be substituted by Z¹, a 1-naphthyl group which may be substituted by Z¹, or a 2-naphthyl group which may be substituted by Z¹.

Hereinafter, specific examples of the group suitable as Ar² include the following, but are not necessarily limited thereto. 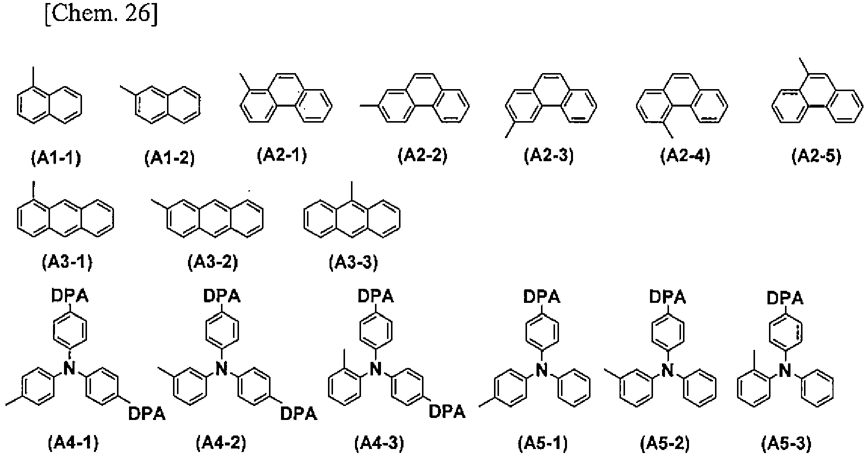 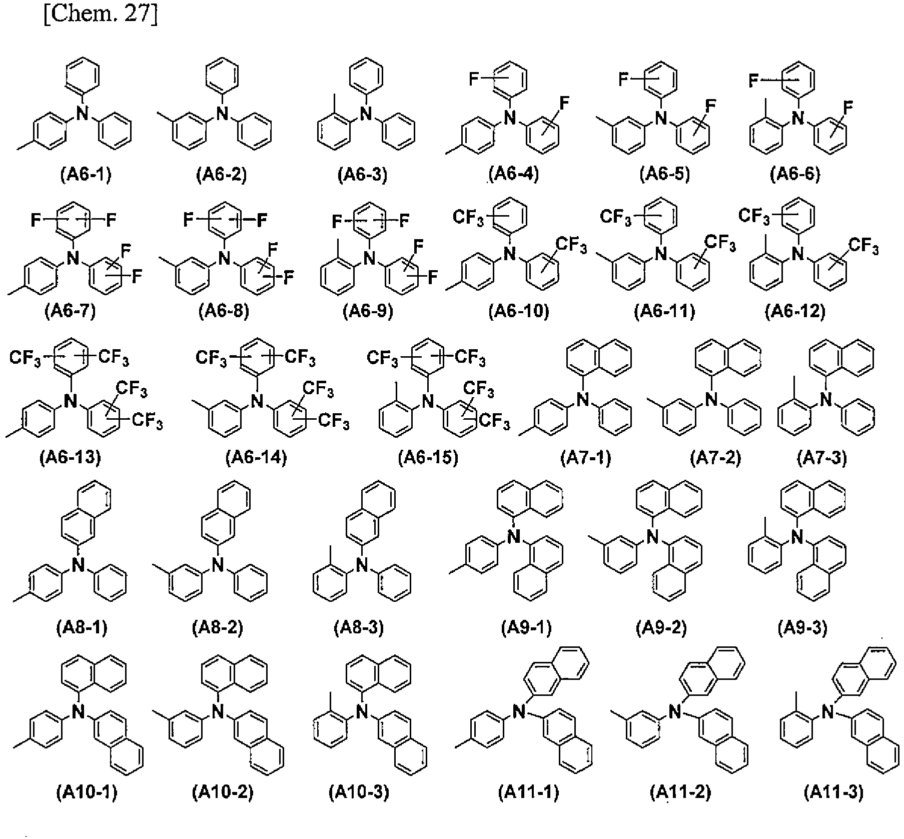 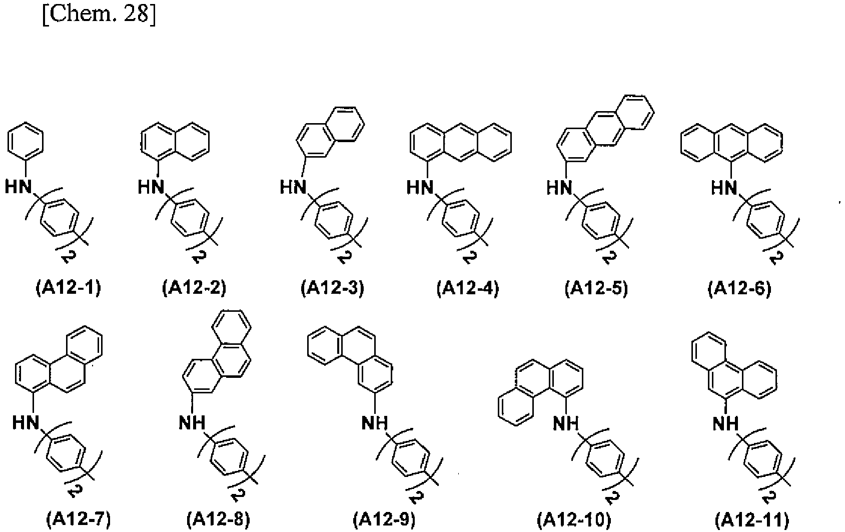 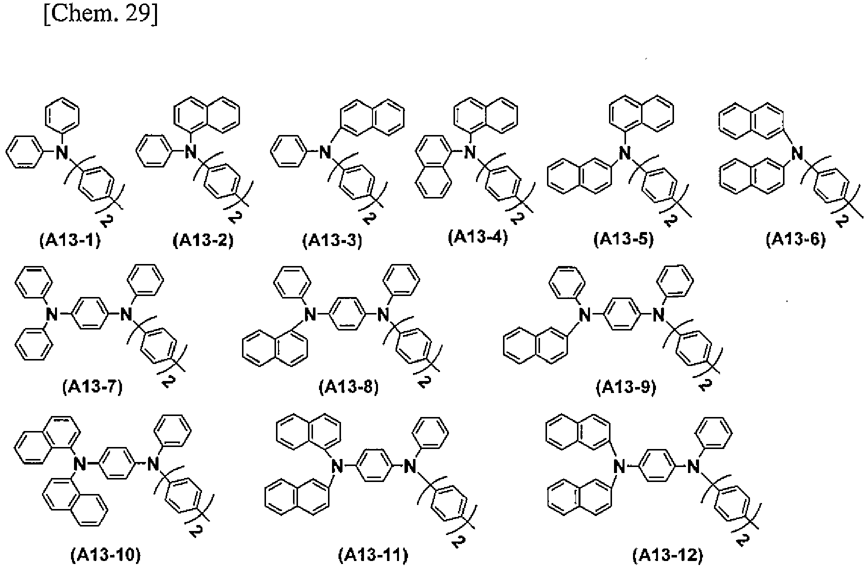 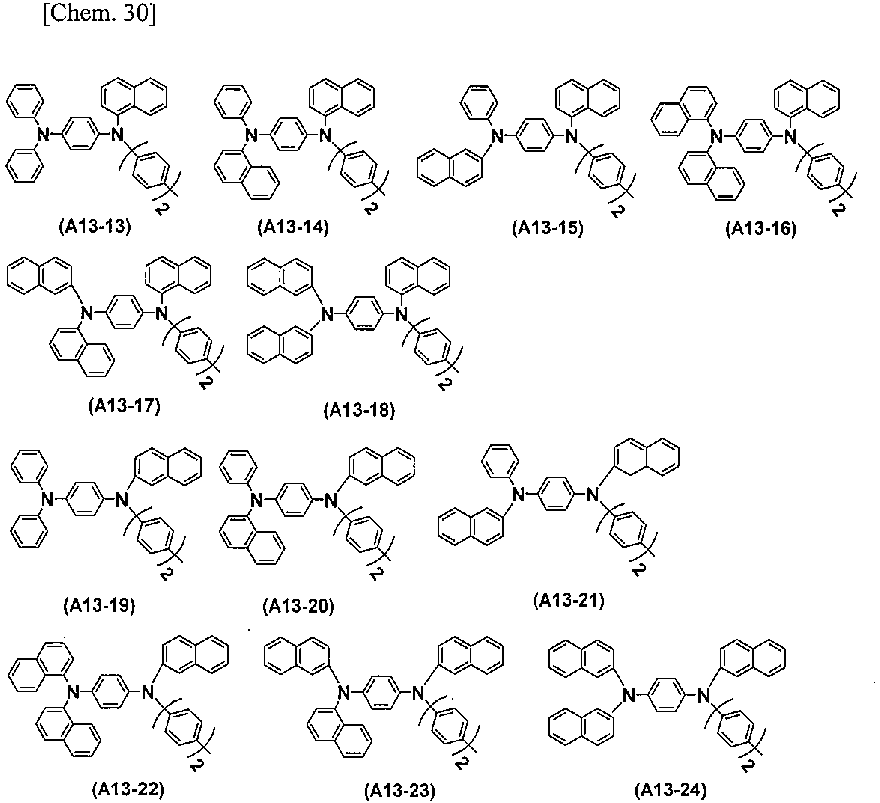 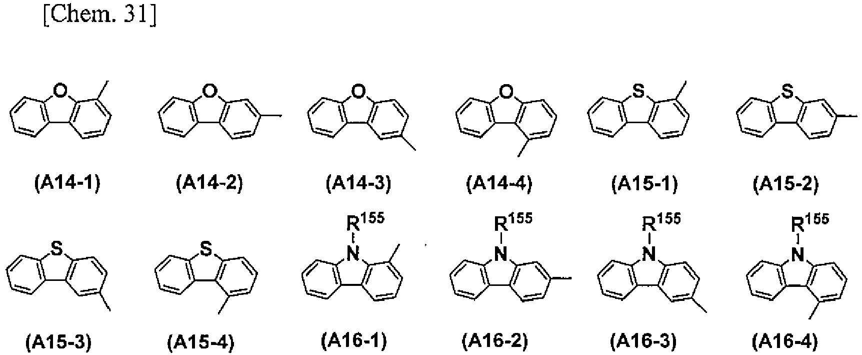 wherein R¹⁵⁵ represents the same meaning as described above.

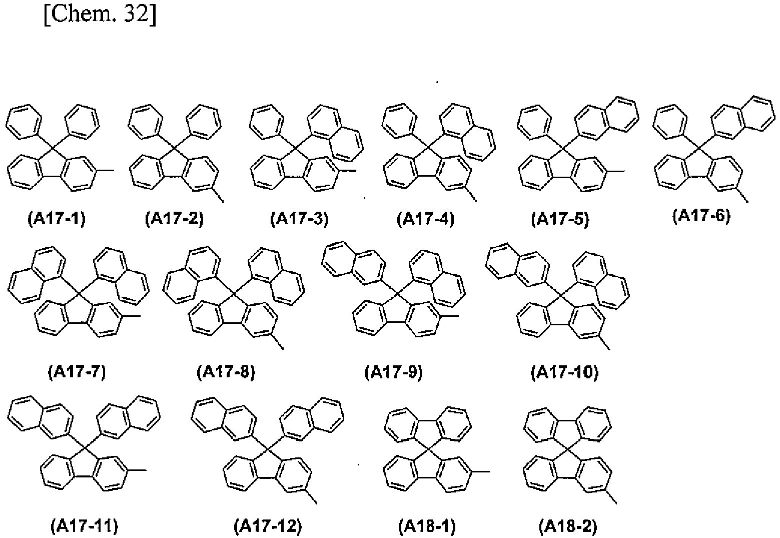

In addition, in formula (1), considering ease of synthesis of the resulting aniline derivative, it is preferred that all Ar¹'s are the same group and all Ar²'s are the same group, and it is more preferred that all Ar¹'s and Ar²'s are the same group. That is, the aniline derivative represented by formula (1) is more preferably the aniline derivative represented by formula (1-1).

Further, use of relatively inexpensive bis(4-aminophenyl)amine as a raw material compound as described later allows relatively easy synthesis, and also due to excellent solubility in the organic solvent, it is preferred that the aniline derivative represented by formula (1) is the aniline derivative represented by formula (1-1).

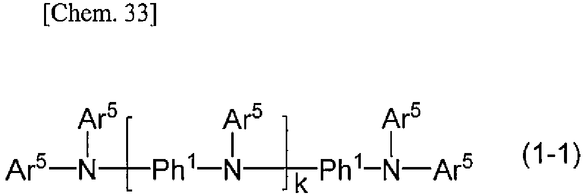

In the above formula (1-1), Ph¹ and k represent the same meaning as described above, Ar⁵ simultaneously represents the group represented by any one of formulae (D1) to (D13), and in particular, the group represented by any one of formulae (D1') to (D13') is preferred.

In addition, specific examples of Ar⁵ include those as described above as the specific examples of Ar¹. 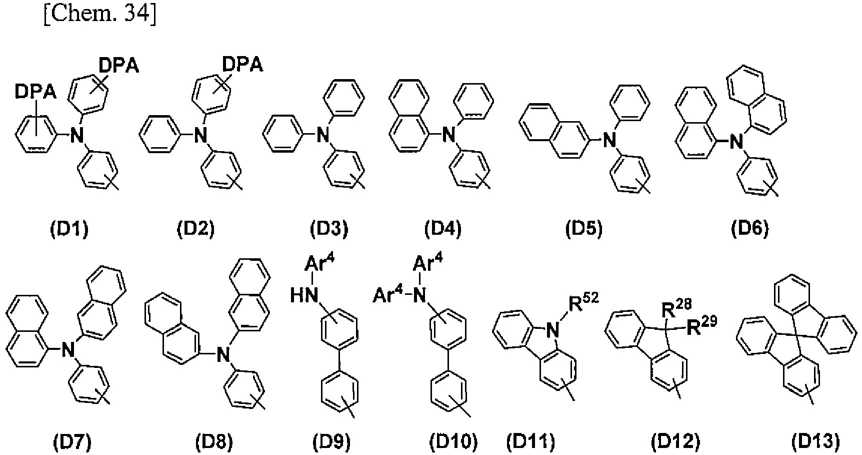 wherein R²⁸, R²⁹, R⁵², Ar⁴, and DPA represent the same meaning as described above. 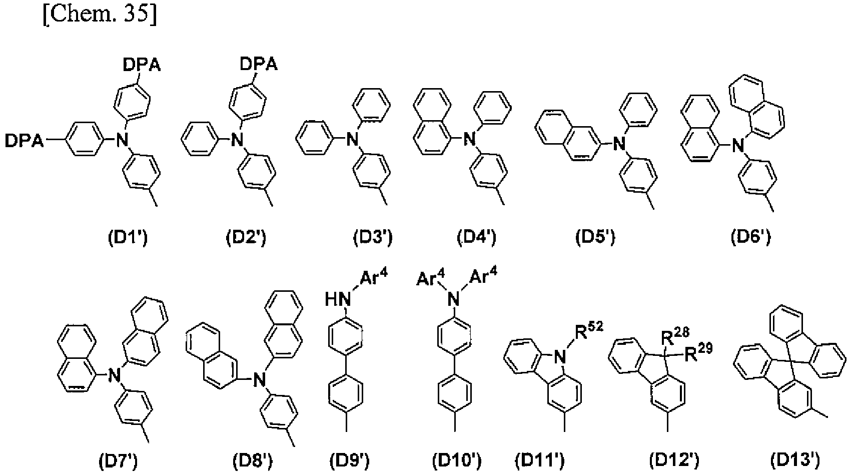 wherein R²⁸, R²⁹, R⁵², Ar⁴, and DPA represent the same meaning as described above.

Further, use of relatively inexpensive bis(4-aminophenyl)amine as a raw material compound as described later allows relatively easy synthesis, and due to excellent solubility of the resulting aniline derivative in the organic solvent, it is preferred that the aniline derivative represented by formula (1) is the aniline derivative represented by formula (1-2).

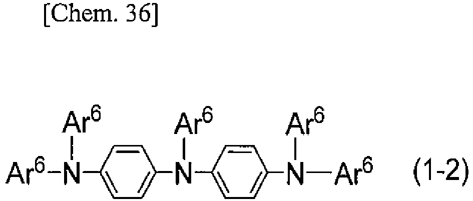

Ar⁶ simultaneously represents a group represented by any one of formulae (E1) to (E14). 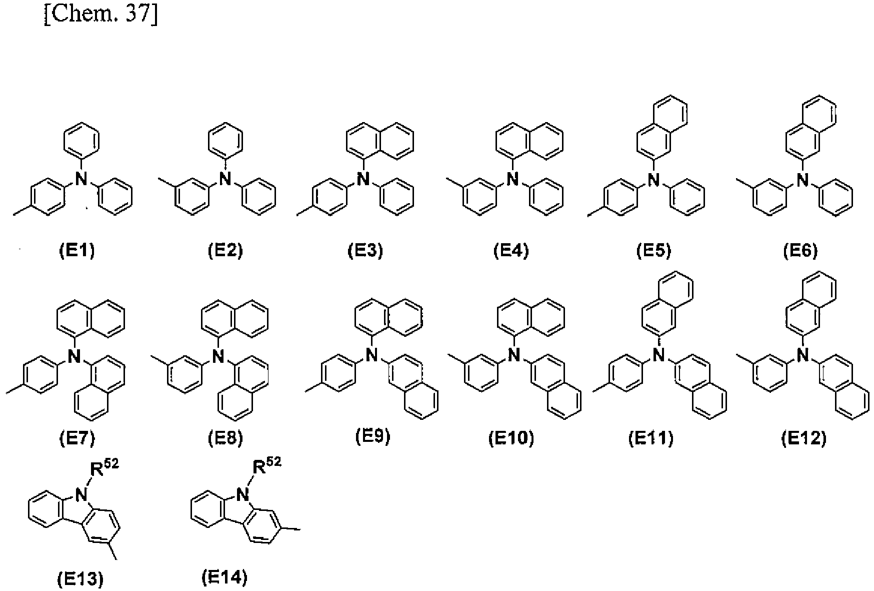 wherein R⁵² represents the same meaning as described above.

In the above formula (2), Ar³ represents a group represented by any one of formulae (C1) to (C8), and in particular, a group represented by any one of formulae (C1') to (C8') is preferred.

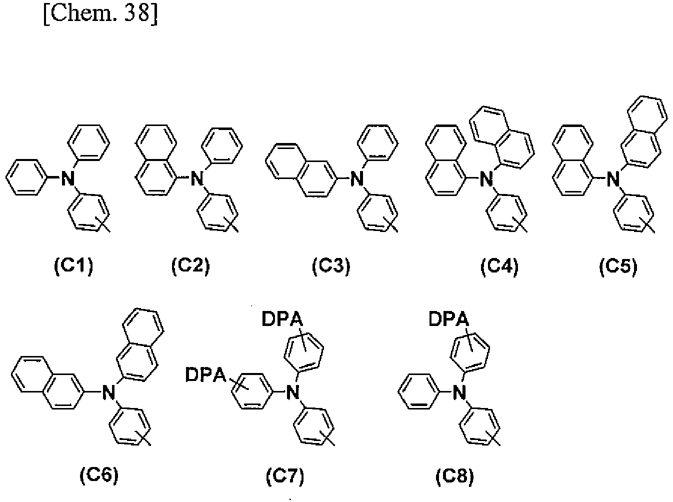

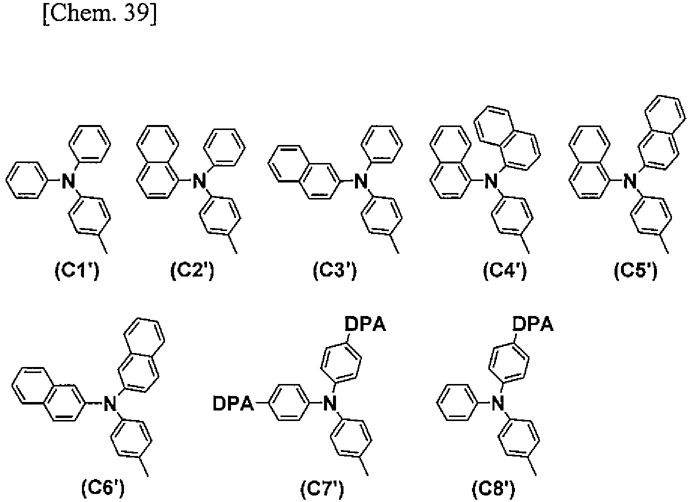

In the above formula (1), k represents an integer of 1 to 10, and from a viewpoint of raising solubility of the compound in an organic solvent, preferably 1 to 5, more preferably 1 to 3, still more preferably 1 or 2, and most preferably 1.

In the above formula (2), 1 represents 1 or 2.

In addition, in R²⁸, R²⁹, R⁵², and R¹⁵⁵ to R¹⁵⁷, Z¹ is preferably a halogen atom, a nitro group, a cyano group, an alkyl group having 1 to 10 carbon atoms which may be substituted by Z², an alkenyl group having 2 to 10 carbon atoms which may be substituted by Z², or an alkynyl group having 2 to 10 carbon atoms which may be substituted by Z², more preferably a halogen atom, a nitro group, a cyano group, an alkyl group having 1 to 3 carbon atoms which may be substituted by Z², an alkenyl group having 2 to 3 carbon atoms which may be substituted by Z², or an alkynyl having 2 to 3 carbon atoms which may be substituted by Z², and still more preferably a fluorine atom, an alkyl group having 1 to 3 carbon atoms which may be substituted by Z², an alkenyl group having 2 to 3 carbon atoms which may be substituted by Z², or an alkynyl group having 2 to 3 carbon atoms which may be substituted by Z².

In R²⁸, R²⁹, R⁵², and R¹⁵⁵ to R¹⁵⁷, Z⁴ is preferably a halogen atom, a nitro group, a cyano group, or an aryl group having 6 to 14 carbon atoms which may be substituted by Z⁵, more preferably a halogen atom, a nitro group, a cyano group, or an aryl group having 6 to 10 carbon atoms which may be substituted by Z⁵, still more preferably a fluorine atom or an aryl group having 6 to 10 carbon atoms which may be substituted by Z⁵, and further preferably a fluorine atom or a phenyl group which may be substituted by Z⁵.

In R²⁸, R²⁹, R⁵², and R¹⁵⁵ to R¹⁵⁷, Z² is preferably a halogen atom, a nitro group, a cyano group, or an aryl group having 6 to 14 carbon atoms which may be substituted by Z³, more preferably a halogen atom, a nitro group, a cyano group, or an aryl group having 6 to 10 carbon atoms which may be substituted by Z³, still more preferably a fluorine atom or an aryl group having 6 to 10 carbon atoms which may be substituted by Z³, and further preferably a fluorine atom or a phenyl group which may be substituted by Z³.

In R²⁸, R²⁹, R⁵², and R¹⁵⁵ to R¹⁵⁷, Z⁵ is preferably a halogen atom, a nitro group, a cyano group, an alkyl group having 1 to 10 carbon atoms which may be substituted by Z³, an alkenyl group having 2 to 10 carbon atoms which may be substituted by Z³, or an alkynyl group having 2 to 10 carbon atoms which may be substituted by Z³, more preferably a halogen atom, a nitro group, a cyano group, an alkyl group having 1 to 3 carbon atoms which may be substituted by Z³, an alkenyl group having 2 to 3 carbon atoms which may be substituted by Z³, or an alkynyl having 2 to 3 carbon atoms which may be substituted by Z³, and still more preferably a fluorine atom, an alkyl group having 1 to 3 carbon atoms which may be substituted by Z³, an alkenyl group having 2 to 3 carbon atoms which may be substituted by Z³, or an alkynyl group having 2 to 3 carbon atoms which may be substituted by Z³.

In R²⁸, R²⁹, R⁵², and R¹⁵⁵ to R¹⁵⁷, Z³ is preferably a halogen atom, and more preferably a fluorine atom.

Meanwhile, in R⁷ to R²⁷, R³⁰ to R⁵¹, and R⁵³ to R¹⁵⁴, Z¹ is preferably a halogen atom, a nitro group, a cyano group, an alkyl group having 1 to 3 carbon atoms which may be substituted by Z², an alkenyl group having 2 to 3 carbon atoms which may be substituted by Z², or an alkynyl group having 2 to 3 carbon atoms which may be substituted by Z², more preferably a halogen atom or an alkyl group having 1 to 3 carbon atoms which may be substituted by Z², and still more preferably a fluorine atom or a methyl group which may be substituted by Z².

In R⁷ to R²⁷, R³⁰ to R⁵¹, and R⁵³ to R¹⁵⁴, Z⁴ is preferably a halogen atom, a nitro group, a cyano group, or an aryl group having 6 to 10 carbon atoms which may be substituted by Z⁵, more preferably a halogen atom or an aryl group having 6 to 10 carbon atoms which may be substituted by Z⁵, and still more preferably a fluorine atom or a phenyl group which may be substituted by Z⁵.

In R⁷ to R²⁷, R³⁰ to R⁵¹, and R⁵³ to R¹⁵⁴, Z² is preferably a halogen atom, a nitro group, a cyano group, or an aryl group having 6 to 10 carbon atoms which may be substituted by Z³, more preferably a halogen atom or an aryl group having 6 to 10 carbon atoms which may be substituted by Z³, and still more preferably a fluorine atom or a phenyl group which may be substituted by Z³.

In R⁷ to R²⁷, R³⁰ to R⁵¹, and R⁵³ to R¹⁵⁴, Z⁵ is preferably a halogen atom, a nitro group, a cyano group, an alkyl group having 1 to 3 carbon atoms which may be substituted by Z³, an alkenyl group having 2 to 3 carbon atoms which may be substituted by Z³, or an alkynyl group having 2 to 3 carbon atoms which may be substituted by Z³, more preferably a halogen atom or an alkyl group having 1 to 3 carbon atoms which may be substituted by Z³, and still more preferably a fluorine atom or a methyl group which may be substituted by Z³.

In R⁷ to R²⁷, R³⁰ to R⁵¹, and R⁵³ to R¹⁵⁴, Z³ is preferably a halogen atom, and more preferably a fluorine atom.

Specific examples of the group suitable as R⁵² and R¹⁵⁵ include the following groups, but are not necessarily limited thereto:

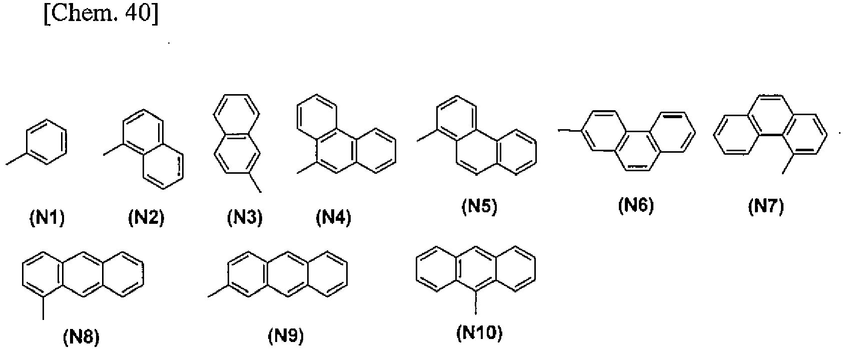

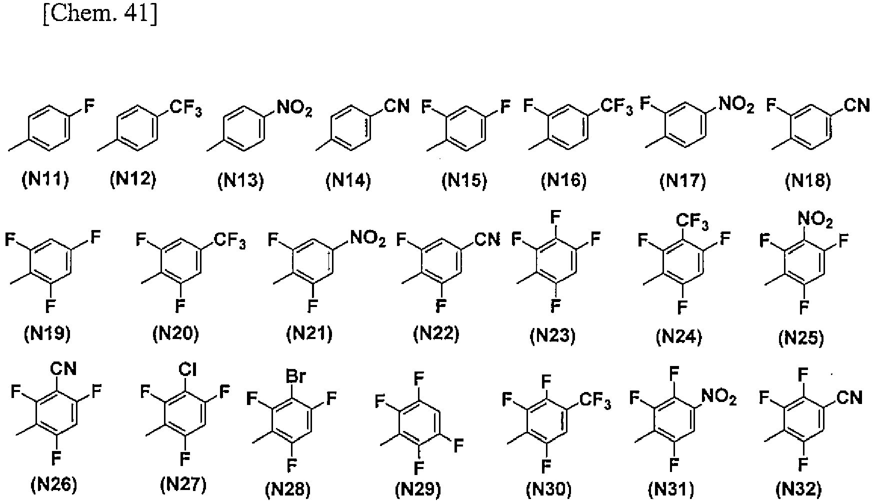

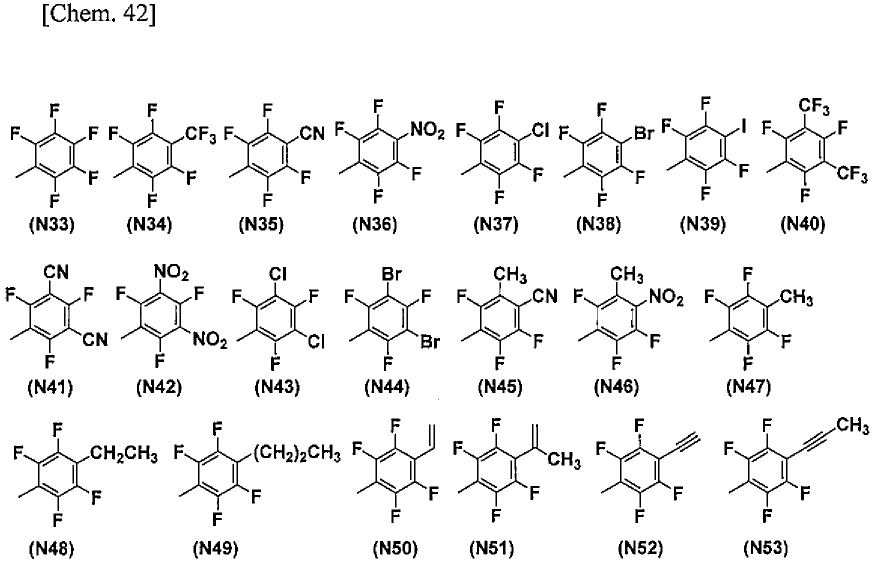

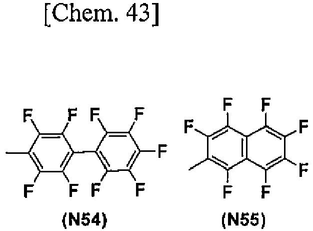

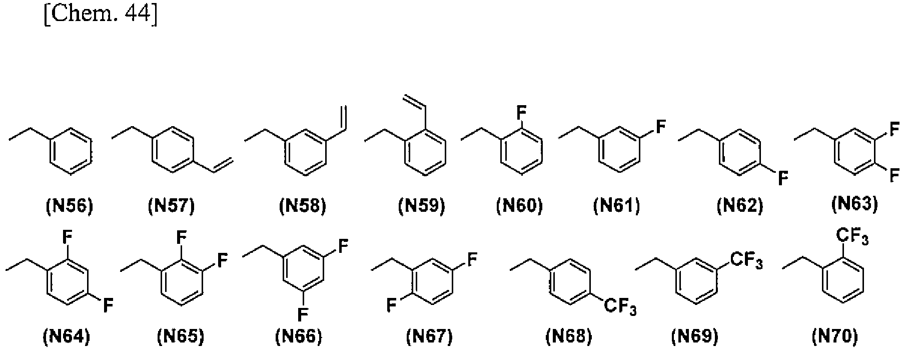

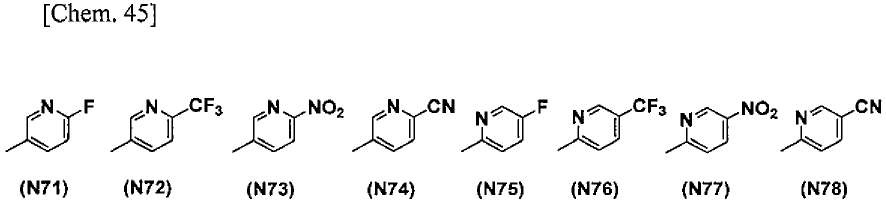

The numbers of carbon atoms of the alkyl group, the alkenyl group, and the alkynyl group are preferably 10 or less, more preferably 6 or less, and still more preferably 4 or less.

Further, the numbers of carbon atoms of the aryl group and the heteroaryl group are preferably 14 or less, more preferably 10 or less, and still more preferably 6 or less.

The aniline derivative represented by the above formula (1) can be produced by reacting the amine compound represented by formula (3) with the aryl compound represented by formula (4) in the presence of a catalyst. 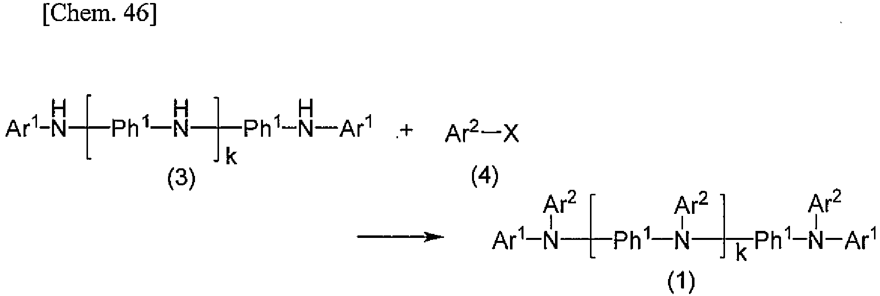 wherein X represents a halogen atom or a pseudo halogen group, and Ar¹, Ar², Ph¹, and k represent the same meaning as described above.

In particular, the aniline derivative represented by formula (1-1) can be produced by reacting the amine compound represented by formula (5) with the aryl compound represented by formula (6) in the presence of a catalyst. 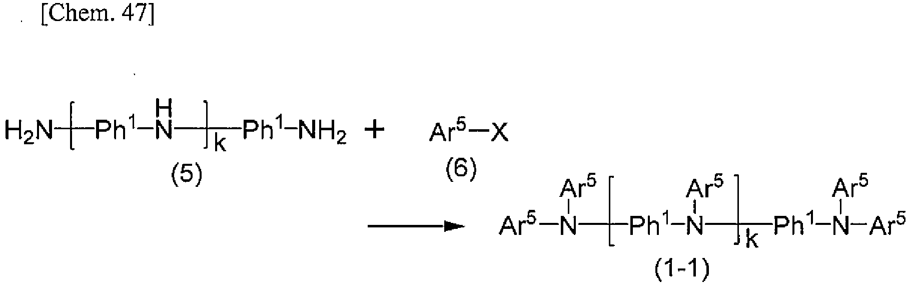 wherein X, Ar⁵, Ph¹, and k represent the same meaning as described above.

Further, the aniline derivative represented by the above formula (1-2) can be produced by reacting bis(4-aminophenyl)amine with the aryl compound represented by formula (7) in the presence of a catalyst. 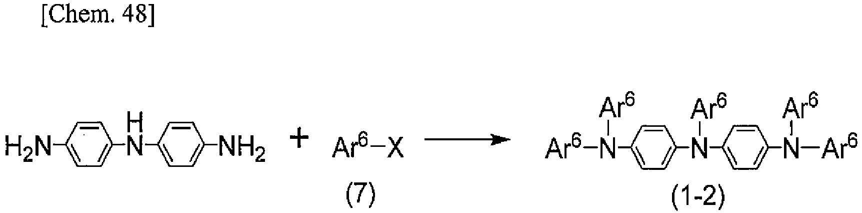 wherein X and Ar⁶ represent the same meaning as described above.

Meanwhile, the aniline derivative represented by the above formula (2) can be produced by reacting the amine compound represented by formula (8) with the aryl compound represented by formula (9) in the presence of a catalyst. 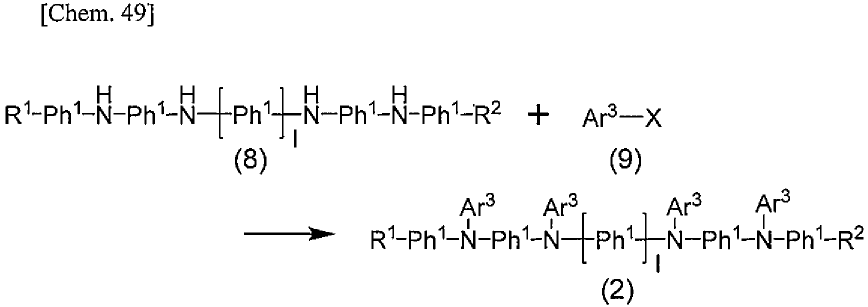 wherein X, R¹, R², Ar³, Ph¹, and 1 represent the same meaning as described above.

Examples of the halogen atom include those as described above.

Examples of the pseudo halogen atom include (fluoro)alkylsulfonyloxy groups such as a methanesulfonyloxy group, a trifluoromethanesulfonyloxy group, and a nonafluorobutanesulfonyloxy group; aromatic sulfonyloxy groups such as a benzenesulfonyloxy group and a toluenesulfonyloxy group; and the like.

A charge ratio between the amine compound represented by formula (3), (5), or (8), or bis(4-aminophenyl)amine and the aryl compound represented by formula (4), (6), (7), or (9) can be equal to or more than the equivalent of the aryl compound, and preferably about 1 to 1.2 equivalents of the aryl compound, per a substance amount of total NH groups of the amine compound or bis(4-aminophenyl)amine.

Examples of the catalyst used in the reaction include copper catalysts such as copper chloride, copper bromide, and copper iodide; palladium catalysts such as Pd(PPh₃)4(tetrakis(triphenylphosphine)palladium), Pd(PPh₃)₂Cl₂(bis(triphenylphosphine)dichloropalladium), Pd(dba)₂(bis(dibenzylideneacetone)palladium), Pd₂(dba)₃(tris(dibenzylideneacetone)dipalladium), Pd(P-t-Bu₃)₂(bis(tri(t-butylphosphine))palladium), and Pd(OAc)₂(palladium acetate); and the like. These catalysts may be used alone or in combination of two or more. Further, these catalysts may be used with a known appropriate ligand.

Examples of such a ligand include tertiary phosphines such as triphenylphosphine, tri-o-tolylphosphine, diphenylmethylphosphine, phenyldimethylphosphine, trimethylphosphine, triethylphosphine, tributylphosphine, tri-tert-butylphosphine, di-t-butyl(phenyl)phosphine, di-tert-butyl(4-dimethylaminophenyl)phosphine, 1,2-bis(diphenylphosphino)ethane, 1,3-bis(diphenylphosphino)propane, 1,4-bis(diphenylphosphino)butane, and 1,1'-bis(diphenylphosphino)ferrocene, tertiary phosphites such as trimethylphosphite, triethylphosphite, and triphenylphosphite, and the like.

A use amount of the catalyst can be about 0.2 mol, and preferably 0.15 mol per 1 mol of the aryl compound represented by formula (4), (6), (7), or (9).

Further, if the ligand is used, the use amount thereof can be 0.1 to 5 equivalents, and preferably 1 to 2 equivalents, per the metal complex to be used.

If the raw material compound is all solid, or from a viewpoint of efficiently obtaining the intended aniline derivative, each of the above reactions is carried out in a solvent. If the solvent is used, the type thereof is not particularly limited as long as it does not adversely affect the reaction. Specific examples thereof include aliphatic hydrocarbons (such as pentane, n-hexane, n-octane, n-decane, and decaline), halogenated aliphatic hydrocarbons (such as chloroform, dichloromethane, dichloroethane, and carbon tetrachloride), aromatic hydrocarbons (such as benzene, nitrobenzene, toluene, o-xylene, m-xylene, p-xylene, and mesitylene), halogenated aromatic hydrocarbons (such as chlorobenzene, bromobenzene, o-dichlorobenzene, m-dichlorobenzene, and p-dichlorobenzene), ethers (such as diethyl ether, diisopropyl ether, t-butylmethyl ether, tetrahydrofuran, dioxane, 1,2-dimethoxyethane, and 1,2-diethoxyethane), ketones (such as acetone, methylethylketone, methylisobutylketone, di-n-butylketone, and cyclohexanone), amides (such as N,N-dimethylformamide and N,N-dimethylacetamide), lactams and lactones (such as N-methylpyrrolidone and γ-butyrolactone), ureas (such as N,N-dimethylimidazolidinone and tetramethylurea), sulfoxides (such as dimethylsulfoxide and sulfolane), nitriles (such as acetonitrile, propionitrile, and butyronitrile), and the like, and these solvents may be used alone or in combination of two or more.

A reaction temperature may be appropriately set in a range from the melting point to the boiling point of the solvent to be used, but in particular, the reaction temperature is preferably about 0 to 200°C and more preferably 20 to 150°C.

After completion of the reaction, work-up is carried out by a usual method and the intended aniline derivative can be obtained.

In a method of producing the aniline derivative represented by the above formula (1), the amine compound represented by formula (3') used as a raw material can be efficiently produced by reacting the amine compound represented by formula (10) with the aryl compound represented by formula (11) in the presence of a catalyst. 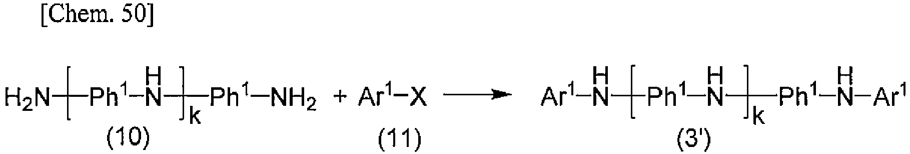 wherein X, Ar¹, Ph¹, and k represent the same meaning as described above, provided that two Ar¹'s are not the group represented by formula (B1) at the same time.

In the method of producing the amine compound represented by formula (3'), the amine compound represented by formula (10) and the aryl compound represented by formula (11) are subjected to a coupling reaction, and it is preferred that a charge ratio between the amine compound represented by formula (10) and the aryl compound represented by formula (11) is about 2 to 2.4 of the aryl compound per 1 of the amine compound, as a substance amount ratio.

In addition, the conditions for the catalyst, the ligand, the solvent, the reaction temperature, and the like in the coupling reaction are the same as the conditions described above for the method of producing the aniline derivative represented by formula (1).

In formula (1), when producing the aniline derivative wherein Ar¹ is the group represented by formula (B4) in which R⁵² is a hydrogen atom or the group represented by formula (B10), or Ar² is the group represented by formula (A12) or the group represented by formula (A16) in which R¹⁵⁵ (in formula (1-1), R⁵² is included) is a hydrogen atom, an aryl compound having a known protecting group on an amino group may be used, in the above-described reaction.

Hereinafter, specific examples of the aniline derivative represented by formula (1) or (2) are shown, but are not limited thereto. In the formulae and tables, "Me" represents a methyl group, "Et" represents an ethyl group, "Prⁿ" represents an n-propyl group, "Prⁱ" represents an i-propyl group, "Buⁿ" represents an n-butyl group, "Buⁱ" represents an i-butyl group, "Bu^{s}" represents a s-butyl group, "Bu^{t}" represents a t-butyl group, "DPA" represents a diphenylamino group, and "SBF" represents a 9,9'-spyrobi[9H-fluorene]-2-yl group.

**[Table 1]**

| | | | | | | | |
|---|---|---|---|---|---|---|---|
| 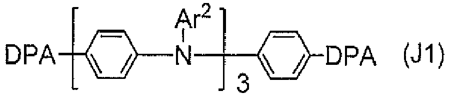 | | | | | | | |

| **Compound** | Ar² | **Compound** | Ar² | **Compound** | Ar² | **Compound** | Ar² |
|---|---|---|---|---|---|---|---|
| (J1-1) | (A1-1) | (J1-27) | (A6-11) | (J1-53) | (A12-7) | (J1-79) | (A13-22) |
| (J1-2) | (A1-2) | (J1-28) | (A6-12) | (J1-54) | (A12-8) | (J1-80) | (A13-23) |
| (J1-3) | (A2-1) | (J1-29) | (A6-13) | (J1-55) | (A12-9) | (J1-81) | (A13-24) |
| (J1-4) | (A2-2) | (J1-30) | (A6-14) | (J1-56) | (A12-10) | (J1-82) | (A14-1) |
| (J1-5) | (A2-3) | (J1-31) | (A6-15) | (J1-57) | (A12-11) | (J1-83) | (A14-2) |
| (J1-6) | (A2-4) | (J1-32) | (A7-1) | (J1-58) | (A13-1) | (J1-84) | (A14-3) |
| (J1-7) | (A2-5) | (J1-33) | (A7-2) | (J1-59) | (A13-2) | (J1-85) | (A14-4) |
| (J1-8) | (A3-1) | (J1-34) | (A7-3) | (J1-60) | (A13-3) | (J1-86) | (A15-1) |
| (J1-9) | (A3-2) | (J1-35) | (A8-1) | (J1-61) | (A13-4) | (J1-87) | (A15-2) |
| (J1-10) | (A3-3) | (J1-36) | (A8-2) | (J1-62) | (A13-5) | (J1-88) | (A15-3) |
| (J1-11) | (A4-1) | (J1-37) | (A8-3) | (J1-63) | (A13-6) | (J1-89) | (A15-4) |
| (J1-12) | (A4-2) | (J1-38) | (A9-1) | (J1-64) | (A13-7) | (J1-90) | (A17-1) |
| (J1-13) | (A4-3) | (J1-39) | (A9-2) | (J1-65) | (A13-8) | (J1-91) | (A17-2) |
| (J1-14) | (A5-1) | (J1-40) | (A9-3) | (J1-66) | (A13-9) | (J1-92) | (A17-3) |
| (J1-15) | (A5-2) | (J1-41) | (A10-1) | (J1-67) | (A13-10) | (J1-93) | (A17-4) |
| (J1-16) | (A5-3) | (J1-42) | (A10-2) | (J1-68) | (A13-11) | (J1-94) | (A17-5) |
| (J1-17) | (A6-1) | (J1-43) | (A10-3) | (J1-69) | (A13-12) | (J1-95) | (A17-6) |
| (J1-18) | (A6-2) | (J1-44) | (A11-1) | (J1-70) | (A13-13) | (J1-96) | (A17-7) |
| (J1-19) | (A6-3) | (J1-45) | (A11-2) | (J1-71) | (A13-14) | (J1-97) | (A17-8) |
| (J1-20) | (A6-4) | (J1-46) | (A11-3) | (J1-72) | (A13-15) | (J1-98) | (A17-9) |
| (J1-21) | (A6-5) | (J1-47) | (A12-1) | (J1-73) | (A13-16) | (J1-99) | (A17-10) |
| (J1-22) | (A6-6) | (J1-48) | (A12-2) | (J1-74) | (A13-17) | (J1-100) | (A17-11) |
| (J1-23) | (A6-7) | (J1-49) | (A12-3) | (J1-75) | (A13-18) | (J1-101) | (A17-12) |
| (J1-24) | (A6-8) | (J1-50) | (A12-4) | (J1-76) | (A13-19) | (J1-102) | (A18-1) |
| (J1-25) | (A6-9) | (J1-51) | (A12-5) | (J1-77) | (A13-20) | (J1-103) | (A18-2) |
| (J1-26) | (A6-10) | (J1-52) | (A12-6) | (J1-78) | (A13-21) | | |

**[Table 2]**

| | | | | | | | |
|---|---|---|---|---|---|---|---|
| 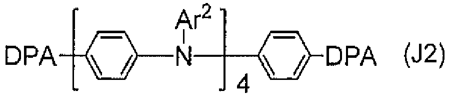 | | | | | | | |

| **Compound** | Ar² | **Compound** | Ar² | **Compound** | Ar² | **Compound** | Ar² |
|---|---|---|---|---|---|---|---|
| (J2-1) | (A1-1) | (J2-27) | (A6-11) | (J2-53) | (A12-7) | (J2-79) | (A13-22) |
| (J2-2) | (A1-2) | (J2-28) | (A6-12) | (J2-54) | (A12-8) | (J2-80) | (A13-23) |
| (J2-3) | (A2-1) | (J2-29) | (A6-13) | (J2-55) | (A12-9) | (J2-81) | (A13-24) |
| (J2-4) | (A2-2) | (J2-30) | (A6-14) | (J2-56) | (A12-10) | (J2-82) | (A14-1) |
| (J2-5) | (A2-3) | (J2-31) | (A6-15) | (J2-57) | (A12-11) | (J2-83) | (A14-2) |
| (J2-6) | (A2-4) | (J2-32) | (A7-1) | (J2-58) | (A13-1) | (J2-84) | (A14-3) |
| (J2-7) | (A2-5) | (J2-33) | (A7-2) | (J2-59) | (A13-2) | (J2-85) | (A14-4) |
| (J2-8) | (A3-1) | (J2-34) | (A7-3) | (J2-60) | (A13-3) | (J2-86) | (A15-1) |
| (J2-9) | (A3-2) | (J2-35) | (A8-1) | (J2-61) | (A13-4) | (J2-87) | (A15-2) |
| (J2-10) | (A3-3) | (J2-36) | (A8-2) | (J2-62) | (A13-5) | (J2-88) | (A15-3) |
| (J2-11) | (A4-1) | (J2-37) | (A8-3) | (J2-63) | (A13-6) | (J2-89) | (A15-4) |
| (J2-12) | (A4-2) | (J2-38) | (A9-1) | (J2-64) | (A13-7) | (J2-90) | (A17-1) |
| (J2-13) | (A4-3) | (J2-39) | (A9-2) | (J2-65) | (A13-8) | (J2-91) | (A17-2) |
| (J2-14) | (A5-1) | (J2-40) | (A9-3) | (J2-66) | (A13-9) | (J2-92) | (A17-3) |
| (J2-15) | (A5-2) | (J2-41) | (A10-1) | (J2-67) | (A13-10) | (J2-93) | (A17-4) |
| (J2-16) | (A5-3) | (J2-42) | (A10-2) | (J2-68) | (A13-11) | (J2-94) | (A17-5) |
| (J2-17) | (A6-1) | (J2-43) | (A10-3) | (J2-69) | (A13-12) | (J2-95) | (A17-6) |
| (J2-18) | (A6-2) | (J2-44) | (A11-1) | (J2-70) | (A13-13) | (J2-96) | (A17-7) |
| (J2-19) | (A6-3) | (J2-45) | (A11-2) | (J2-71) | (A13-14) | (J2-97) | (A17-8) |
| (J2-20) | (A6-4) | (J2-46) | (A11-3) | (J2-72) | (A13-15) | (J2-98) | (A17-9) |
| (J2-21) | (A6-5) | (J2-47) | (A12-1) | (J2-73) | (A13-16) | (J2-99) | (A17-10) |
| (J2-22) | (A6-6) | (J2-48) | (A12-2) | (J2-74) | (A13-17) | (J2-100) | (A17-11) |
| (J2-23) | (A6-7) | (J2-49) | (A12-3) | (J2-75) | (A13-18) | (J2-101) | (A17-12) |
| (J2-24) | (A6-8) | (J2-50) | (A12-4) | (J2-76) | (A13-19) | (J2-102) | (A18-1) |
| (J2-25) | (A6-9) | (J2-51) | (A12-5) | (J2-77) | (A13-20) | (J2-103) | (A18-2) |
| (J2-26) | (A6-10) | (J2-52) | (A12-6) | (J2-78) | (A13-21) | | |

**[Table 3]**

| | | | | | | | |
|---|---|---|---|---|---|---|---|
| 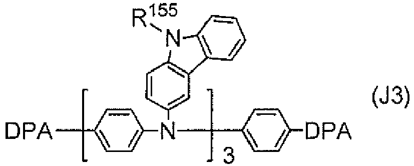 | | | | | | | |

| **Compound** | R¹⁵⁵ | **Compound** | R¹⁵⁵ | **Compound** | R¹⁵⁵ | **Compound** | R¹⁵⁵ |
|---|---|---|---|---|---|---|---|
| (J3-1) | (N1) | (J3-27) | (N27) | (J3-53) | (N53) | (J3-79) | -H |
| (J3-2) | (N2) | (J3-28) | (N28) | (J3-54) | (N54) | (J3-80) | -Me |
| (J3-3) | (N3) | (J3-29) | (N29) | (J3-55) | (N55) | (J3-81) | -Et |
| (J3-4) | (N4) | (J3-30) | (N30) | (J3-56) | (N56) | (J3-82) | -Prⁿ |
| (J3-5) | (N5) | (J3-31) | (N31) | (J3-57) | (N57) | (J3-83) | -Prⁱ |
| (J3-6) | (N6) | (J3-32) | (N32) | (J3-58) | (N58) | (J3-84) | -Buⁿ |
| (J3-7) | (N7) | (J3-33) | (N33) | (J3-59) | (N59) | (J3-85) | -Buⁱ |
| (J3-8) | (N8) | (J3-34) | (N34) | (J3-60) | (N60) | (J3-86) | -Bu^{s} |
| (J3-9) | (N9) | (J3-35) | (N35) | (J3-61) | (N61) | (J3-87) | -Bu^{t} |
| (J3-10) | (N10) | (J3-36) | (N36) | (J3-62) | (N62) | | |
| (J3-11) | (N11) | (J3-37) | (N37) | (J3-63) | (N63) | | |
| (J3-12) | (N12) | (J3-38) | (N38) | (J3-64) | (N64) | | |
| (J3-13) | (N13) | (J3-39) | (N39) | (J3-65) | (N65) | | |
| (J3-14) | (N14) | (J3-40) | (N40) | (J3-66) | (N66) | | |
| (J3-15) | (N15) | (J3-41) | (N41) | (J3-67) | (N67) | | |
| (J3-16) | (N16) | (J3-42) | (N42) | (J3-68) | (N68) | | |
| (J3-17) | (N17) | (J3-43) | (N43) | (J3-69) | (N69) | | |
| (J3-18) | (N18) | (J3-44) | (N44) | (J3-70) | (N70) | | |
| (J3-19) | (N19) | (J3-45) | (N45) | (J3-71) | (N71) | | |
| (J3-20) | (N20) | (J3-46) | (N46) | (J3-72) | (N72) | | |
| (J3-21) | (N21) | (J3-47) | (N47) | (J3-73) | (N73) | | |
| (J3-22) | (N22) | (J3-48) | (N48) | (J3-74) | (N74) | | |
| (J3-23) | (N23) | (J3-49) | (N49) | (J3-75) | (N75) | | |
| (J3-24) | (N24) | (J3-50) | (N50) | (J3-76) | (N76) | | |
| (J3-25) | (N25) | (J3-51) | (N51) | (J3-77) | (N77) | | |
| (J3-26) | (N26) | (J3-52) | (N52) | (J3-78) | (N78) | | |

**[Table 4]**

| | | | | | | | |
|---|---|---|---|---|---|---|---|
| 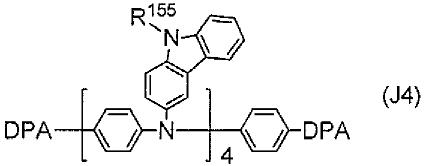 | | | | | | | |

| **Compound** | R¹⁵⁵ | **Compound** | R¹⁵⁵ | **Compound** | R¹⁵⁵ | **Compound** | R¹⁵⁵ |
|---|---|---|---|---|---|---|---|
| (J4-1) | (N1) | (J4-27) | (N27) | (J4-53) | (N53) | (J4-79) | -H |
| (J4-2) | (N2) | (J4-28) | (N28) | (J4-54) | (N54) | (J4-80) | -Me |
| (J4-3) | (N3) | (J4-29) | (N29) | (J4-55) | (N55) | (J4-81) | -Et |
| (J4-4) | (N4) | (J4-30) | (N30) | (J4-56) | (N56) | (J4-82) | -Prⁿ |
| (J4-5) | (N5) | (J4-31) | (N31) | (J4-57) | (N57) | (J4-83) | -Prⁱ |
| (J4-6) | (N6) | (J4-32) | (N32) | (J4-58) | (N58) | (J4-84) | -Buⁿ |
| (J4-7) | (N7) | (J4-33) | (N33) | (J4-59) | (N59) | (J4-85) | -Buⁱ |
| (J4-8) | (N8) | (J4-34) | (N34) | (J4-60) | (N60) | (J4-86) | -Bu^{s} |
| (J4-9) | (N9) | (J4-35) | (N35) | (J4-61) | (N61) | (J4-87) | -Bu^{t} |
| (J4-10) | (N10) | (J4-36) | (N36) | (J4-62) | (N62) | | |
| (J4-11) | (N11) | (J4-37) | (N37) | (J4-63) | (N63) | | |
| (J4-12) | (N12) | (J4-38) | (N38) | (J4-64) | (N64) | | |
| (J4-13) | (N13) | (J4-39) | (N39) | (J4-65) | (N65) | | |
| (J4-14) | (N14) | (J4-40) | (N40) | (J4-66) | (N66) | | |
| (J4-15) | (N15) | (J4-41) | (N41) | (J4-67) | (N67) | | |
| (J4-16) | (N16) | (J4-42) | (N42) | (J4-68) | (N68) | | |
| (J4-17) | (N17) | (J4-43) | (N43) | (J4-69) | (N69) | | |
| (J4-18) | (N18) | (J4-44) | (N44) | (J4-70) | (N70) | | |
| (J4-19) | (N19) | (J4-45) | (N45) | (J4-71) | (N71) | | |
| (J4-20) | (N20) | (J4-46) | (N46) | (J4-72) | (N72) | | |
| (J4-21) | (N21) | (J4-47) | (N47) | (J4-73) | (N73) | | |
| (J4-22) | (N22) | (J4-48) | (N48) | (J4-74) | (N74) | | |
| (J4-23) | (N23) | (J4-49) | (N49) | (J4-75) | (N75) | | |
| (J4-24) | (N24) | (J4-50) | (N50) | (J4-76) | (N76) | | |
| (J4-25) | (N25) | (J4-51) | (N51) | (J4-77) | (N77) | | |
| (J4-26) | (N26) | (J4-52) | (N52) | (J4-78) | (N78) | | |

**[Table 5]**

| | | | | | | | |
|---|---|---|---|---|---|---|---|
| 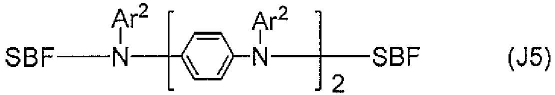 | | | | | | | |

| **Compound** | Ar² | **Compound** | Ar² | **Compound** | Ar² | **Compound** | Ar² |
|---|---|---|---|---|---|---|---|
| (J5-1) | (A1-1) | (J5-27) | (A6-11) | (J5-53) | (A12-7) | (J5-79) | (A13-22) |
| (J5-2) | (A1-2) | (J5-28) | (A6-12) | (J5-54) | (A12-8) | (J5-80) | (A13-23) |
| (J5-3) | (A2-1) | (J5-29) | (A6-13) | (J5-55) | (A12-9) | (J5-81) | (A13-24) |
| (J5-4) | (A2-2) | (J5-30) | (A6-14) | (J5-56) | (A12-10) | (J5-82) | (A14-1) |
| (J5-5) | (A2-3) | (J5-31) | (A6-15) | (J5-57) | (A12-11) | (J5-83) | (A14-2) |
| (J5-6) | (A2-4) | (J5-32) | (A7-1) | (J5-58) | (A13-1) | (J5-84) | (A14-3) |
| (J5-7) | (A2-5) | (J5-33) | (A7-2) | (J5-59) | (A13-2) | (J5-85) | (A14-4) |
| (J5-8) | (A3-1) | (J5-34) | (A7-3) | (J5-60) | (A13-3) | (J5-86) | (A15-1) |
| (J5-9) | (A3-2) | (J5-35) | (A8-1) | (J5-61) | (A13-4) | (J5-87) | (A15-2) |
| (J5-10) | (A3-3) | (J5-36) | (A8-2) | (J5-62) | (A13-5) | (J5-88) | (A15-3) |
| (J5-11) | (A4-1) | (J5-37) | (A8-3) | (J5-63) | (A13-6) | (J5-89) | (A15-4) |
| (J5-12) | (A4-2) | (J5-38) | (A9-1) | (J5-64) | (A13-7) | (J5-90) | (A17-1) |
| (J5-13) | (A4-3) | (J5-39) | (A9-2) | (J5-65) | (A13-8) | (J5-91) | (A17-2) |
| (J5-14) | (A5-1) | (J5-40) | (A9-3) | (J5-66) | (A13-9) | (J5-92) | (A17-3) |
| (J5-15) | (A5-2) | (J5-41) | (A10-1) | (J5-67) | (A13-10) | (J5-93) | (A17-4) |
| (J5-16) | (A5-3) | (J5-42) | (A10-2) | (J5-68) | (A13-11) | (J5-94) | (A17-5) |
| (J5-17) | (A6-1) | (J5-43) | (A10-3) | (J5-69) | (A13-12) | (J5-95) | (A17-6) |
| (J5-18) | (A6-2) | (J5-44) | (A11-1) | (J5-70) | (A13-13) | (J5-96) | (A17-7) |
| (J5-19) | (A6-3) | (J5-45) | (A11-2) | (J5-71) | (A13-14) | (J5-97) | (A17-8) |
| (J5-20) | (A6-4) | (J5-46) | (A11-3) | (J5-72) | (A13-15) | (J5-98) | (A17-9) |
| (J5-21) | (A6-5) | (J5-47) | (A12-1) | (J5-73) | (A13-16) | (J5-99) | (A17-10) |
| (J5-22) | (A6-6) | (J5-48) | (A12-2) | (J5-74) | (A13-17) | (J5-100) | (A17-10 |
| (J5-23) | (A6-7) | (J5-49) | (A12-3) | (J5-75) | (A13-18) | (J5-101) | (A17-12) |
| (J5-24) | (A6-8) | (J5-50) | (A12-4) | (J5-76) | (A13-19) | (J5-102) | (A18-1) |
| (J5-25) | (A6-9) | (J5-51) | (A12-5) | (J5-77) | (A13-20) | (J5-103) | (A18-2) |
| (J5-26) | (A6-10) | (J5-52) | (A12-6) | (J5-78) | (A13-21) | | |

**[Table 6]**

| | | | | | | | |
|---|---|---|---|---|---|---|---|
| 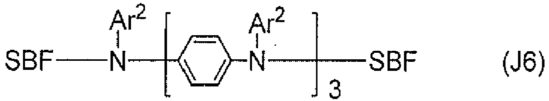 | | | | | | | |

| **Compound** | Ar² | **Compound** | Ar² | **Compound** | Ar² | **Compound** | Ar² |
|---|---|---|---|---|---|---|---|
| (J6-1) | (A1-1) | (J6-27) | (A6-11) | (J6-53) | (A12-7) | (J6-79) | (A13-22) |
| (J6-2) | (A1-2) | (J6-28) | (A6-12) | (J6-54) | (A12-8) | (J6-80) | (A13--23) |
| (J6-3) | (A2-1) | (J6-29) | (A6-13) | (J6-55) | (A12-9) | (J6-81) | (A13-24) |
| (J6-4) | (A2-2) | (J6-30) | (A6-14) | (J6-56) | (A12-10) | (J6-82) | (A14-1) |
| (J6-5) | (A2-3) | (J6-31) | (A6-15) | (J6-57) | (A12-11) | (J6-83) | (A14-2) |
| (J6-6) | (A2-4) | (J6-32) | (A7-1) | (J6-58) | (A13-1) | (J6-84) | (A14-3) |
| (J6-7) | (A2-5) | (J6-33) | (A7-2) | (J6-59) | (A13-2) | (J6-85) | (A14-4) |
| (J6-8) | (A3-1) | (J6-34) | (A7-3) | (J6-60) | (A13-3) | (J6-86) | (A15-1) |
| (J6-9) | (A3-2) | (J6-35) | (A8-1) | (J6-61) | (A13-4) | (J6-87) | (A15-2) |
| (J6-10) | (A3-3) | (J6-36) | (A8-2) | (J6-62) | (A13-5) | (J6-88) | (A15-3) |
| (J6-11) | (A4-1) | (J6-37) | (A8-3) | (J6-63) | (A13-6) | (J6-89) | (A15-4) |
| (J6-12) | (A4-2) | (J6-38) | (A9-1) | (J6-64) | (A13-7) | (J6-90) | (A17-1) |
| (J6-13) | (A4-3) | (J6-39) | (A9-2) | (J6-65) | (A13-8) | (J6-91) | (A17-2) |
| (J6-14) | (A5-1) | (J6-40) | (A9-3) | (J6-66) | (A13-9) | (J6-92) | (A17-3) |
| (J6-15) | (A5-2) | (J6-41) | (A10-1) | (J6-67) | (A13-10) | (J6-93) | (A17-4) |
| (J6-16) | (A5-3) | (J6-42) | (A10-2) | (J6-68) | (A13-11) | (J6-94) | (A17-5) |
| (J6-17) | (A6-1) | (J6-43) | (A10-3) | (J6-69) | (A13-12) | (J6-95) | (A17-6) |
| (J6-18) | (A6-2) | (J6-44) | (A11-1) | (J6-70) | (A13-13) | (J6-96) | (A17-7) |
| (J6-19) | (A6-3) | (J6-45) | (A11-2) | (J6-71) | (A13-14) | (J6-97) | (A17-8) |
| (J6-20) | (A6-4) | (J6-46) | (A11-3) | (J6-72) | (A13-15) | (J6-98) | (A17-9) |
| (J6-21) | (A6-5) | (J6-47) | (A12-1) | (J6-73) | (A13-16) | (J6-99) | (A17-10) |
| (J6-22) | (A6-6) | (J6-48) | (A12-2) | (J6-74) | (A13-17) | (J6-100) | (A17-11) |
| (J6-23) | (A6-7) | (J6-49) | (A12-3) | (J6-75) | (A13-18) | (J6-101) | (A17-12) |
| (J6-24) | (A6-8) | (J6-50) | (A12-4) | (J6-76) | (A13-19) | (J6-102) | (A18-1) |
| (J6-25) | (A6-9) | (J6-51) | (A12-5) | (J6-77) | (A13-20) | (J6-103) | (A18-2) |
| (J6-26) | (A6-10) | (J6-52) | (A12-6) | (J6-78) | (A13-21) | | |

**[Table 7]**

| | | | | | | | |
|---|---|---|---|---|---|---|---|
| 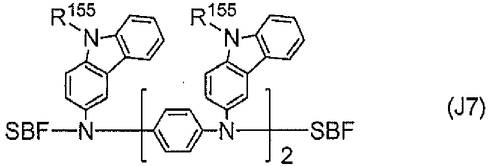 | | | | | | | |

| **Compound** | R¹⁵⁵ | **Compound** | R¹⁵⁵ | **Compound** | R¹⁵⁵ | **Compound** | R¹⁵⁵ |
|---|---|---|---|---|---|---|---|
| (J7-1) | (N1) | (J7-27) | (N27) | (J7-53) | (N53) | (J7-79) | -H |
| (J7-2) | (N2) | (J7-28) | (N28) | (J7-54) | (N54) | (J7-80) | -Me |
| (J7-3) | (N3) | (J7-29) | (N29) | (J7-55) | (N55) | (J7-81) | -Et |
| (J7-4) | (N4) | (J7-30) | (N30) | (J7-56) | (N56) | (J7-82) | -Prⁿ |
| (J7-5) | (N5) | (J7-31) | (N31) | (J7-57) | (N57) | (J7-83) | -Prⁱ |
| (J7-6) | (N6) | (J7-32) | (N32) | (J7-58) | (N58) | (J7-84) | -Buⁿ |
| (J7-7) | (N7) | (J7-33) | (N33) | (J7-59) | (N59) | (J7-85) | -Buⁱ |
| (J7-8) | (N8) | (J7-34) | (N34) | (J7-60) | (N60) | (J7-86) | -Bu^{s} |
| (J7-9) | (N9) | (J7-35) | (N35) | (J7-61) | (N61) | (J7-87) | -Bu^{t} |
| (J7-10) | (N10) | (J7-36) | (N36) | (J7-62) | (N62) | | |
| (J7-11) | (N11) | (J7-37) | (N37) | (J7-63) | (N63) | | |
| (J7-12) | (N12) | (J7-38) | (N38) | (J7-64) | (N64) | | |
| (J7-13) | (N13) | (J7-39) | (N39) | (J7-65) | (N65) | | |
| (J7-14) | (N14) | (J7-40) | (N40) | (J7-66) | (N66) | | |
| (J7-15) | (N15) | (J7-41) | (N41) | (J7-67) | (N67) | | |
| (J7-16) | (N16) | (J7-42) | (N42) | (J7-68) | (N68) | | |
| (J7-J7) | (N17) | (J7-43) | (N43) | (J7-69) | (N69) | | |
| (J7-18) | (N18) | (J7-44) | (N44) | (J7-70) | (N70) | | |
| (J7-19) | (N19) | (J7-45) | (N45) | (J7-71) | (N71) | | |
| (J7-20) | (N20) | (J7-46) | (N46) | (J7-72) | (N72) | | |
| (J7-21) | (N21) | (J7-47) | (N47) | (J7-73) | (N73) | | |
| (J7-22) | (N22) | (J7-48) | (N48) | (J7-74) | (N74) | | |
| (J7-23) | (N23) | (J7-49) | (N49) | (J7-75) | (N75) | | |
| (J7-24) | (N24) | (J7-50) | (N50) | (J7-76) | (N76) | | |
| (J7-25) | (N25) | (J7-51) | (N51) | (J7-77) | (N77) | | |
| (J7-26) | (N26) | (J7-52) | (N52) | (J7-78) | (N78) | | |

**[Table 8]**

| | | | | | | | |
|---|---|---|---|---|---|---|---|
| 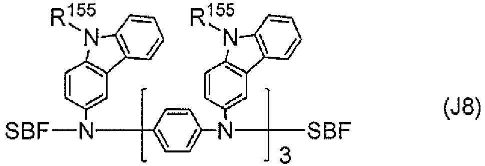 | | | | | | | |

| **Compound** | R¹⁵⁵ | **Compound** | R¹⁵⁵ | **Compound** | R¹⁵⁵ | **Compound** | R¹⁵⁵ |
|---|---|---|---|---|---|---|---|
| (J8-1) | (N1) | (J8-27) | (N27) | (J8-53) | (N53) | (J8-79) | -H |
| (J8-2) | (N2) | (J8-28) | (N28) | (J8-54) | (N54) | (J8-80) | -Me |
| (J8-3) | (N3) | (J8-29) | (N29) | (J8-55) | (N55) | (J8-81) | -Et |
| (J8-4) | (N4) | (J8-30) | (N30) | (J8-56) | (N56) | (J8-82) | -Prⁿ |
| (J8-5) | (N5) | (J8-31) | (N31) | (J8-57) | (N57) | (J8-83) | -Prⁱ |
| (J8-6) | (N6) | (J8-32) | (N32) | (J8-58) | (N58) | (J8-84) | -Buⁿ |
| (J8-7) | (N7) | (J8-33) | (N33) | (J8-59) | (N59) | (J8-85) | -Buⁱ |
| (J8-8) | (N8) | (J8-34) | (N34) | (J8-60) | (N60) | (J8-86) | -Bu^{s} |
| (J8-9) | (N9) | (J8-35) | (N35) | (J8-61) | (N61) | (J8-87) | -Bu^{t} |
| (J8-10) | (N10) | (J8-36) | (N36) | (J8-62) | (N62) | | |
| (J8-11) | (N11) | (J8-37) | (N37) | (J8-63) | (N63) | | |
| (J8-12) | (N12) | (J8-38) | (N38) | (J8-64) | (N64) | | |
| (J8-13) | (N13) | (J8-39) | (N39) | (J8-65) | (N65) | | |
| (J8-14) | (N14) | (J8-40) | (N40) | (J8-66) | (N66) | | |
| (J8-15) | (N15) | (J8-41) | (N41) | (J8-67) | (N67) | | |
| (J8-16) | (N16) | (J8-42) | (N42) | (J8-68) | (N68) | | |
| (J8-17) | (N17) | (J8-43) | (N43) | (J8-69) | (N69) | | |
| (J8-18) | (N18) | (J8-44) | (N44) | (J8-70) | (N70) | | |
| (J8-19) | (N19) | (J8-45) | (N45) | (J8-71) | (N71) | | |
| (J8-20) | (N20) | (J8-46) | (N46) | (J8-72) | (N72) | | |
| (J8-21) | (N21) | (J8-47) | (N47) | (J8-73) | (N73) | | |
| (J8-22) | (N22) | (J8-48) | (N48) | (J8-74) | (N74) | | |
| (J8-23) | (N23) | (J8-49) | (N49) | (J8-75) | (N75) | | |
| (J8-24) | (N24) | (J8-50) | (N50) | (J8-76) | (N76) | | |
| (J8-25) | (N25) | (J8-51) | (N51) | (J8-77) | (N77) | | |
| (J8-26) | (N26) | (J8-52) | (N52) | (J8-78) | (N78) | | |

**[Table 9]**

| | | | | | | | |
|---|---|---|---|---|---|---|---|
| 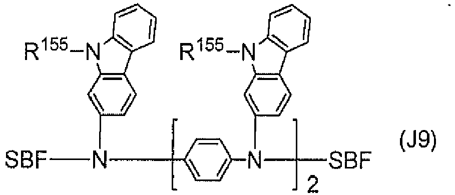 | | | | | | | |

| **Compound** | R¹⁵⁵ | **Compound** | R¹⁵⁵ | **Compound** | R¹⁵⁵ | **Compound** | R¹⁵⁵ |
|---|---|---|---|---|---|---|---|
| (J9-1) | (N1) | (J9-27) | (N27) | (J9-53) | (N53) | (J9-79) | -H |
| (J9-2) | (N2) | (J9-28) | (N28) | (J9-54) | (N54) | (J9-80) | -Me |
| (J9-3) | (N3) | (J9-29) | (N29) | (J9-55) | (N55) | (J9-81) | -Et |
| (J9-4) | (N4) | (J9-30) | (N30) | (J9-56) | (N56) | (J9-82) | -Prⁿ |
| (J9-5) | (N5) | (J9-31) | (N31) | (J9-57) | (N57) | (J9-83) | -Prⁱ |
| (J9-6) | (N6) | (J9-32) | (N32) | (J9-58) | (N58) | (J9-84) | -Buⁿ |
| (J9-7) | (N7) | (J9-33) | (N33) | (J9-59) | (N59) | (J9-85) | -Buⁱ |
| (J9-8) | (N8) | (J9-34) | (N34) | (J9-50) | (N60) | (J9-86) | -Bu^{s} |
| (J9-9) | (N9) | (J9-35) | (N35) | (J9-61) | (N61) | (J9-87) | -Bu^{t} |
| (J9-10) | (N10) | (J9-36) | (N36) | (J9-62) | (N62) | | |
| (J9-11) | (N11) | (J9-37) | (N37) | (J9-63) | (N63) | | |
| (J9-12) | (N12) | (J9-38) | (N38) | (J9-64) | (N64) | | |
| (J9-13) | (N13) | (J9-39) | (N39) | (J9-65) | (N65) | | |
| (J9-14) | (N14) | (J9-40) | (N40) | (J9-66) | (N66) | | |
| (J9-15) | (N15) | (J9-41) | (N41) | (J9-67) | (N67) | | |
| (J9-16) | (N16) | (J9-42) | (N42) | (J9-68) | (N68) | | |
| (J9-17) | (N17) | (J9-43) | (N43) | (J9-69) | (N69) | | |
| (J9-18) | CN18) | (J9-44) | (N44) | (J9-70) | (N70) | | |
| (J9-19) | (N19) | (J9-45) | (N45) | (J9-71) | (N71) | | |
| (J9-20) | (N20) | (J9-46) | (N46) | (J9-72) | (N72) | | |
| (J9-21) | (N21) | (J9-47) | (N47) | (J9-73) | (N73) | | |
| (J9-22) | (N22) | (J9-48) | (N48) | (J9-74) | (N74) | | |
| (J9-23) | (N23) | (J9-49) | (N49) | (J9-75) | (N75) | | |
| (J9-24) | (N24) | (J9-50) | (N50) | (J9-76) | (N76) | | |
| (J9-25) | (N25) | (J9-51) | (N51) | (J9-77) | (N77) | | |
| (J9-26) | (N26) | (J9-52) | (N52) | (J9-78) | (N78) | | |

**[Table 10]**

| | | | | | | | |
|---|---|---|---|---|---|---|---|
| 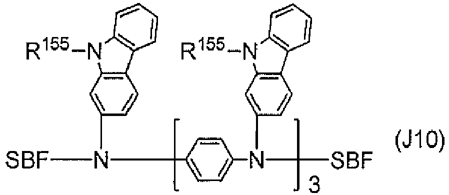 | | | | | | | |

| **Compound** | R¹⁵⁵ | **Compound** | R¹⁵⁵ | **Compound** | R¹⁵⁵ | **Compound** | R¹⁵⁵ |
|---|---|---|---|---|---|---|---|
| (J10-1) | (N1) | (J10-27) | (N27) | (J10-53) | (N53) | (J10-79) | -H |
| (J10-2) | (N2) | (J10-28) | (N28) | (J10-54) | (N54) | (J10-80) | -Me |
| (J10-3) | (N3) | (J10-29) | (N29) | (J10-55) | (N55) | (J10-81) | -Et |
| (J10-4) | (N4) | (J10-30) | (N30) | (J10-56) | (N56) | (J10-82) | -Prⁿ |
| (J10-5) | (N5) | (J10-31) | (N31) | (J10-57) | (N57) | (J10-83) | -Prⁱ |
| (J10-6) | (N6) | (J10-32) | (N32) | (J10-58) | (N58) | (J10-84) | -Buⁿ |
| (J10-7) | (N7) | (J10-33) | (N33) | (J10-59) | (N59) | (J10-85) | -Buⁱ |
| (J10-8) | (N8) | (J10-34) | (N34) | (J10-60) | (N60) | (J10-86) | -Bu^{s} |
| (J10-9) | (N9) | (J10-35) | (N35) | (J10-61) | (N61) | (J10-87) | -Bu^{t} |
| (J10-10) | (N10) | (J10-36) | (N36) | (J10-62) | (N62) | | |
| (J10-1) | (N11) | (J10-37) | (N37) | (J10-63) | (N63) | | |
| (J10-12) | (N12) | (J10-38) | (N38) | (J10-64) | (N64) | | |
| (J10-13) | (N13) | (J10-39) | (N39) | (J10-55) | (N65) | | |
| (J10-14) | (N14) | (J10-40) | (N40) | (J10-66) | (N66) | | |
| (J10-15) | (N15) | (J10-41) | (N41) | (J10-67) | (N67) | | |
| (J10-16) | (N16) | (J10-42) | (N42) | (J10-68) | (N68) | | |
| (J10-17) | (N17) | (J10-43) | (N43) | (J10-69) | (N69) | | |
| (J10-18) | (N18) | (J20-44) | (N44) | (J10-70) | (N70) | | |
| (J10-19) | (N19) | (J10-45) | (N45) | (J10-71) | (N71) | | |
| (J10-20) | (N20) | (J10-46) | (N46) | (J10-72) | (N72) | | |
| (J10-21) | (N21) | (J10-47) | (N47) | (J10-73) | (N73) | | |
| (J10-22) | (N22) | (J10-48) | (N48) | (J10-74) | (N74) | | |
| (J10-23) | (N23) | (J10-49} | (N49) | (J10-75) | (N75) | | |
| (J10-24) | (N24) | (J10-50) | (N50) | (J10-76) | (N76) | | |
| (J10-25) | (N25) | (J10-51) | (N51) | (J10-77) | (N77) | | |
| (J10-26) | (N26) | (J10-52) | (N52) | (J10-78) | (N78) | | |

**[Table 11]**

| | | | | | | | |
|---|---|---|---|---|---|---|---|
| 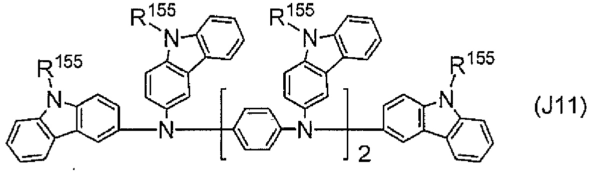 | | | | | | | |

| **Compound** | R¹⁵⁵ | **Compound** | R¹⁵⁵ | **Compound** | R¹⁵⁵ | **Compound** | R¹⁵⁵ |
|---|---|---|---|---|---|---|---|
| (J11-1) | (N1) | (J11-27) | (N27) | (J11-53) | (N53) | (J11-79) | -H |
| (J11-2) | (N2) | (J11-28) | (N28) | (J11-54) | (N54) | (J11-80) | -Me |
| (J11-3) | (N3) | (J11-29) | (N29) | (J11-55) | (N55) | (J11-81) | -Et |
| (J11-4) | (N4) | (J11-30) | (N30) | (J11-56) | (N56) | (J11-82) | -Prⁿ |
| (J11-5) | (N5) | (J11-31) | (N31) | (J11-57) | (N57) | (J11-83) | -Prⁱ |
| (J11-6) | (N6) | (J11-32) | (N32) | (J11-58) | (N58) | (J11-84) | -Suⁿ |
| (J11-7) | (N7) | (J11-33) | (N33) | (J11-59) | (N59) | (J11-85) | -Buⁱ |
| (J11-B) | (N8) | (J11-34) | (N34) | (J11-60) | (N60) | (J11-86) | -Bu^{s} |
| (J11-9) | (N9) | (J11-35) | (N35) | (J11-61) | (N61) | (J11-87) | -Bu^{t} |
| (J11-10) | (N10) | (J11-36) | (N36) | (J11-62) | (N62) | | |
| (J11-11) | (N11) | (J11-37) | (N37) | (J11-63) | (N63) | | |
| (J11-12) | (N12) | (J11-38) | (N38) | (J11-64) | (N64) | | |
| (J11-13) | (N13) | (J11-39) | (N39) | (J11-65) | (N65) | | |
| (J11-14) | (N14) | (J11-40) | (N40) | (J11-66) | (N66) | | |
| (J11-15) | (N15) | (J11-41) | (N41) | (J11-67) | (N67) | | |
| (J11-16) | (N16) | (J11-42) | (N42) | (J11-68) | (N68) | | |
| (J11-17) | (N17) | (J11-43) | (N43) | (J11-69) | (N69) | | |
| (J11-18) | (N18) | (J11-44) | (N44) | (J11-70) | (N70) | | |
| (J11-19) | (N19) | (J11-45) | (N45) | (J11-71) | (N71) | | |
| (J11-20) | (N20) | (J11-46) | (N46) | (J11-72) | (N72) | | |
| (J11-21) | (N21) | (J11-47) | (N47) | (J11-73) | (N73) | | |
| (J11-22) | (N22) | (J11-48) | (N48) | (J11-74) | (N74) | | |
| (J11-23) | (N23) | (J11-49) | (N49) | (J11-75) | (N75) | | |
| (J11-24) | (N24) | (J11-50) | (N50) | (J11-76) | (N76) | | |
| (J11-25) | (N25) | (J11-51) | (N51) | (J11-77) | (N77) | | |
| (J11-26) | (N26) | (J11-52) | (N52) | (J11-78) | (N78) | | |

**[Table 12]**

| | | | | | | | |
|---|---|---|---|---|---|---|---|
| 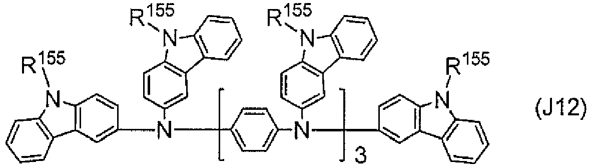 | | | | | | | |

| **Compound** | R¹⁵⁵ | **Compound** | R¹⁵⁵ | **Compound** | R¹⁵⁵ | **Compound** | R¹⁵⁵ |
|---|---|---|---|---|---|---|---|
| (J12-1) | (N1) | (J12-27) | (N27) | (J12-53) | (N53) | (J12-79) | -H |
| (J12-2) | (N2) | (J12-28) | (N28) | (J12-54) | (N54) | (J12-80) | -Me |
| (J12-3) | (N3) | (J12-29) | (N29) | (J12-55) | (N55) | (J12-81) | -Et |
| (J12-4) | (N4) | (J12-30) | (N30) | (J12-56) | (N56) | (J12-82) | -Prⁿ |
| (J12-5) | (N5) | (J12-31) | (N31) | (J12-57) | (N57) | (J12-83) | -Prⁱ |
| (J12-6) | (N6) | (J12-32) | (N32) | (J12-58) | (N58) | (J12-84) | -Buⁿ |
| (J12-7) | (N7) | (J12-33) | (N33) | (J12-59) | (N59) | (J12-85) | -Buⁱ |
| (J12-8) | (N8) | (J12-34) | (N34) | (J12-60) | (N60) | (J12-86) | -Bu^{s} |
| (J12-9) | (N9) | (J12-35) | (N35) | (J12-61) | (N61) | (J12-87) | -Bu^{t} |
| (J12-10) | (N10) | (J12-36) | (N36) | (J12-62) | (N62) | | |
| (J12-11) | (N11) | (J12-37) | (N37) | (J12-63) | (N63) | | |
| (J12-12) | (N12) | (J12-38) | (N38) | (J12-64) | (N64) | | |
| (J12-13) | (N13) | (J12-39) | (N39) | (J12-65) | (N65) | | |
| (J12-14) | (N14) | (J12-40) | (N40) | (J12-66) | (N66) | | |
| (J12-15) | (N15) | (J12-41) | (N41) | (J12-67) | (N67) | | |
| (J12-16) | (N16) | (J12-42) | (N42) | (J12-68) | (N68) | | |
| (J12-17) | (N17) | (J12-43) | (N43) | (J12-69) | (N69) | | |
| (J12-18) | (N18) | (J12-44) | (N44) | (J12-70) | (N70) | | |
| (J12-19) | (N19) | (J12-45) | (N45) | (J12-71) | (N71) | | |
| (J12-20) | (N20) | (J12-46) | (N46) | (J12-72) | (N72) | | |
| (J12-21) | (N21) | (J12-47) | (N47) | (J12-73) | (N73) | | |
| (J12-22) | (N22) | (J12-48) | (N48) | (J12-74) | (N74) | | |
| (J12-23) | (N23) | (J12-49) | (N49) | (J12-75) | (N75) | | |
| (J12-24) | (N24) | (J12-50) | (N50) | (J12-76) | (N76) | | |
| (J12-25) | (N25) | (J12-51) | (N51) | (J12-77) | (N77) | | |
| (J12-26) | (N26) | (J12-52) | (N52) | (J12-78) | (N78) | | |

**[Table 13]**

| | | | | | | | |
|---|---|---|---|---|---|---|---|
| 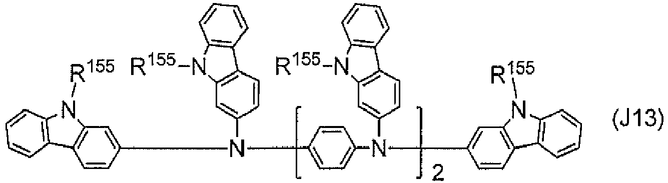 | | | | | | | |

| **Compound** | R¹⁵⁵ | **Compound** | R¹⁵⁵ | **Compound** | R¹⁵⁵ | **Compound** | R¹⁵⁵ |
|---|---|---|---|---|---|---|---|
| (J13-1) | (N1) | (J13-27) | (N27) | (J13-53) | (N53) | (J13-79) | -H |
| (J13-2) | (N2) | (J13-28) | (N28) | (J13-54) | (N54) | (J13-80) | -Me |
| (J13-3) | (N3) | (J13-29) | (N29) | (J13-55) | (N55) | (J13-81) | -Et |
| (J13-4) | (N4) | (J13-30) | (N30) | (J13-56) | (N56) | (J13-82) | -Prⁿ |
| (J13-5) | (N5) | (J13-31) | (N31) | (J13-57) | (N57) | (J13-83) | -Prⁱ |
| (J13-6) | (N6) | (J13-32) | (N32) | (J13-58) | (N58) | (J13-84) | -Buⁿ |
| (J13-7) | (N7) | (J13-33) | (N33) | (J13-59) | (N59) | (J13-85) | -Buⁱ |
| (J13-8) | (N8) | (J13-34) | (N34) | (J13-60) | (N60) | (J13-86) | -Bu^{s} |
| (J13-9) | (N9) | (J13-35) | (N35) | (J13-61) | (N61) | (J13-87) | -Bu^{t} |
| (J13-10) | (N10) | (J13-36) | (N36) | (J13-62) | (N62) | | |
| (J13-11) | (N11) | (J13-37) | (N37) | (J13-63) | (N63) | | |
| (J13-12) | (N12) | (J13-38) | (N38) | (J13-64) | (N64) | | |
| (J13-13) | (N13) | (J13-39) | (N39) | (J13-65) | (N65) | | |
| (J13-14) | (N14) | (J13-40) | (N40) | (J13-66) | (N66) | | |
| (J13-15) | (N15) | (J13-41) | (N41) | (J13-67) | (N67) | | |
| (J13-16) | (N16) | (J13-42) | (N42) | (J13-68) | (N68) | | |
| (J13-17) | (N17) | (J13-43) | (N43) | (J13-69) | (N69) | | |
| (J13-18) | (N18) | (J13-44) | (N44) | (J13-70) | (N70) | | |
| (J13-19) | (N19) | (J13-45) | (N45) | (J13-71) | (N71) | | |
| (J13-20) | (N20) | (J13-46) | (N46) | (J13-72) | (N72) | | |
| (J13-21) | (N21) | (J13-47) | (N47) | (J13-73) | (N73) | | |
| (J13-22) | (N22) | (J13-48) | (N48) | (J13-74) | (N74) | | |
| (J13-23) | (N23) | (J13-49) | (N49) | (J13-75) | (N75) | | |
| (J13-24) | (N24) | (J13-50) | (N50) | (J13-76) | (N76) | | |
| (J13-25) | (N25) | (J13-51) | (N51) | (J13-77) | (N77) | | |
| (J13-26) | (N26) | (J13-52) | (N52) | (J13-78) | (N78) | | |

**[Table 14]**

| | | | | | | | |
|---|---|---|---|---|---|---|---|
| 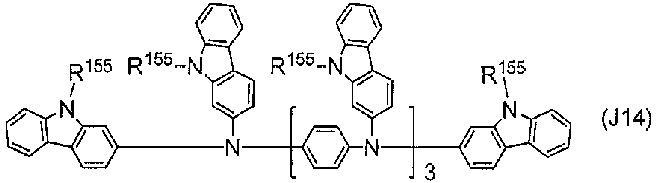 | | | | | | | |

| **Compound** | R¹⁵⁵ | **Compound** | R¹⁵⁵ | **Compound** | R¹⁵⁵ | **Compound** | R¹⁵⁵ |
|---|---|---|---|---|---|---|---|
| (J14-1) | (N1) | (J14-27) | (N27) | (J14-53) | (N53) | (J14-79) | -H |
| (J14-2) | (N2) | (J14-28) | (N28) | (J14-54) | (N54) | (J14-80) | -Me |
| (J14-3) | (N3) | (J14-29) | (N29) | (J14-55) | (N55) | (J14-81) | -Et |
| (J14-4) | (N4) | (J14-30) | (N30) | (J14-56) | (N56) | (J14-82) | -Prⁿ |
| (J14-5) | (N5) | (J14-31) | (N31) | (J14-57) | (N57) | (J14-83) | -Prⁱ |
| (J14-6) | (N6) | (J14-32) | (N32) | (J14-58) | (N58) | (J14-84) | -Buⁿ |
| (J14-7) | (N7) | (J14-33) | (N33) | (J14-59) | (N59) | (J14-85) | -Buⁱ |
| (J14-8) | (N8) | (J 14-34) | (N34) | (J14-60) | (N60) | (J 14-86) | -Bu^{s} |
| (J14-9) | (N9) | (J14-35) | (N35) | (J14-61) | (N61) | (J 14-87) | -Bu^{t} |
| (J14-10) | (N10) | (J14-36) | (N36) | (J14-62) | (N62) | | |
| (J14-11) | (N11) | (J14-37) | (N37) | (J14-63) | (N63) | | |
| (J14-12) | (N12) | (J14-38) | (N38) | (J14-64) | (N64) | | |
| (J14-13) | (N13) | (J14-39) | (N39) | (J14-65) | (N65) | | |
| (J14-14) | (N14) | (J14-40) | (N40) | (J14-66) | (N66) | | |
| (J14-15) | (N15) | (J14-41) | (N41) | (J14-67) | (N67) | | |
| (J14-16) | (N16) | (J14-42) | (N42) | (J14-68) | (N68) | | |
| (J14-17) | (N17) | (J14-43) | (N43) | (J14-69) | (N69) | | |
| (J14-18) | (N18) | (J14-44) | (N44) | (J14-70) | (N70) | | |
| (J14-19) | (N19) | (J14-45) | (N45) | (J14-71) | (N71) | | |
| (J14-20) | (N20) | (J14-46) | (N46) | (J14-72) | (N72) | | |
| (J14-21) | (N21) | (J14-47) | (N47) | (J14-73) | (N73) | | |
| (J14-22) | (N22) | (J14-48) | (N48) | (J14-74) | (N74) | | |
| (J14-23) | (N23) | (J14-49) | (N49) | (J14-75) | (N75) | | |
| (J14-24) | (N24) | (J14-50) | (N50) | (J14-76) | (N76) | | |
| (J14-25) | (N25) | (J14-51) | (N51) | (J14-77) | (N77) | | |
| (J14-26) | (N26) | (J14-52) | (N52) | (J14-78) | (N78) | | |

**[Table 15]**

| | | | | | | | |
|---|---|---|---|---|---|---|---|
| 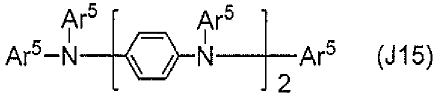 | | | | | | | |

| **Compound** | Ar⁵ | **Compound** | Ar⁵ | **Compound** | Ar⁵ | **Compound** | Ar⁵ |
|---|---|---|---|---|---|---|---|
| (J15-1) | (A1-1) | (J15-27) | (A6-11) | (J15-53) | (A12-7) | (J15-79) | (A13-22) |
| (J15-2) | (A1-2) | (J15-28) | (A6-12) | (J15-54) | (A12-8) | (J15-80) | (A13-23) |
| (J15-3) | (A2-1) | (J15-29) | (A6-13) | (J15-55) | (A12-9) | (J15-81) | (A13-24) |
| (J15-4) | (A2-2) | (J15-30) | (A6-14) | (J15-56) | (A12-10) | (J15-82) | (A14-1) |
| (J15-5) | (A2-3) | (J15-31) | (A6-15) | (J15-57) | (A12-11) | (J15-83) | (A14-2) |
| (J15-6) | (A2-4) | (J15-32) | (A7-1) | (J15-58) | (A13-1) | (J15-84) | (A14-3) |
| (J15-7) | (A2-5) | (J15-33) | (A7-2) | (J15-59) | (A13-2) | (J15-85) | (A14-4) |
| (J15-8) | (A3-1) | (J15-34) | (A7-3) | (J15-60) | (A13-3) | (J15-86) | (A15-1) |
| (J15-9) | (A3-2) | (J15-35) | (A8-1) | (J15-61) | (A13-4) | (J15-87) | (A15-2) |
| (J15-10) | (A3-3) | (J15-36) | (A8-2) | (J15-62) | (A13-5) | (J15-88) | (A15-3) |
| (J15-11) | (A4-1) | (J15-37) | (A8-3) | (J15-63) | (A13-6) | (J15-89) | (A15-4) |
| (J15-12) | (A4-2) | (J15-38) | (A9-1) | (J15-64) | (A13-7) | (J15-90) | (A17-1) |
| (J15-13) | (A4-3) | (J15-39) | (A9-2) | (J15-65) | (A13-8) | (J15-91) | (A17-2) |
| (J15-14) | (A5-1) | (J15-40) | (A9-3) | (J15-66) | (A13-9) | (J15-92) | (A17-3) |
| (J15-15) | (A5-2) | (J15-41) | (A10-1) | (J15-67) | (A13-10) | (J15-93) | (A17-4) |
| (J15-16) | (A5-3) | (J15-42) | (A10-2) | (J15-68) | (A13-11) | (J15-94) | (A17-5) |
| (J15-17) | (A6-1) | (J15-43) | (A10-3) | (J15-69) | (A13-12) | (J15-95) | (A17-6) |
| (J15-18) | (A6-2) | (J15-44) | (A11-1) | (J15-70) | (A13-13) | (J15-96) | (A17-7) |
| (J15-19) | (A6-3) | (J15-45) | (A11-2) | (J15-71) | (A13-14) | (J15-97) | (A17-8) |
| (J15-20) | (A6-4) | (J15-46) | (A11-3) | (J15-72) | (A13-15) | (J15-98) | (A17-9) |
| (J15-21) | (A6-5) | (J15-47) | (A12-1) | (J15-73) | (A13-16) | (J15-99) | (A17-10) |
| (J15-22) | (A6-6) | (J15-48) | (A12-2) | (J15-74) | (A13-17) | (J15-100) | (A17-11) |
| (J15-23) | (A6-7) | (J15-49) | (A12-3) | (J15-75) | (A13-18) | (J15-101) | (A17-12) |
| (J15-24) | (A6-8) | (J15-50) | (A12-4) | (J15-76) | (A13-19) | (J15-102) | (A18-1) |
| (J15-25) | (A6-9) | (J15-51) | (A12-5) | (J15-77) | (A13-20) | (J15-103) | (A18-2) |
| (J15-26) | (A6-10) | (J15-52) | (A12-6) | (J15-78) | (A13-21) | | |

**[Table 16]**

| | | | | | | | |
|---|---|---|---|---|---|---|---|
| 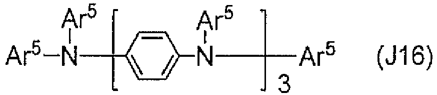 | | | | | | | |

| **Compound** | Ar⁵ | **Compound** | Ar⁵ | **Compound** | Ar⁵ | **Compound** | Ar⁵ |
|---|---|---|---|---|---|---|---|
| (J16-1) | (A1-1) | (J16-27) | (A6-11) | (J16-53) | (A12-7) | (J16-79) | (A13-22) |
| (J16-2) | (A1-2) | (J16-28) | (A6-12) | (J16-54) | (A12-8) | (J16-80) | (A13-23) |
| (J16-3) | (A2-1) | (J16-29) | (A6-13) | (J16-55) | (A12-9) | (J16-81) | (A13-24) |
| (J16-4) | (A2-2) | (J16-30) | (A6-14) | (J16-56) | (A12-10) | (J16-82) | (A14-1) |
| (J16-5) | (A2-3) | (J16-31) | (A6-15) | (J16-57) | (A12-11) | (J16-83) | (A14-2) |
| (J16-6) | (A2-4) | (J16-32) | (A7-1) | (J16-58) | (A13-1) | (J16-84) | (A14-3) |
| (J16-7) | (A2-5) | (J16-33) | (A7-2) | (J16-59) | (A 13-2) | (J16-85) | (A14-4) |
| (J16-8) | (A3-1) | (J16-34) | (A7-3) | (J16-60) | (A13-3) | (J16-86) | (A15-1) |
| (J16-9) | (A3-2) | (J16-35) | (A8-1) | (J16-61) | (A13-4) | (J16-87) | (A15-2) |
| (J16-10) | (A3-3) | (J16-36) | (A8-2) | (J16-62) | (A13-5) | (J16-88) | (A15-3) |
| (J16-11) | (A4-1) | (J16-37) | (A8-3) | (J16-63) | (A13-6) | (J16-89) | (A15-4) |
| (J16-12) | (A4-2) | (J16-38) | (A9-1) | (J16-64) | (A13-7) | (J16-90) | (A17-1) |
| (J16-13) | (A4-3) | (J16-39) | (A9-2) | (J16-65) | (A13-8) | (J16-91) | (A17-2) |
| (J16-14) | (A5-1) | (J16-40) | (A9-3) | (J16-66) | (A13-9) | (J16-92) | (A17-3) |
| (J16-15) | (A5-2) | (J16-41) | (A10-1) | (J16-67) | (A13-10) | (J16-93) | (A17-4) |
| (J16-16) | (A5-3) | (J16-42) | (A10-2) | (J16-68) | (A13-11) | (J16-94) | (A17-5) |
| (J16-17) | (A6-1) | (J16-43) | (A10-3) | (J16-69) | (A13-12) | (J16-95) | (A17-6) |
| (J16-18) | (A6-2) | (J16-44) | (A11-1) | (J16-70) | (A13-13) | (J16-96) | (A17-7) |
| (J16-19) | (A6-3) | (J16-45) | (A11-2) | (J16-71) | (A13-14) | (J16-97) | (A17-8) |
| (J16-20) | (A6-4) | (J16-46) | (A11-3) | (J16-72) | (A13-15) | (J16-98) | (A17-9) |
| (J16-21) | (A6-5) | (J16-47) | (A12-1) | (J16-73) | (A13-16) | (J16-99) | (A17-10) |
| (J16-22) | (A6-6) | (J16-48) | (A12-2) | (J16-74) | (A13-17) | (J16-100) | (A17-11) |
| (J16-23) | (A6-7) | (J16-49) | (A12-3) | (J16-75) | (A13-18) | (J16-101) | (A17-12) |
| (J16-24) | (A6-8) | (J16-50) | (A12-4) | (J16-76) | (A13-19) | (J16-102) | (A18-1) |
| (J16-25) | (A6-9) | (J16-51) | (A12-5) | (J16-77) | (A13-20) | (J16-103) | (A18-2) |
| (J16-26) | (A6-10) | (J16-52) | (A12-6) | (J16-78) | (A13-21) | | |

**[Table 17]**

| | | | | | | | |
|---|---|---|---|---|---|---|---|
| 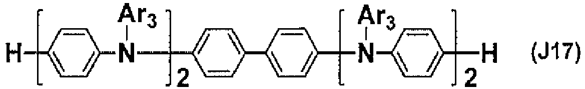 | | | | | | | |

| **Compound** | Ar³ | **Compound** | Ar³ | **Compound** | Ar³ | **Compound** | Ar³ |
|---|---|---|---|---|---|---|---|
| (J17-1) | (C1') | (J17-3) | (C3') | (J17-5) | (C5') | (J17-7) | (C7') |
| (J17-2) | (C2') | (J17-4) | (C4') | (J17-6) | (C6') | (J17-8) | (C8') |

**[Table 18]**

| | | | | | | | |
|---|---|---|---|---|---|---|---|
| 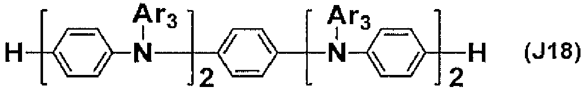 | | | | | | | |

| **Compound** | Ar³ | **Compound** | Ar³ | **Compound** | Ar³ | **Compound** | Ar³ |
|---|---|---|---|---|---|---|---|
| (J18-1) | (C1') | (J18-3) | (C3') | (J18-5) | (C5') | (J18-7) | (C7') |
| (J18-2) | (C2') | (J18-4) | (C4') | (J18-6) | (C6') | (J18-8) | (C8') |

Examples of the thiophene derivative which can be suitably used in the present invention include the polythiophene including the repeating unit represented by the following formula (1). 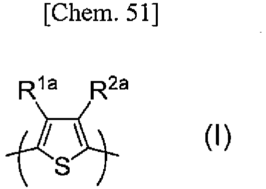 Wherein R^{1a} and R^{2a} are, independently of each other, a hydrogen atom, an alkyl group having 1 to 40 carbon atoms, a fluoroalkyl group having 1 to 40 carbon atoms, an alkoxy group having 1 to 40 carbon atoms, a fluoroalkoxy group having 1 to 40 carbon atoms, an aryloxy group having 6 to 20 carbon atoms, or -O-[Z-O]ₚ-R^{e}, or R^{1a} and R^{2a} form -O-Z-O- together, Z is a hydrocarbylene group having 1 to 40 carbon atoms which may be substituted by Y (wherein Y is a halogen atom, an alkyl group having 1 to 10 carbon atoms, or an alkoxyalkyl group having 1 to 10 carbon atoms, and the alkyl group and the alkoxyalkyl group may be substituted by a sulfonic acid group at any position), p is 1 or more, and R^{e} is a hydrogen atom, an alkyl group having 1 to 40 carbon atoms, a fluoroalkyl group having 1 to 40 carbon atoms, or an aryl group having 6 to 20 carbon atoms.

In particular, it is preferred that R^{1a} and R^{2a} are, independently of each other, a hydrogen atom, a fluoroalkyl group having 1 to 40 carbon atoms, -O[C(R^{a}R^{b})-C(R^{c}R^{d})-O]ₚ-R^{e}, or -OR^{f}. R^{a} to R^{d}, independently of one another, represent a hydrogen atom, a halogen atom, an alkyl group having 1 to 40 carbon atoms, a fluoroalkyl group having 1 to 40 carbon atoms, or an aryl group having 6 to 20 carbon atoms. R^{e} is as described above. p is preferably 1, 2, or 3. R^{f} is preferably an alkyl group having 1 to 40 carbon atoms, a fluoroalkyl group having 1 to 40 carbon atoms, or an aryl group having 6 to 20 carbon atoms.

Examples of the alkyl group having 1 to 40 carbon atoms include a behenyl group, a triacontyl group, a tetracontyl group, and the like, in addition to the alkyl group having 1 to 20 carbon atoms exemplified above.

The fluoroalkyl group having 1 to 40 carbon atoms is not particularly limited as long as it is a linear or branched alkyl group in which at least one hydrogen atom on a carbon atom is substituted by a fluorine atom, and examples thereof include a difluoromethyl group, a trifluoromethyl group, a perfluoropropenyl group, a 1H,1H,2H,2H-perfluorooctyl group, a perfluoroethyl group, -CH₂CF₃, and the like.

In the alkoxy group having 1 to 40 carbon atoms, the alkyl group therein may be linear, branched, or cyclic, and examples of the alkoxy group include a methoxy group, an ethoxy group, an n-propoxy group, an isopropoxy group, an n-butoxy group, an isobutoxy group, and a tert-butoxy.

The fluoroalkoxy group having 1 to 40 carbon atoms is not particularly limited as long as it is an alkoxy group in which at least one hydrogen on a carbon atom is substituted by a fluorine atom, but examples thereof include a fluoromethoxy group, a difluoromethoxy group, a trifluoromethoxy group, a 1-fluoroethoxy group, a 2-fluoroethoxy group, a 1,2-difluoroethoxy group, a 1,1-difluoroethoxy group, a 2,2-difluoroethoxy group, a 1,1,2-trifluoroethoxy group, a 1,2,2-trifluoroethoxy group, a 2,2,2-trifluoroethoxy group, a 1,1,2,2-tetrafluoroethoxy group, a 1,2,2,2-tetrafluoroethoxy group, a 1,1,2,2,2-pentafluoroethoxy group, a 1-fluoropropoxy group, a 2-fluoropropoxy group, a 3-fluoropropoxy group, a 1,1-difluoropropoxy group, a 1,2-difluoropropoxy group, a 1,3-difluoropropoxy group, a 2,2-difluoropropoxy group, a 2,3-difluoropropoxy group, a 3,3-difluoropropoxy group, a 1,1,2-trifluoropropoxy group, a 1,1,3-trifluoropropoxy group, a 1,2,3-trifluoropropoxy group, a 1,3,3-trifluoropropoxy group, a 2,2,3-trifluoropropoxy group, a 2,3,3-trifluoropropoxy group, a 3,3,3-trifluoropropoxy group, a 1,1,2,2-tetrafluoropropoxy group, a 1,1,2,3-tetrafluoropropoxy group, a 1,2,2,3-tetrafluoropropoxy group, a 1,3,3,3-tetrafluoropropoxy group, a 2,2,3,3-tetrafluoropropoxy group, a 2,3,3,3-tetrafluoropropoxy group, a 1,1,2,2,3-pentafluoropropoxy group, a 1,2,2,3,3-pentafluoropropoxy group, a 1,1,3,3,3-pentafluoropropoxy group, a 1,2,3,3,3-pentafluoropropoxy group, a 2,2,3,3,3-pentafluoropropoxy group, a heptafluoropropoxy group, and the like.

A hydrocarbylene group is a divalent hydrocarbon group formed by removing two hydrogen atoms from a hydrocarbon. The hydrocarbylene group may be linear, branched, or cyclic and thus, may be saturated or unsaturated. Examples of the hydrocarbylene group having 1 to 40 carbon atoms include a methylene group, an ethylene group, a 1-phenylethylene group, a propylene group, a trimethylene group, a butylene group, a 1,2-phenylene group, a 1,3-phenylene group, a 1,4-phenylene group, and a 2,6-naphthylene group, but the hydrocarbylene group is not limited thereto.

Examples of the aryl group having 6 to 20 carbon atoms include those as exemplified above.

Examples of the aryloxy group having 6 to 20 carbon atoms include a phenoxy group, a anthracenoxy group, a naphthoxy group, a phenanthrenoxy group, and a fluorenoxy group, but the aryloxy group is not limited thereto.

The alkoxyalkyl group having 1 to 10 carbon atoms may be linear, branched, or cyclic, and specific examples thereof include, a methoxyethyl group, an ethoxyethyl group, a methoxypropyl group, an ethoxypropyl group, a methoxybutyl group, an ethoxybutyl group, and the like, and an alkoxyalkyl group having 1 to 6 carbon atoms is preferred.

Examples of an embodiment of the polythiophene of the above formula (I) include an embodiment in which R^{1a} is a hydrogen atom and R^{2a} is other than a hydrogen atom. In this case, the repeating unit is derived from 3-substituted thiophene.

Polythiophene may be a regiorandom type or regioregular type compound. Due to its asymmetric structure, a mixture having a polythiophene structure containing three kinds of possible regiochemical bonds between the repeating units is produced from polymerization of 3-substituted thiophene. The three orientations available when two thiophene rings are bonded are 2,2', 2,5', and 5,5' couplings. The 2.2' (that is, head to head) coupling and the 5,5' (that is, tail to tail) coupling are referred to as a regiorandom coupling. In contrast, 2,5' (that is, head to tail) coupling is referred to as a regioregular coupling. A degree of regioregularity may be for example, about 0 to 100%, or about 25 to 99. 9%, or about 50 to 98%. The regioregularity can be determined by a standard method known to a person skilled in the art, such as, for example, using NMR spectroscopy.

In an embodiment derived from the 3-substituted thiophene, it is preferred that polythiophene is a regioregular type. In this case, the regioregularity of polythiophene is preferably at least about 85%, more preferably at least about 95%, and still more preferably at least about 98%. In other embodiments, the degree of regioregularity is preferably at least about 70%, more preferably at least about 80%. In still other embodiments, a regioregular type polythiophene has a regioregularity of at least about 90%, and typically has a regioregularity of at least about 98%.

A 3-substituted thiophene monomer (including a polymer derived from the monomer) is commercially available or can be prepared by a method known to a person skilled in the art. A synthetic method, a doping method, and polymer characteristic evaluation, including a regioregular type polythiophene with a side group are described in for example, U.S. Patent No. 6,602,974 of McCullough, et al. and U.S. Patent No. 6,166,172 of McCullough, et al.

Examples of another embodiment of the polythiophene of the above formula (I) include an embodiment in which R^{1a} and R^{2a} are both other than a hydrogen atom. In this case, the repeating unit is derived from 3,4-disubstituted thiophene.

In an embodiment derived from the 3,4-disubstituted thiophene, R^{1a} and R^{2a} are, independently of each other, -O[C(R^{a}R^{b})-C(R^{c}R^{d})-O]ₚ-R^{e} or OR^{f}. In this case, R^{1a} and R^{2a} are more preferably both -O[C(R^{a}R^{b})-C(R^{c}R^{d})-O]ₚ-R^{e}. R^{1a} and R^{2a} may be identical to or different from each other.

Further, R^{a} to R^{d} are preferably a hydrogen atom, an alkyl group having 1 to 8 carbon atoms, a fluoroalkyl group having 1 to 8 carbon atoms, or a phenyl group, and R^{e} is preferably an alkyl group having 1 to 8 carbon atoms, a fluoroalkyl group having 1 to 8 carbon atoms, or a phenyl group.

In an embodiment derived from the 3,4-disubstituted thiophene, R^{1a} and R^{2a} are each preferably O[CH₂-CH₂-O]ₚ-R^{e} or -O[CH(CH₃)-CH₂-O]ₚ-R^{e}.

In addition, R^{e} is more preferably a methyl group, a propyl group, or a butyl group.

Further, in an embodiment in which R^{1a} and R^{2a} form -O-Z-O- together, an embodiment having a repeating unit which is the group represented by the following formulae (Y1) and (Y2) is preferred.

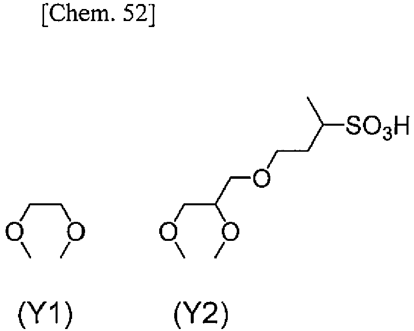

Specific examples of the polythiophene include polythiophene containing one or more repeating units represented by the following formulae (I-1) to (I-5).

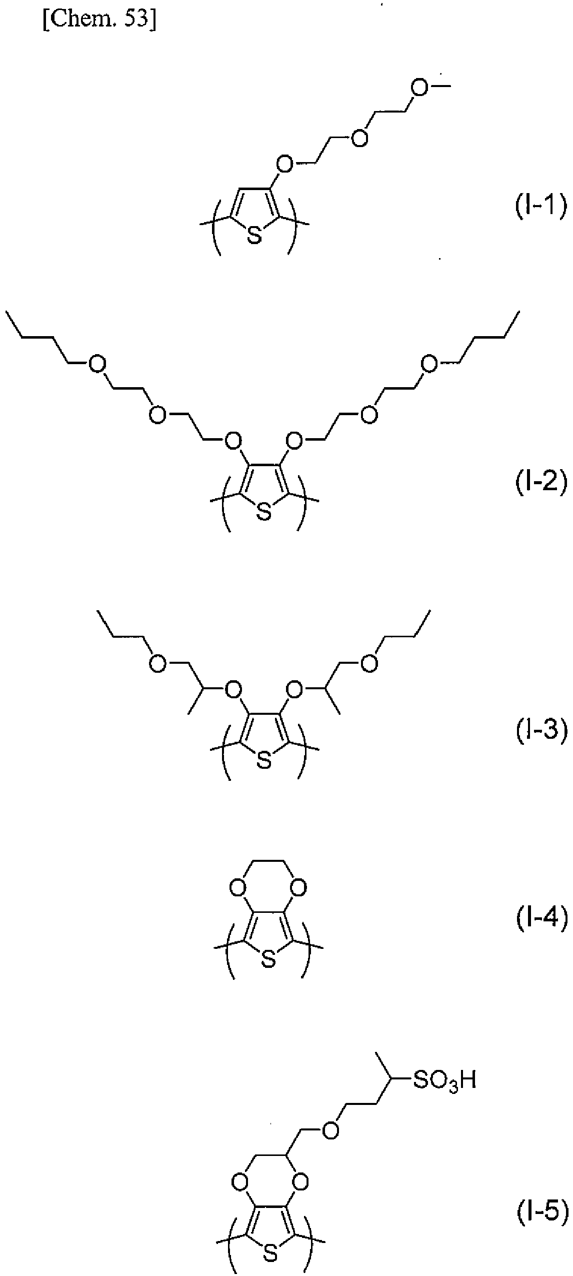

The repeating unit represented by the above formula (I-1) is derived from a monomer having a structure represented by 3-(2-(2-methoxyethoxy)ethoxy)thiophene of the following formula (3-MEET in the following). (In the present specification, referred to as poly(3-MEET).)

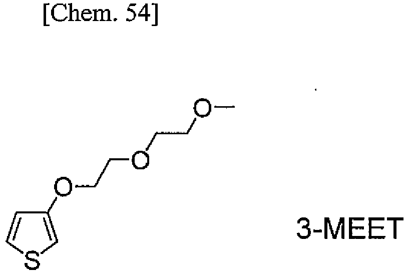

The repeating unit represented by the above formula (I-2) is derived from a monomer having a structure represented by 3,4-bis(2-(2-butoxyethoxy)ethoxy)thiophene of the following formula (3,4-diBEET in the following).

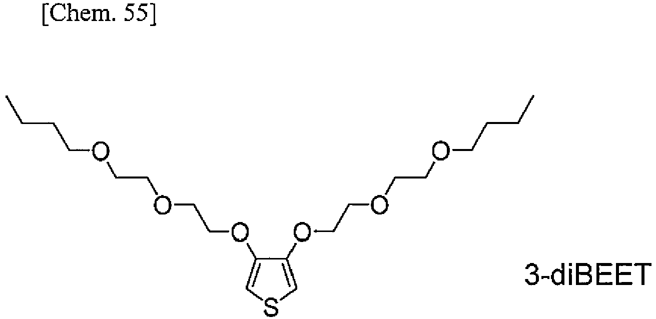

The repeating unit represented by the above formula (I-3) is derived from a monomer having a structure represented by 3,4-bis((1-propoxypropane-2-yl)oxy)thiophene of the following formula (3,4-diPPT in the following).

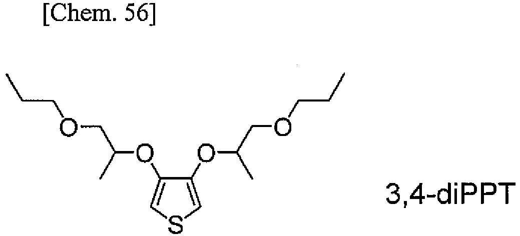

The repeating unit represented by the above formula (I-4) is derived from a monomer having a structure represented by 3,4-ethylenedioxythiophene of the following formula.

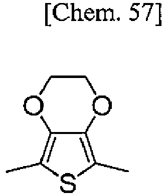

The repeating unit represented by the above formula (I-5) is derived from a monomer having a structure represented by the following formula.

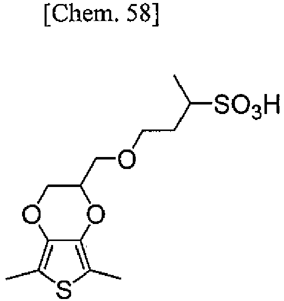

A 3,4-disubstituted thiophene monomer (including a polymer derived from the monomer) is commercially available or can be prepared by a method known to a person skilled in the art. For example, the 3,4-disubstituted thiophene monomer can be produced by reacting 3,4-dibromothiophene with a metal salt, preferably a sodium salt of a compound represented by the formula: HO-[Z-O]ₚ-R^{e} or HOR^{f} (wherein Z, R^{e}, R^{f}, and p represent the same meaning as described above).

Polymerization of 3,4-disubstituted thiophene monomer is carried out first, by brominating 2- and 5-positions of the 3,4-disubstituted thiophene monomer to form a 2,5-dibromo derivative of the 3,4-disubstituted thiophene monomer. Next, the polymer can be obtained by GRIM (Grignard metathesis) polymerization of the 2,5-dibromo derivative of the 3,4-disubstituted thiophene in the presence of a nickel catalyst. Such a method is described in, for example, U.S. Patent No. 8,865,025. Further, examples of other known methods of polymerizing a thiophene monomer include oxidative polymerization using for example, a metal-free organic oxidizing agent such as 2,3-dichloro-5,6-dicyano-1,4-benzoquinone (DDQ) as an oxidizing agent, a transition metal halide such as iron chloride (III), molybdenum chloride (V), and ruthenium chloride (III), and the like.

Examples of the compound represented by formula: HO-[Z-O]ₚ-R^{e} or HOR^{f}, which can be converted to a metal salt, preferably a sodium salt and used to produce a 3,4-disubstituted thiophene monomer, include trifluoroethanol, ethylene glycol monohexyl ether (hexyl cellosolve), propylene glycol monobutyl ether (Dowanol PnB), diethylene glycol monoethyl ether (ethylcarbitol), dipropylene glycol n-butyl ether (Dowanol DPnB), diethylene glycol monophenyl ether (phenylcarbitol), ethylene glycol monobutyl ether (butyl cellosolve), diethylene glycol monobutyl ether (butylcarbitol), dipropylene glycol monomethyl ether (Dowanol DPM), diisobutyl carbinol, 2-ethylhexyl alcohol, methyl isobutyl carbinol, ethylene glycol monophenyl ether (Dowanol Eph), propylene glycol monopropyl ether (Dowanol PnP), propylene glycol monophenyl ether (Dowanol PPh), diethylene glycol monopropyl ether (propylcarbitol), diethylene glycol monohexyl ether (hexylcarbitol), 2-ethylhexyl carbitol, dipropylene glycol monopropyl ether (Dowanol DPnP), tripropylene glycol monomethyl ether (Dowanol TPM), diethylene glycol monomethyl ether (methylcarbitol), tripropylene glycol monobutyl ether (Dowanol TPnB), and the like, but are not limited thereto.

The polythiophene having the repeating unit represented by the above formula (I) can be further modified after polymerization. For example, polythiophene having one or more repeating units derived from a 3-substituted thiophene monomer may have one or more sites at which a hydrogen atom may be substituted by a substituent such as a sulfonic acid group (-SO₃H) by sulfonation.

In the present invention, the term "sulfonation" means that the polythiophene contains one or more sulfonic acid groups (-SO₃H) (the polythiophene is also referred to as "sulfonated polythiophene").

Typically, the sulfur atom of the -SO₃H group is directly bonded to a basic backbone of the polythiophene polymer and is not bonded to a side group. In the present invention, though the side group is theoretically or practically released from the polymer, it is a monovalent group which does not reduce a length of a polymer chain. The sulfonated polythiophene polymer and/or copolymer can be produced using any method known to a person skilled in the art. Examples of the method can include a method of sulfonation by reacting the polythiophene after polymerization with a sulfonation reagent such as fuming sulfuric acid, acetyl sulfate, and pyridine SO₃. Further, a method of polymerization by a known method, using a monomer which is previously sulfonated using the sulfonation reagent, can be included. In addition, for example, in the presence of a basic compound, for example, an alkali metal hydroxide, ammonia, and alkylamine (for example, mono-, di- and trialkylamine, for example, triethylamine, and the like), the sulfonic acid group can result in formation of the corresponding salt or adduct. Thus, the term "sulfonation" related to the polythiophene polymer includes the meaning that the polythiophene may contain one or more -SO₃M groups (wherein M may be an alkali metal ion (for example, Na⁺, Li⁺, K⁺, Rb⁺, Cs⁺, and the like), ammonium (NH₄⁺), or mono-, di-, and trialkylammonium (triethylammonium and the like).

Sulfonation of a conjugated polymer and sulfonated conjugated polymer (including sulfonated polythiophene) are described in U.S. Patent No. 8,017,241 of Seshadri, et al.

Further, sulfonated polythiophene is described in WO 2008/073149 and WO 2016/171935.

Specific examples of sulfonated polythiophene include the polythiophene including the repeating unit represented by the following formula (Is): 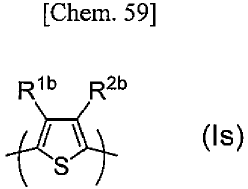 wherein R^{1b} and R^{2b} are, independently of each other, a hydrogen atom, an alkyl group having 1 to 40 carbon atoms, a fluoroalkyl group having 1 to 40 carbon atoms, an alkoxy group having 1 to 40 carbon atoms, a fluoroalkoxy group having 1 to 40 carbon atoms, an aryloxy group having 6 to 20 carbon atoms, -O-[Z-O]ₚ-R^{e}, or -SO₃M, Z is a hydrocarbylene group having 1 to 40 carbon atoms which may be substituted by a halogen atom, p is 1 or more, R^{e} is a hydrogen atom, an alkyl group having 1 to 40 carbon atoms, a fluoroalkyl group having 1 to 40 carbon atoms, or an aryl group having 6 to 20 carbon atoms, M is a hydrogen atom, an alkali metal ion, ammonium, monoalkylammonium, dialkylammonium, or trialkylammonium. However, at least one of R^{1b} and R^{2b} is -SO₃M.

In the formula, it is preferred that R^{1b} and R^{2b} are, independently of each other, a hydrogen atom, a fluoroalkyl group having 1 to 40 carbon atoms, -O[C(R^{a}R^{b})-C(R^{c}R^{d})-O]ₚ-R^{e}, -OR^{f}, or -SO₃M. R^{a} to R^{d}, independently of one another, represent a hydrogen atom, an alkyl group having 1 to 40 carbon atoms, a fluoroalkyl group having 1 to 40 carbon atoms, or an aryl group having 6 to 20 carbon atoms. R^{e} is as described above. p is preferably 1, 2, or 3. R^{f} is preferably an alkyl group having 1 to 40 carbon atoms, a fluoroalkyl group having 1 to 40 carbon atoms, or an aryl group having 6 to 20 carbon atoms.

A preferred embodiment of the sulfonated polythiophene includes, for example, an embodiment in which R^{1b} is -SO₃M and R^{2b} is other than -SO₃M.

Another preferred embodiment of the sulfonated polythiophene includes, for example, an embodiment in which R^{1b} is -SO₃M and R^{2b} is -O[C(R^{a}R^{b})-C(R^{c}R^{d})-O]ₚ-R^{e} or OR^{f}.

Still another preferred embodiment of the sulfonated polythiophene includes, for example, an embodiment in which R^{1b} is -SO₃M and R^{2b} is -O[C(R^{a}R^{b})-C(R^{c}R^{d})-O]ₚ-R^{e}.

Still another preferred embodiment of the sulfonated polythiophene includes, for example, an embodiment in which R^{1b} is -SO₃M and R^{2b} is -O-CH₂CH₂-O-CH₂CH₂-O-CH₃.

Examples of the alkyl group having 1 to 40 carbon atoms, the fluoroalkyl group having 1 to 40 carbon atoms, the alkoxy group having 1 to 40 carbon atoms, the fluoroalkoxy group having 1 to 40 carbon atoms, the aryloxy group having 6 to 20 carbon atoms, the hydrocarbylene group having 1 to 40 carbon atoms, and the aryl group having 6 to 20 carbon atoms include those as exemplified above.

The sulfonated polythiophene represented by the above formula (Is) is obtained by sulfonating polythiophene containing the repeating unit represented by the following formula (II): 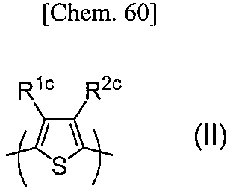 wherein R^{1c} and R^{2c} are, independently of each other, a hydrogen atom, an alkyl group having 1 to 40 carbon atoms, a fluoroalkyl group having 1 to 40 carbon atoms, an alkoxy group having 1 to 40 carbon atoms, a fluoroalkoxy group having 1 to 40 carbon atoms, an aryloxy group having 6 to 20 carbon atoms, or -O-[Z-O]ₚ-R^{e}, Z is a hydrocarbylene group having 1 to 40 carbon atoms which may be substituted by a halogen atom, p is 1 or more, and R^{e} is a hydrogen atom, an alkyl group having 1 to 40 carbon atoms, a fluoroalkyl group having 1 to 40 carbon atoms, or an aryl group having 6 to 20 carbon atoms.

Examples of the alkyl group having 1 to 40 carbon atoms, the fluoroalkyl group having 1 to 40 carbon atoms, the alkoxy group having 1 to 40 carbon atoms, the fluoroalkoxy group having 1 to 40 carbon atoms, the aryloxy group having 6 to 20 carbon atoms, the hydrocarbylene group having 1 to 40 carbon atoms, and the aryl group having 6 to 20 carbon atoms include those as exemplified above.

In the formula, it is preferred that R^{1c} and R^{2c} are, independently of each other, a hydrogen atom, a fluoroalkyl group having 1 to 40 carbon atoms, -O[C(R^{a}R^{b})-C(R^{c}R^{d})-O]ₚ-R^{e}, or -OR^{f}. R^{a} to R^{d}, independently of one another, represent a hydrogen atom, an alkyl group having 1 to 40 carbon atoms, a fluoroalkyl group having 1 to 40 carbon atoms, or an aryl group having 6 to 20 carbon atoms. R^{e} is as described above. p is preferably 1, 2, or 3. R^{f} is preferably an alkyl group having 1 to 40 carbon atoms, a fluoroalkyl group having 1 to 40 carbon atoms, or an aryl group having 6 to 20 carbon atoms.

Examples of an embodiment of the polythiophene of the above formula (II) include an embodiment in which R^{1c} is a hydrogen atom and R^{2c} is other than a hydrogen atom. In this case, the repeating unit is derived from 3-substituted thiophene.

The sulfonated polythiophene is obtained from polythiophene of a regiorandom type or regioregular type compound. Due to its asymmetric structure, a mixture having a polythiophene structure containing a chemical bond at three possible positions between the repeating units is produced from polymerization of 3-substituted thiophene. The three orientations available when two thiophene rings are bonded are 2,2', 2,5', and 5,5' couplings. The 2.2' (that is, head to head) coupling and the 5,5' (that is, tail to tail) coupling are referred to as a regiorandom coupling. In contrast, 2,5' (that is, head to tail) coupling is referred to as a regioregular coupling. A regioregularity degree is for example, about 0 to 100%, or about 25 to 99.9%, or about 50 to 98%. The regioregularity can be determined by a standard method known to a person skilled in the art, such as, for example, using NMR spectroscopy.

A 3-substituted thiophene monomer (including a polymer derived from the monomer) is commercially available or can be prepared by a method known to a person skilled in the art. A synthetic method, a doping method, and polymer characteristic evaluation including regioregular type polythiophene with a side group are provided in for example, U.S. Patent No. 6,602,974 of McCullough, et al. and U.S. Patent No. 6,166,172 of McCullough, et al. Sulfonation of a conjugated polymer and sulfonated conjugated polymer (including sulfonated polythiophene) are described in U.S. Patent No. 8,017,241 of Seshadri, et al.

In the above formula (Is), an embodiment in which R^{1b} is a hydrogen atom, R^{2b} is -O[C(R^{a}R^{b})-C(R^{c}R^{d})-O]ₚ-R^{e}, or OR^{f} is preferred, and an embodiment in which R^{1b} is hydrogen atom and R^{2b} is -O[C(R^{a}R^{b})-C(R^{c}R^{d})-O]ₚ-R^{e} is more preferred.

It is preferred that R^{a} to R^{d} are, independently of one another, a hydrogen atom, an alky group having 1 to 8 carbon atoms, a fluoroalkyl group having 1 to 8 carbon atoms, or a phenyl group, and it is preferred that R^{e} and R^{f} are, independently of each other, a hydrogen atom, an alkyl group having 1 to 8 carbon atoms, a fluoroalkyl group having 1 to 8 carbon atoms, or a phenyl group.

Further, in the above formula (Is), it is preferred that R^{2b} is -O[CH₂-CH₂-O]ₚ-R^{e} or -OR^{f}.

Examples of the compound represented by formula: -O[C(R^{a}R^{b})-C(R^{c}R^{d})-O]ₚ-R^{e} or HOR^{f}, which can be converted to a metal salt, preferably a sodium salt and bonded to a thiophene monomer to form a 3-substituted thiophene (which is then used for sulfonation to produce polythiophene), include trifluoroethanol, ethylene glycol monohexyl ether (hexyl cellosolve), propylene glycol monobutyl ether (Dowanol PnB), diethylene glycolmonoethyl ether (ethylcarbitol), dipropylene glycol n-butyl ether (Dowanol DPnB), diethylene glycol monophenyl ether (phenylcarbitol), ethylene glycol monobutyl ether (butyl cellosolve), diethylene glycol monobutyl ether (butylcarbitol), dipropylene glycol monomethyl ether (Dowanol DPM), diisobutyl carbinol, 2-ethylhexyl alcohol, methyl isobutyl carbinol, ethylene glycol monophenyl ether (Dowanol Eph), propylene glycol monopropyl ether (Dowanol PnP), propylene glycol monophenyl ether (Dowanol PPh), diethylene glycol monopropyl ether (propylcarbitol), diethylene glycol monohexyl ether (hexylcarbitol), 2-ethylhexylcarbitol, dipropylene glycol monopropyl ether (Dowanol DPnP), tripropylene glycol monomethyl ether (Dowanol TPM), diethylene glycol monomethyl ether (methylcarbitol), tripropylene glycol monobutyl ether (Dowanol TPnB), and the like, but are not limited thereto.

Further, R^{e} is preferably a hydrogen atom, a methyl group, a propyl group, or a butyl group, and R^{f} is preferably -CH₂CF₃.

In the present invention, the sulfonated polythiophene can be obtained by sulfonating polythiophene containing the repeating unit represented by the above formulae (I-1) to (I-5).

Each of the above polythiophene polymers may be a homopolymer or a copolymer (including statistical, random, gradient, and block copolymers). As a polymer including monomer A and monomer B, the block copolymer includes for example, an A-B diblock copolymer, an A-B-A triblock copolymer, and an (AB)ₙ-multiblock copolymer. Polythiophene may include a repeating unit derived from other types of monomer (for example, thienothiophene, selenophene, pyrrole, furan, tellurophene, aniline, arylamine, and arylene (for example, phenylene, phenylene vinylene, fluorene, and the like)).

In the present invention, the content of the repeating unit represented by formula (I) or (Is) in the polythiophene is preferably more than 50% by weight, more preferably more than 80% by weight, still more preferably more than 90% by weight, and most preferably more than 95% by weight, per the total weight of the repeating unit.

In the present invention, depending on the purity of the starting monomer compound used in polymerization, the polymer to be formed may contain repeating units derived from impurities. In the present invention, the term "homopolymer" means a polymer containing repeating units derived from one type of monomer, but may also contain repeating units derived from impurities. In the present invention, it is preferred that the polythiophene is a homopolymer in which basically all of the repeating units are the repeating unit represented by the above formula (I) or (Is).

The number average molecular weight of the polythiophene polymer is preferably about 1,000 to 1,000,000 g/mol, more preferably about 5,000 to 100,000 g/mol, and still more preferably about 10,000 to about 50,000 g/mol. The number average molecular weight can be determined, for example, by a method known to a person skilled in the art such as gel permeation chromatography.

In the present invention, the polythiophene may be used after being treated with a reducing agent.

In the polythiophene, a chemical structure of a part of the repeating units forming the polythiophene may be an oxidative structure called "a quinoid structure". The term "quinoid structure" is used against the term "benzenoid structure", and for the latter which is a structure containing an aromatic ring, the former means a structure in which an endocyclic double bond in the aromatic ring is moved out of the ring (as a result, the aromatic ring disappears) and two exocyclic double bonds which remain in the ring and are bonded to other double bonds are formed. A person skilled in the art can easily understand the relationship between these two structures from the relationship between the structures of benzoquinone and hydroquinone. The quinoid structure for the repeating units of various conjugated polymers is well known to a person skilled in the art. As an example, the quinoid structure corresponding to the repeating unit represented by the above formula (I) is shown in the following formula (I').

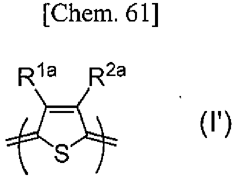

Wherein R^{1a} and R^{2a} are as defined in the above formula (I).

The quinoid structure forms a part of structures referred to as "a polaron structure" and "a bipolaron structure", which are produced by a process in which the polythiophene represented by the above formula (I) undergoes an oxidation reaction by a dopant, a so-called doping reaction, and imparts charge transportability to polythiophene. These structures are known in the art. Introduction of the "polaron structure" and/or the "bipolaron structure" is essential in manufacture of an organic EL device, and actually, when the organic EL device is manufactured, it is achieved by intentionally causing the above doping reaction, at the time of a baking treatment of a charge-transporting thin film formed from a charge-transporting varnish. The reason that the polythiophene prior to causing the doping reaction includes the quinoid structure is considered as being that in the production process (in the case of sulfonated polythiophene, in particular, the sulfonation process among them), an unintended oxidation reaction which is equivalent to the doping reaction is caused.

There is a correlation between the amount of the quinoid structure included in the polythiophene and the dispersibility of the polythiophene in an organic solvent, and as the amount of the quinoid structure is increased, the dispersibility tends to be decreased. For this reason, introduction of the quinoid structure after forming the charge-transporting thin film formed from the charge-transporting varnish does not cause a problem, but if the quinoid structure is excessively introduced into polythiophene by the above unintended oxidation reaction, production of the charge-transporting varnish may be hindered. In polythiophenes, it is known that dispersibility in organic solvents varies, and one of the causes is considered as being that the amount of the quinoid structure introduced into the polythiophene by the above unintended oxidation reaction fluctuates depending on a difference in the production conditions of each polythiophene.

Therefore, when the polythiophene is subjected to a reduction treatment using a reducing agent, even though the quinoid structure is excessively introduced to the polythiophene, reduction decreases the quinoid structure and improves dispersibility of polythiophene in an organic solvent, and thus, it is possible to stably produce a good charge-transporting varnish which provides a charge-transporting thin film having excellent homogeneity.

The reducing agent used in the reduction treatment is not particularly limited, as long as it can reduce the quinoid structure to convert the structure into a non-oxidative structure, that is, the above benzenoid structure (for example, in the polythiophene represented by the above formula (I), the quinoid structure represented by the above formula (I') is converted into the structure represented by the above formula (I)), and for example, it is preferred to use ammonia water, hydrazine, or the like. The amount of the reducing agent is preferably 0.1 to 10 parts by weight, and more preferably 0.5 to 2 parts by weight, per 100 parts by weight of the polythiophene to be treated.

The method and conditions of reduction treatment are not particularly limited. For example, the treatment can be carried out simply by bringing polythiophene into contact with a reducing agent in the presence or absence of an appropriate solvent. In general, dispersibility of polythiophene in an organic solvent is sufficiently improved by a reduction treatment under relatively mild conditions such as stirring polythiophene in 28% ammonia water (for example, at room temperature overnight).

In the case of sulfonated polythiophenes, if necessary, it may be converted into an ammonium salt corresponding to the sulfonated polythiophene, for example, a trialkyl ammonium salt (a sulfonated polythiophene adduct), and then subjected to a reduction treatment.

As a result of changing the dispersibility of polythiophene in a solvent by the reduction treatment, the polythiophene which was not dissolved in a reaction system at the beginning of the treatment may be dissolved at the completion of the treatment. In such a case, polythiophene can be recovered by a method such as adding polythiophene and an incompatible organic solvent (in the case of sulfonated polythiophene, acetone, isopropyl alcohol, and the like) to a reaction system to cause precipitation of polythiophene and filtering the precipitated polythiophene.

In the present invention, a ratio of the onium borate salt to the charge-transporting material can be the charge-transporting material: the onium borate salt = about 1 : 0.1 to 10, as a substance amount (mole) ratio.

Further, in the present invention, a ratio of the onium borate salt to the charge-transporting material can be the charge-transporting material: the onium borate salt = about 1 : 0.01 to 20, preferably about 1 : 0.01 to 10, more preferably about 1 : 0.01 to 2, still more preferably about 1 : 0.1 to 2, and most preferably 1 : 0.15 to 1, as a mass ratio.

As the organic solvent used when preparing the charge-transporting varnish, a highly soluble solvent capable of dissolving the charge-transporting material and the onium borate salt well can be used.

Examples of such a highly soluble solvent include organic solvents such as cyclohexanone, N,N-dimethylformamide, N,N-dimethylacetamide, N-methyl pyrrolidone, 1,3-dimethyl-2-imidazolidinone, diethylene glycol monomethyl ether, 3-phenoxytoluene, 4-methoxytoluene, toluene, anisole, cyclohexylbenzene, methyl benzoate, tetralin, and isophorone, but are not limited thereto. These solvents can be used alone or in combination of two or more, and the use amount thereof can be 5 to 100% by weight per the entire solvent used in the varnish.

Further, in the present invention, the varnish includes at least one high-viscosity solvent having a viscosity of 10 to 200 mPa·s, particularly 35 to 150 mPa·s at 25°C and a boiling point of 50 to 300°C, particularly 150 to 250°C under normal pressure (atmospheric pressure), whereby it is easy to adjust the viscosity of the varnish, and as a result, it is possible to prepare a varnish according to an application method, which provides a thin film having high flatness with good producibility.

Examples of the high-viscosity organic solvent include cyclohexanol, ethylene glycol, ethylene glycol diglycidyl ether, 1,3-octylene glycol, diethylene glycol, dipropylene glycol, triethylene glycol, tripropylene glycol, 1,3-butanediol, 2,3-butanediol, 1,4-butanediol, propylene glycol, hexylene glycol, and the like, but are not limited thereto. These solvents may be used alone or in combination of two or more.

The addition ratio of the high-viscosity organic solvent to the entire solvent used in the varnish of the present invention is preferably in a range in which solids are not precipitated, and the addition ratio in the range in which solids are not precipitated is preferably 5 to 90% by weight.

In addition, for the purpose of improving wettability to a substrate, adjusting surface tension of the solvent, adjusting polarity, adjusting a boiling point, and the like, other solvents can be mixed at a ratio of 1 to 90% by weight, and preferably 1 to 50% by weight per the entire solvent used in the varnish.

Examples of such a solvent include propylene glycol monomethyl ether, ethylene glycol monobutyl ether, diethylene glycol diethyl ether, diethylene glycol dimethyl ether, diethylene glycol monoethyl ether acetate, diethylene glycol monobutyl ether acetate, dipropylene glycol monomethyl ether, propylene glycol monomethyl ether acetate, diethylene glycol monoethyl ether, diacetone alcohol, γ-butyrolactone, ethyl lactate, n-hexyl acetate, and the like, but are not limited thereto. These solvents can be used alone or in combination of two or more.

The viscosity of the varnish of the present invention is appropriately set depending on the thickness and the like of the thin film to be manufactured and the solid content concentration, but usually 1 to 50 mPa·s at 25°C, and the surface tension thereof is usually 20 to 50 mN/m.

Further, the solid content concentration of the charge-transporting varnish is appropriately set in consideration of the viscosity, surface tension, and the like of the varnish, the thickness of the thin film to be manufactured, and the like, but usually about 0.1 to 10.0% by weight, and considering improving applicability of the varnish, the solid content concentration is preferably about 0.5 to 5.0% by weight, and more preferably about 1.0 to 3.0% by weight.

The preparation method of the varnish is not particularly limited, but examples thereof include a method in which the onium borate salt is first dissolved in a solvent and a charge-transporting material is sequentially added thereto, and a method in which a mixture of the onium borate salt and a charge-transporting material is dissolved in a solvent.

Further, if there are a plurality of organic solvents, for example, the onium borate salt and the charge-transporting material may be first dissolved in a solvent which dissolves them well, other solvents may be added thereto, and the onium borate salt and the charge-transporting material may be dissolved in a mixed solvent of a plurality of organic solvents, sequentially or simultaneously.

In the present invention, from a viewpoint of obtaining a thin film having high flatness with good reproducibility, it is desirable that the charge-transporting varnish is prepared by dissolving the onium borate salt, the charge-transporting compound, and the like in an organic solvent, and then performing filtration using a filter of a submicro-order and the like.

The charge-transporting thin film of the present invention can be formed on a substrate by applying the above-described charge-transporting varnish on the substrate and baking the applied varnish.

The method of applying the varnish is not particularly limited, but examples thereof include a dip method, a spin coating method, a transfer printing method, a roll coating method, a brush coating method, an inkjet method, a spray method, a slit coating method, and the like, and it is preferred to adjust the viscosity and surface tension of the varnish depending on the application method.

Further, if the varnish of the present invention is used, the baking atmosphere is also not particularly limited, and a thin film having a uniform film formation surface and high charge transportability can be obtained not only in the air atmosphere but also in an inert gas such as nitrogen or in vacuum, but depending on the type of charge-transporting compounds and the like, a thin film having higher charge transportability can be obtained with good reproducibility, by baking the varnish under the air atmosphere.

A baking temperature is appropriately set within a range of about 100 to 260°C, considering the use of the obtained thin film, the charge transportability degree which is imparted to the obtained thin film, a solvent type, a boiling point, and the like, and if the obtained thin film is used as a hole injection layer of an organic EL device, about 140 to 250°C is preferred and about 145 to 240°C is more preferred.

In addition, at the time of baking, for the purpose of expressing a film having higher uniformity or proceeding with a reaction on the substrate, two or more stages of temperature change may be applied and heating may be performed using an appropriate device such as for example, a hot plate or an oven.

The thickness of the charge-transporting thin film is not particularly limited, but is preferably 5 to 200 nm, if the thin film is used as a hole injection layer, a hole transport layer, or a hole injection/transport layer of an organic EL device. As a method of changing the film thickness, there are methods such as changing a solid content concentration in the varnish or changing a solution amount on a substrate at the time of application.

The organic EL device of the present invention has a pair of electrodes and has the charge-transporting thin film of the present invention between the electrodes.

The typical configurations of the organic EL device include the following (a) to (f), but are not limited thereto. In addition, in the following configuration, if necessary, an electron block layer and the like can be provided between the light-emitting layer and a positive electrode, and a hole block layer and the like can be provided between the light-emitting layer and a negative electrode. Further, a hole injection layer, a hole transport layer, or a hole injection/transport layer may have a function as the electron block layer and the like, and an electron injection layer, an electron transport layer, or an electron injection/transport layer may have a function as the hole block layer and the like.
(a) positive electrode/hole injection layer/hole transport layer/light-emitting layer/electron transport layer/electron injection layer/negative electrode
(b) positive electrode/hole injection layer/hole transport layer/light-emitting layer/electron injection/transport layer/negative electrode
(c) positive electrode/hole injection/transport layer/light-emitting layer/electron transport layer/electron injection layer/negative electrode
(d) positive electrode/hole injection/transport layer/light-emitting layer/electron injection/ transport layer/negative electrode
(e) positive electrode/hole injection layer/hole transport layer/light-emitting layer/negative electrode
(f) positive electrode/hole injection/transport layer/light-emitting layer/negative electrode

The "hole injection layer", the "hole transport layer", and the "hole injection/transport layer" are layers formed between the light-emitting layer and the positive electrode and have a function of transporting holes from the positive electrode to the light-emitting layer, and if only one layer of hole transporting materials is provided between the light-emitting layer and the positive electrode, it is the "hole injection/transport layer", and if two or more layers of hole transporting materials are provided between the light-emitting layer and the positive electrode, the layer near the positive electrode is the "hole injection layer" and the other layer is the "hole transport layer". In particular, as the hole injection (transport) layer, a thin film which is excellent not only in a hole-accepting property from the positive electrode but also in a hole injecting property to the hole transport (light emitting) layer is used.

The "electron injection layer", the "electron transport layer", and the "electron injection/transport layer" are layers formed between the light-emitting layer and the negative electrode and have a function of transporting electrons from the negative electrode to the light-emitting layer, and if only one layer of hole transporting materials is provided between the light-emitting layer and the negative electrode, it is the "electron injection/transport layer", and if two or more layers of electron transporting materials are provided between the light-emitting layer and the negative electrode, the layer near the negative electrode is the "electron injection layer" and the other layer is the "electron transport layer".

The "light-emitting layer" is an organic layer having a light emitting function, and includes host materials and dopant materials if a doping system is employed. In this case, the host materials promote mainly a recombination of electrons and holes, and has a function of confining excitons in the light-emitting layer, and the dopant materials have a function of efficiently emitting excitons obtained by the recombination. In the case of a phosphorescent element, the host materials mainly have a function of confining excitons produced by a dopant in the light-emitting layer.

The charge-transporting thin film of the present invention can be suitably used as the hole injection layer, the hole transport layer, and the hole injection/transport layer in the organic EL device, and can be more suitably used as the hole injection layer.

Examples of the materials used and the manufacturing method when manufacturing the organic EL device using the charge-transporting varnish of the present invention include the following, but are not limited thereto.

It is preferred that the electrode substrate to be used is cleaned in advance by a liquid cleaning with a detergent, alcohol, pure water, and the like, and for example, it is preferred to perform surface treatment such as a UV ozone treatment and an oxygen-plasma treatment immediately prior to use on the positive electrode substrate. However, if the positive electrode materials include an organic material as a main component, surface treatment may not be performed.

An example of a method of manufacturing the organic EL device having the hole injection layer including the thin film obtained from the charge-transporting varnish of the present invention is as follows.

The charge-transporting varnish of the present invention is applied on a positive electrode substrate and baked by the above-described method and the hole injection layer on the electrode is manufactured.

On the hole injection layer, a hole transport layer, a light-emitting layer, an electron transport layer, an electron injection layer, and a negative electrode are provided in this order. The hole transport layer, the light-emitting layer, the electron transport layer, and the electron injection layer may be formed by either a vapor deposition method or a coating method (wet process), depending on the properties of the materials to be used and the like.

Examples of the positive electrode materials include a transparent electrode represented by indium tin oxide (ITO) or indium zinc oxide (IZO), or a metal positive electrode including a metal represented by aluminum, an alloy thereof, or the like, and it is preferred to perform a flattening treatment. A polythiophene derivative or a polyaniline derivative having high charge transportability can also be used.

In addition, examples of other metals forming the metal positive electrode include scandium, titanium, vanadium, chromium, manganese, iron, cobalt, nickel, copper, zinc, gallium, yttrium, zirconium, niobium, molybdenum, ruthenium, rhodium, palladium, cadmium, indium, scandium, lanthanum, cerium, praseodymium, neodymium, promethium, samarium, europium, gadolinium, terbium, dysprosium, holmium, erbium, thulium, ytterbium, hafnium, thallium, tungsten, rhenium, osmium, iridium, platinum, gold, titanium, lead, bismuth, an alloy thereof, and the like, but are not limited thereto.

Examples of the materials forming the hole transport layer include hole-transporting low molecular weight materials, for example, triarylamines such as a (triphenylamine)dimer derivative, a [(triphenylamine)dimer]spirodimer, N,N'-bis(naphthalen-1-yl)-N,N'-bis(phenyl)-benzidine (α-NPD), N,N'-bis(naphthalen-2-yl)-N,N'-bis(phenyl)-benzidine, N,N'-bis(3-methylphenyl)-N,N'-bis(phenyl)-benzidine,N,N'-bis(3-methylphenyl)-N,N'-bis(phenyl)-9,9-spirobifluorene, N,N'-bis(naphthalene-1-yl)-N,N'-bis(phenyl)-9,9-spirobifluorene, N,N'-bis(3-methylphenyl)-N,N'-bis(phenyl)-9,9-dimethyl-fluorene, N,N'-bis(naphthalen-1-yl)-N,N'-bis(phenyl)-9,9-dimethyl-fluorene, N,N'-bis(3-methylphenyl)-N,N'-bis(phenyl)-9,9-diphenyl-fluorene, N,N'-bis(naphthalen-1-yl)-N,N'-bis(phenyl)-9,9-diphenyl-fluorene, N,N'-bis(naphthalen-1-yl)-N,N'-bis(phenyl)-2,2'-dimethylbenzidine,2,2',7,7'-tetrakis(N,N-diphenylamino)-9,9-spirobifluorene, 9,9-bis[4-(N,N-bis-biphenyl-4-yl-amino)phenyl]-9H-fluorene, 9,9-bis[4-(N,N-bis-naphthalen-2-yl-amino)phenyl]-9H-fluorene, 9,9-bis[4-(N-naphthalen-1-yl-N-phenylamino)-phenyl]-9H-fluorene, 2,2',7,7'-tetrakis[N-naphthalenyl(phenyl)-amino]-9,9-spirobifluorene, N,N'-bis(phenanthrene-9-yl)-N,N'-bis(phenyl)-benzidine,2,2'-bis[N,N-bis(biphenyl-4-yl)amino]-9,9-spirobifluorene, 2,2'-bis(N,N-diphenylamino)-9,9-spirobifluorene, di-[4-(N,N-di(p-tolyl)amino)-phenyl]cyclohexane, 2,2',7,7'-tetra(N,N-di(p-tolyl))amino-9,9-spirobifluorene, N,N,N',N'-tetra-naphthalen-2-yl-benzidine, N,N,N',N'-tetra-(3 - methylphenyl)-3,3'-dimethylbenzidine, N,N'-di(naphthalenyl)-N,N'-di(naphthalen-2-yl)-benzidine, N,N,N',N'-tetra(naphthalenyl)-benzidine, N,N'-di(naphthalen-2-yl)-N,N'-diphenylbenzidine-1,4-diamine, N¹,N⁴-diphenyl-N¹,N⁴-di(m-tolyl)benzene-1,4-diamine, N²,N²,N⁶,N⁶-tetraphenylnaphthalen-2,6-diamine, tris(4-(quinoline-8-yl)phenyl)amine, 2,2'-bis(3-(N,N-di(p-tolyl)amino)phenyl)biphenyl, 4,4',4"-tris[3-methylphenyl(phenyl)-amino]triphenylamine (m-MTDATA), and 4,4',4"-tris[1-naphthyl(phenyl)amino]triphenylamine (1-TNATA), oligothiophenes such as 5,5"-bis-{4-[bis(4-methylphenyl)amino]-phenyl}-2,2':5',2"-terthiophene (BMA-3T), and the like.

Examples of the materials forming the light-emitting layer include tris(8-quinolinolat)aluminum(III) (Alq₃), bis(8-quinolinolat)zinc(II) (Znq₂), bis(2-methyl-8-quinolinolat)-4-(p-phenylphenolat)aluminum(III) (BAlq), 4,4'-bis(2,2-diphenylvinyl)biphenyl, 9,10-di(naphthalen-2-yl)anthracene, 2-t-butyl-9,10-di(naphthalen-2-yl)anthracene, 2,7-bis[9,9-di(4-methylphenyl)-fluorene-2-yl]-9,9-di(4-methylphenyl)fluorene, 2-methyl-9,10-bis(naphthalen-2-yl)anthracene, 2-(9,9-spirobifluorene-2-yl)-9,9-spirobifluorene,2,7-bis(9,9-spirobifluorene-2-yl)-9,9-spirobifluorene, 2-[9,9-di(4-methylphenyl)-fluorene-2-yl]-9,9-di(4-methylphenyl)fluorene, 2,2'-dipyrenyl-9,9-spirobifluorene, 1,3,5-tris(pyrene-1-yl)benzene, 9,9-bis[4-(pyrenyl)-phenyl]-9H-fluorene, 2,2'-bi(9,10-diphenylanthracene), 2,7-dipyrenyl-9,9-spirobifluorene, 1,4-di(pyrene-1-yl)benzene, 1,3-di(pyrene-1-yl)benzene, 6,13-di(biphenyl-4-yl)pentacene, 3,9-di(naphthalen-2-yl)perylene, 3,10-di(naphthalen-2-yl)perylene, tris[4-(pyrenyl)-phenyl]amine, 10,10'-di(biphenyl-4-yl)-9,9'-bianthracene,N,N'-di(naphthalen-1-yl)-N,N'-diphenyl-[1,1':4',1":4",1"'-quarterphenyl]-4,4"'-diamine, 4,4'-di[10-(naphthalen-1-yl)anthracen-9-yl]biphenyl, dibenzo{[f,f']-4,4',7,7'-tetraphenyl}diindeno[1,2,3-cd:1',2',3'-lm]perylene, 1-(7-(9,9'-bianthracen-10-yl)-9,9-dimethyl-9H-fluorene-2-yl)pyrene, 1-(7-(9,9'-bianthracen-10-yl)-9,9-dihexyl-9H-fluorene-2-yl)pyrene, 1,3-bis(carbazole-9-yl)benzene, 1,3,5-tris(carbazole-9-yl)benzene, 4,4',4"-tris(carbazole-9-yl)triphenylamine, 4,4'-bis(carbazole-9-yl)biphenyl (CBP), 4,4'-bis(carbazole-9-yl)-2,2'-dimethylbiphenyl, 2,7-bis(carbazole-9-yl)-9,9-dimethylfluorene, 2,2',7,7'-tetrakis(carbazole-9-yl)-9,9-spirobifluorene, 2,7-bis(carbazole-9-yl)-9,9-di(p-tolyl)fluorene, 9,9-bis[4-(carbazole-9-yl)-phenyl]fluorene, 2,7-bis(carbazole-9-yl)-9,9-spirobifluorene, 1,4-bis(triphenylsilyl)benzene, 1,3-bis(triphenylsilyl)benzene, bis(4-N,N-diethylamino-2-methylphenyl)-4-methylphenyl-methane, 2,7-bis(carbazole-9-yl)-9,9-dioctylfluorene, 4,4"-di(triphenylsilyl)-p-terphenyl, 4,4'-di(triphenylsilyl)biphenyl, 9-(4-t-butylphenyl)-3,6-bis(triphenylsilyl)-9H-carbazole, 9-(4-t-butylphenyl)-3,6-ditrityl-9H-carbazole, 9-(4-t-butylphenyl)-3,6-bis(9-(4-methoxyphenyl)-9H-fluorene-9-yl)-9H-carbazole, 2,6-bis(3-(9H-carbazole-9-yl)phenyl)-pyridine, triphenyl(4-(9-phenyl-9H-fluorene-9-yl)phenyl)silane, 9,9-dimethyl-N,N-diphenyl-7-(4-(1-phenyl-1H-benzo[d]imidazol-2-yl)phenyl)-9H-fluorene-2-amine, 3,5-bis(3-(9H-carbazole-9-yl)phenyl)pyridine, 9,9-spirobifluorene-2-yl-diphenylphosphine oxide, 9,9'-(5-(triphenylsilyl)-1,3-phenylene)bis(9H-carbazole), 3-(2,7-bis(diphenylphosphoryl)-9-phenyl-9H-fluorene-9-yl)-9-phenyl-9H-carbazole, 4,4,8,8,12,12-hexa(p-tolyl)-4H-8H-12H-12C-azadibenzo[cd,mn]pyrene, 4,7-di(9H-carbazole-9-yl)-1,10-phenanthroline, 2,2'-bis(4-(carbazole-9-yl)phenyl)biphenyl, 2,8-bis(diphenylphosphoryl)dibenzo[b,d]thiophene, bis(2-methylphenyl)diphenylsilane, bis[3,5-di(9H-carbazole-9-yl)phenyl]diphenylsilane, 3,6-bis(carbazole-9-yl)-9-(2-ethylhexyl)-9H-carbazole, 3-(diphenylphosphoryl)-9-(4-(diphenylphosphoryl)phenyl)-9H-carbazole, 3,6-bis[(3,5-diphenyl)phenyl]-9-phenylcarbazole, and the like, and the light-emitting layer may be formed by co-deposition with a light-emitting dopant.

Examples of the light-emitting dopant include 3-(2-benzothiazolyl)-7-(diethylamino)coumarin, 2,3,6,7-tetrahydro-1,1,7,7-tetramethyl-1H,5H,11H-10-(2-benzothiazolyl)quinolizino[9,9a,1gh]coumarin, quinacridone, N,N'-dimethyl-quinacridone, tris(2-phenylpyridine)iridium(III)(Ir(ppy)₃), bis(2-phenylpyridine)(acetylacetonate)iridium(III)(Ir(ppy)₂(acac)), tris[2-(p-tolyl)pyridine]iridium(III)(Ir(mppy)₃), 9,10-bis[N,N-di(p-tolyl)amino]anthracene, 9,10-bis[phenyl(m-tolyl)amino]anthracene, bis[2-(2-hydroxyphenyl)benzothiazolate]zinc(II), N¹⁰,N¹⁰,N^{10'},N^{10'}-tetra(p-tolyl)-9,9'-bianthracen-10,10'-diamine, N¹⁰,N¹⁰,N^{10'},N^{10'}-tetraphenyl-9,9'-bianthracen-10,10'-diamine, N¹⁰,N^{10'}-diphenyl-N¹⁰,N^{10'}-dinaphthalenyl-9,9'-bianthracen-10,10'-diamine, 4,4'-bis(9-ethyl-3-carbazovinylene)-1,1'-biphenyl, perylene, 2,5,8,11-tetra-t-butylperylene, 1,4-bis[2-(3-N-ethylcarbazolyl)vinyl]benzene, 4,4'-bis[4-(di-p-tolylamino)styryl]biphenyl, 4-(di-p-tolylamino)-4'-[(di-p-tolylamino)styryl]stilbene, bis[3,5-difluoro-2-(2-pyridyl)phenyl-(2-carboxypyridyl)]iridium(III), 4,4'-bis[4-(diphenylamino)styryl]biphenyl, bis(2,4-difluorophenylpyridinato)tetrakis(1-pyrazolyl)borate iridium(III), N,N'-bis(naphthalen-2-yl)-N,N'-bis(phenyl)-tris(9,9-dimethylfluorenylene), 2,7-bis{2-[phenyl(m-tolyl)amino]-9,9-dimethyl-fluorene-7-yl}-9,9-dimethyl-fluorene, N-(4-((E)-2-(6((E)-4-(diphenylamino)styryl)naphthalen-2-yl)vinyl)phenyl)-N-phenylbenzeneamine, fac-iridium(III)tris(1-phenyl-3-methylbenzimidazolin-2-ylidene-C,C^{2,}),mer-iridium(III)tris(1-phenyl-3-methylbenzimidazolin-2-ylidene-C,C^{2,}), 2,7-bis[4-(diphenylamino)styryl]-9,9-spirobifluorene,6-methyl-2-(4-(9-(4-(6-methylbenzo[d]thiazol-2-yl)phenyl)anthracen-10-yl)phenyl)benzo[d]thiazole, 1,4-di[4-(N,N-diphenyl)amino]styrylbenzene, 1,4-bis(4-(9H-carbazole-9-yl)styryl)benzene, (E)-6-(4-(diphenylamino)styryl)-N,N-diphenylnaphthalen-2-amine, bis(2,4-difluorophenylpyridinato)(5-(pyridine-2-yl)-1H-tetrazolate)iridium(III), bis(3-trifluoromethyl-5-(2-pyridyl)pyrazole)((2,4-difluorobenzyl)diphenylphosphinate)-iridium(III), bis(3-trifluoromethyl-5-(2-pyridyl)pyrazolate)(benzyldiphenylphosphinate)-iridium(III), bis(1-(2,4-difluorobenzyl)-3-methylbenzimidazolium)(3-(trifluoromethyl)-5-(2-pyridyl)-1,2,4-triazolate)iridium(III), bis(3-trifluoromethyl-5-(2-pyridyl)pyrazolate)-(4',6'-difluorophenylpyridinate)iridium(III), bis(4',6'-difluorophenylpyridinato)(3,5-bis(trifluoromethyl)-2-(2'-pyridyl)pyrolate)iridium(III), bis(4',6'-difluorophenyl-pyridinato)(3-(trifluoromethyl)-5-(2-pyridyl)-1,2,4-triazolate)iridium(III), (Z)-6-mesityl-N-(6-mesitylquinoline-2(1H)-ylidene)quinoline-2-amine-BF₂, (E)-2-(2-(4-(dimethylamino)-styryl)-6-methyl-4H-pyrane-4-ylidene)malononitrile, 4-(dicyanomethylene)-2-methyl-6-julolidine-9-enyl-4H-pyran, 4-(dicyanomethylene)-2-methyl-6-(1,1,7,7-tetramethyl-julolidyl-9-enyl)-4H-pyran, 4-(dicyanomethylene)-2-t-butyl-6-(1,1,7,7-tetramethyl-julolidine-4-yl-vinyl)-4H-pyran, tris(dibenzoylmethane)phenanthroline europium(III), 5,6,11,12-tetraphenylnaphthacene, bis(2-benzo[b]thiophene-2-yl-pyridine)(acetylacetonate)iridium(III), tris(1-phenylisoquinoline)iridium(III), bis(1-phenylisoquinoline)(acetylacetonate)iridium(III), bis[1-(9,9-dimethyl-9H-fluorene-2-yl)-isoquinoline](acetylacetonate)iridium(III), bis[2-(9,9-dimethyl-9H-fluorene-2-yl)quinoline](acetylacetonate)iridium(III), tris[4,4'-di-t-butyl-(2,2')-bipyridine]-ruthenium(III) bis(hexafluorophosphate), tris(2-phenylquinoline)iridium(III), bis(2-phenylquinoline)(acetylacetonate)iridium(III), 2,8-di-t-butyl-5,11-bis(4-t-butylphenyl)-6,12-diphenyltetracene, bis(2-phenylbenzothiazolate)(acetylacetonate)iridium(III), 5,10,15,20-tetraphenyltetrabenzoporphyrin platinum, osmium(II)bis(3-trifluoromethyl-5-(2-pyridine)-pyrazolate)dimethylphenylphosphine, osmium(II)bis(3-(trifluoromethyl)-5-(4-t-butylpyridyl)-1,2,4-triazolate)diphenylmethylphosphine, osmium(II)bis(3-(trifluoromethyl)-5-(2-pyridyl)-1,2,4-triazole)dimethylphenylphosphine, osmium(II)bis(3-(trifluoromethyl)-5-(4-t-butylpyridyl)-1,2,4-triazolat)dimethylphenyl-phosphine, bis[2-(4-n-hexylphenyl)quinoline](acetylacetonate)iridium(III), tris[2-(4-n-hexylphenyl)quinoline]iridium(III), tris[2-phenyl-4-methylquinoline]iridium(III), bis(2-phenylquinoline)(2-(3-methylphenyl)pyridinate)iridium(III), bis(2-(9,9-diethyl-fluorene-2-yl)-1-phenyl-1H-benzo[d]imidazolat)(acetylacetonate)iridium(III), bis(2-phenylpyridine)(3-(pyridine-2-yl)-2H-chromen-2-onate)iridium(III), bis(2-phenylquinoline)(2,2,6,6-tetramethytheptane-3,5-dionate)iridium(III), bis(phenylisoquinoline)(2,2,6,6-tetramethylheptane-3,5-dionate)iridium(III), iridium(III)bis(4-phenylthieno[3,2-c]pyridinato-N,C^{2'})acetylacetonate, (E)-2-(2-t-butyl-6-(2-(2,6,6-trimethyl-2,4,5,6-tetrahydro-1H-pyrrolo[3,2,1-ij]quinoline-8-yl)vinyl)-4H-pyran-4-ylidene)malononitrile, bis(3-trifluoromethyl-5-(1-isoquinolyl)pyrazolate)(methyldiphenylphosphine)ruthenium, bis[(4-n-hexylphenyl)isoquinoline](acetylacetonate)iridium(III), platinum(II)octaethylporphine, bis(2-methyldibenzo [f,h] quinoxaline)(acetylacetonate)iridium(III), tris[(4-n-hexylphenyl)quinoquinoline]iridium(III), and the like.

Examples of the materials forming the electron transport layer include 8-hydroxyquinolinolate-lithium, 2,2',2"-(1,3,5-benzinetolyl)-tris(1-phenyl-1-H-benzimidazole), 2-(4-biphenyl)5-(4-t-butylphenyl)-1,3,4-oxadiazole, 2,9-dimethyl-4,7-diphenyl-1,10-phenanthroline, 4,7-diphenyl-1,10-phenanthroline, bis(2-methyl-8-quinolinolat)-4-(phenylphenolat)aluminum, 1,3-bis[2-(2,2'-bipyridine-6-yl)-1,3,4-oxadiazol-5-yl]benzene, 6,6'-bis[5-(biphenyl-4-yl)-1,3,4-oxadiazol-2-yl]-2,2'-bipyridine, 3-(4-biphenyl)-4-phenyl-5-t-butylphenyl-1,2,4-triazole, 4-(naphthalen-1-yl)-3,5-diphenyl-4H-1,2,4-triazole, 2,9-bis(naphthalen-2-yl)-4,7-diphenyl-1,10-phenanthroline, 2,7-bis[2-(2,2'-bipyridine-6-yl)-1,3,4-oxadiazol-5-yl]-9,9-dimethylfluorene, 1,3-bis[2-(4-t-butylphenyl)-1,3,4-oxadiazol-5-yl]benzene, tris(2,4,6-trimethyl-3-(pyridine-3-yl)phenyl)boran, 1-methyl-2-(4-(naphthalen-2-yl)phenyl)-1H-imidazo[4,5f][1,10]-phenanthroline, 2-(naphthalen-2-yl)-4,7-diphenyl-1,10-phenanthroline, phenyl-dipyrenylphosphineoxide, 3,3',5,5'-tetra[(m-pyridyl)-phen-3-yl]biphenyl, 1,3,5-tris[(3-pyridyl)-phen-3-yl]benzene,4,4'-bis(4,6-diphenyl-1,3,5-triazin-2-yl)biphenyl, 1,3-bis[3,5-di(pyridine-3-yl)phenyl]benzene, bis(10-hydroxybenzo[h]quinolinato)beryllium, diphenylbis(4-(pyridine-3-yl)phenyl)silane, 3,5-di(pyrene-1-yl}pyridine, and the like.

Examples of the materials forming the electron injection layer includes lithium oxide (Li₂O), magnesium oxide (MgO), alumina (Al₂O₃), lithium fluoride (LiF), sodium fluoride (NaF), magnesium fluoride (MgF₂), cesium fluoride (CsF), strontium fluoride (SrF₂), molybdenum trioxide (MoO₃), aluminum, Li(acac), lithium acetate, lithium benzoate, and the like.

Examples of the negative electrode materials include aluminum, a magnesium-silver alloy, an aluminum-lithium alloy, lithium, sodium, potassium, cesium, and the like.

Further, other examples of the method of manufacturing the organic EL device having a hole injection layer including the thin film obtained from the charge-transporting varnish of the present invention are as follows.

In the manufacture of the EL device, instead of performing a vacuum deposition operation of a hole transport layer, a light-emitting layer, an electron transport layer, and an electron injection layer, an organic EL device having a charge-transporting thin film formed by the charge-transporting varnish of the present invention can be manufactured, by sequentially forming the hole transport layer (hereinafter, referred to as a hole-transporting polymer layer) and the light-emitting layer (hereinafter, referred to as a light-emitting polymer layer).

Specifically, the charge-transporting varnish of the present invention is applied on a positive electrode substrate to manufacture a hole injection layer by the above-described method, a hole-transporting polymer layer and a light-emitting polymer layer are sequentially formed thereon, and a negative electrode is further deposited to form an organic EL device.

As the positive electrode and negative electrode materials to be used, those as described above are used, and the same cleaning treatment and surface treatment can be performed.

Examples of the method of forming the hole-transporting polymer layer and the light-emitting polymer layer include a method in which a solvent is added to hole-transporting polymer materials or light-emitting polymer materials, or materials to which a dopant material is added, which are then dissolved or uniformly dispersed therein, and applied on a hole injection layer or a hole transporting polymer layer and each baked, thereby forming a thin film.

Examples of the hole-transporting polymer materials include poly[(9,9-dihexylfluorenyl-2,7-diyl)-co-(N,N'-bis{p-butylphenyl}-1,4-diaminophenylene)], poly[(9,9-dioctylfluorenyl-2,7-diyl)-co-(N,N'-bis{p-butylphenyl}-1,1'-biphenylene-4,4-diamine)], poly[(9,9-bis{1'-penten-5'-yl}fluorenyl-2,7-diyl)-co-(N,N'-bis{p-butylphenyl}-1,4-diaminophenylene)], poly[N,N'-bis(4-butylphenyl)-N,N'-bis(phenyl)-benzidine]-end capped with polysilsesquinoxane, poly[(9,9-didioctylfluorenyl-2,7-diyl)-co-(4,4'-(N-(p-butylphenyl))diphenylamine)], and the like.

Examples of the light-emitting polymer layer materials include polyfluorene derivatives such as poly(9,9-dialkylfluorene) (PDAF), polyphenylenevinylene derivatives such as poly(2-methoxy-5-(2'-ethylhexoxy)-1,4-phenylenevinylene) (MEH-PPV), polythiophene derivatives such as poly(3-alkylthiophene) (PAT), polyvinylcarbazole (PVCz), and the like.

Examples of the solvent include toluene, xylene, chloroform, and the like, and examples of a dissolution or uniform dispersion method include methods such as stirring, heating and stirring, and ultrasonic dispersion.

The application method is not particularly limited, but examples thereof include an inkjet method, a spray method, a dip method, a spin coating method, a transfer printing method, a roll coating method, a brush coating method, and the like. In addition, it is preferred that the application is performed under an inert gas such as nitrogen or argon.

Examples of the baking method include a method of heating in an oven or a hot plate under an inert gas or under a vacuum.

An example of a method of manufacturing the EL device having the hole transport layer including the thin film obtained from the charge-transporting varnish of the present invention is as follows:
A hole injection layer is formed on a positive electrode substrate. On the layer, the charge-transporting varnish of the present invention is applied and baked by the above-described method, thereby manufacturing a hole transport layer.

On the hole transport layer, a light-emitting layer, an electron transport layer, an electron injection layer, and a negative electrode are provided in this order. A method of forming the light-emitting layer, the electron transport layer, and the electron injection layer, and specific examples thereof are as described above. Further, the hole injection layer may be formed by either a vapor deposition method or a coating method (wet process), depending on the properties of the materials to be used and the like.

Examples of the materials forming the hole injection layer include copper phthalocyanine, titanium oxide phthalocyanine, platinum phthalocyanine, pyrazino[2,3-f][1,10]phenanthroline-2,3-dicarbonitrile, N,N,N',N'-tetrakis(4-methoxyphenyl)benzidine, 2,7-bis[N,N-bis(4-methoxy-phenyl)amino]-9,9-spirobifluorene, 2,2'-bis[N,N-bis(4-methoxy-phenyl)amino]-9,9-spirobifluorene, N,N'-diphenyl-N,N'-di[4-(N,N-ditolylamino)phenyl]benzidine, N,N'-diphenyl-N,N'-di[4-(N,N-diphenylamino)phenyl]benzidine, N⁴,N^{4'}-(biphenyl-4,4'-diyl)bis(N⁴,N^{4'},N^{4'}-triphenylbiphenyl-4,4'-diamine)N¹,N^{1'}-(biphenyl-4,4'-diyl)bis(N¹-phenyl-N⁴,N⁴-di-m-tolylbenzene-1,4-diamine), charge-transporting materials described in WO 2004/043117, WO 2004/105446, WO 2005/000832, WO 2005/043962, WO 2005/042621, WO 2005/107335, WO 2006/006459, WO 2006/025342, WO 2006/137473, WO 2007/049631, WO 2007/099808, WO 2008/010474, WO 2008/032617, WO 2008/032616, WO 2008/129947, WO 2009/096352, WO 2010/041701, WO 2010/058777, WO 2010/058776, WO 2013/042623, WO 2013/129249, WO 2014/115865, WO 2014/132917, WO 2014/141998, and WO 2014/132834, and the like.

Examples of the positive electrode materials, the materials forming the light-emitting layer, the light-emitting dopant, the electron transport layer, and the electron block layer, and the negative electrode materials include those as described above.

An example of a method of manufacturing the organic EL device having a hole injection/transport layer including the thin film obtained from the charge-transporting varnish of the present invention is as follows.

On a positive electrode substrate, a hole injection/transport layer is formed, and on the hole injection/transport layer, a light-emitting layer, an electron transport layer, an electron injection layer, and a negative electrode are provided in this order. A method of forming the light-emitting layer, the electron transport layer, and the electron injection layer, and specific examples thereof are as described above.

Examples of the positive electrode materials, the materials forming the light-emitting layer, the light-emitting dopant, the electron transport layer, and the electron block layer, and the negative electrode materials include those as described above.

In addition, if necessary, a hole block layer, an electron block layer, and the like may be provided between the electrodes and each of the layers described above. Examples of the materials forming the electron block layer include tris(phenylpyrazole)iridium, and the like.

The materials constituting the positive electrode, the negative electrode, and the layer formed between the positive electrode and the negative electrode are different depending on whether the device is provided with a bottom emission structure or a top emission structure, and thus, appropriate materials are selected considering such a point.

Usually, in the device having the bottom emission structure, a transparent positive electrode is used on a substrate side and light is extracted from the substrate side, while in the device having the top emission structure, a reflective positive electrode composed of metal is used and light is extracted from the transparent electrode (negative electrode) in the opposite direction to the substrate, and thus, for example, in the case of the positive electrode materials, the transparent positive electrode such as ITO is used in manufacture of the device having the bottom emission structure and the reflective positive electrode such as Al/Nd is used in manufacture of the device having the top emission structure.

The organic EL device of the present invention may be sealed with a water-collecting agent and the like, if necessary, according to a standard method, for preventing deterioration of the characteristics.

### EXAMPLES

Hereinafter, the present invention is described in detail, with reference to the Examples and the Comparative Examples, but the present invention is not limited to the following Examples. In addition, apparatuses to be used are as follows.

| | |
|---|---|
| (1) ¹H, ¹⁹F-NMR: | a nuclear magnetic resonance apparatus AL-300 manufactured by JEOL Ltd. |
| (2) Substrate cleaning: | a substrate cleaning apparatus (low-pressure plasma system) manufactured by Choshu Industry Co., Ltd. |
| (3) Application of varnish: | Spincoater MS-A100 manufactured by Mikasa Co., Ltd. |
| (4) Film thickness measurement: | a fine shape measurement device Surf coder ET-400 manufactured by Kosaka Laboratory, Ltd. |
| (5) Surface observation of film: | a confocal laser microscope real time scanning laser microscope 1LM21D manufactured by Lasertec Corporation |
| (6) Manufacture of EL device: | a multifunctional deposition device system C-E2L1G1-N manufactured by Choshu Industry Co., Ltd. |
| (7) Measurement of brightness and the like of EL device: | a multi-channel IVL measurement device manufactured by EHC Co., Ltd. |
| (8) Lifetime measurement (brightness half-life measurement) of EL device: | an organic EL brightness lifetime evaluation system PEL-105S manufactured by EHC Co., Ltd. |
| (9) LDI-MS: | AutoFlex manufactured by Bruker Corporation |

### [1] Synthesis of onium borate salt

### [Synthesis Example 1] Synthesis of onium borate salt (P-3)

P-3 represented by the following formula was synthesized by the following method.

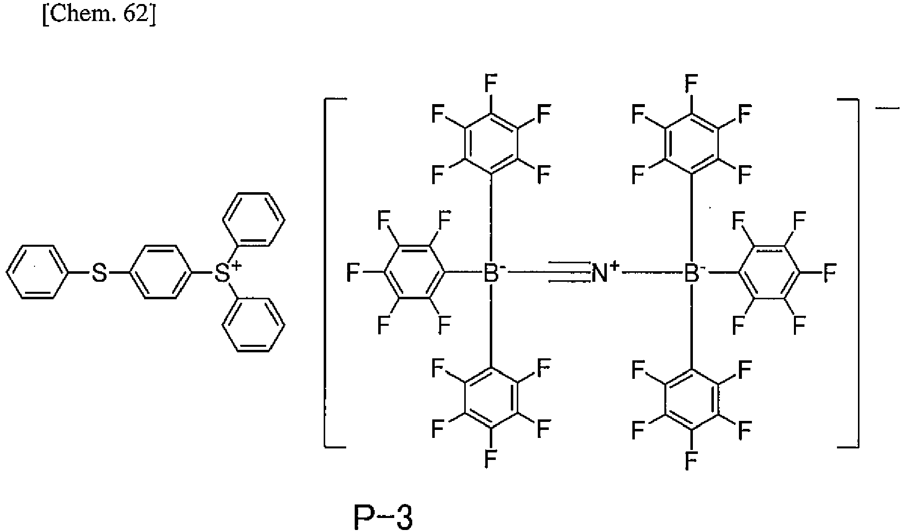

In a 10 L four neck flask, 6,068 mL of diethyl ether, 151.7 g of a compound represented by the following formula (Q-1), and 9.4 g of KCN were charged, and reacted at 34 to 36°C for 3 hours. After the reaction, atmospheric pressure concentration was performed, and 267.2 g of a brown liquid was obtained. The obtained brown liquid was concentrated with an evaporator at 55°C, and then dried under reduced pressure at 35°C for 16 hours, thereby obtaining an intermediate represented by formula (Q-2) which is 157.7 g of a pale brown solid. The obtained intermediate (Q-2) was identified by LDI-MS.
LDI-MS m/Z found: 1050.12 ([M]⁻ calcd: 1049.97)

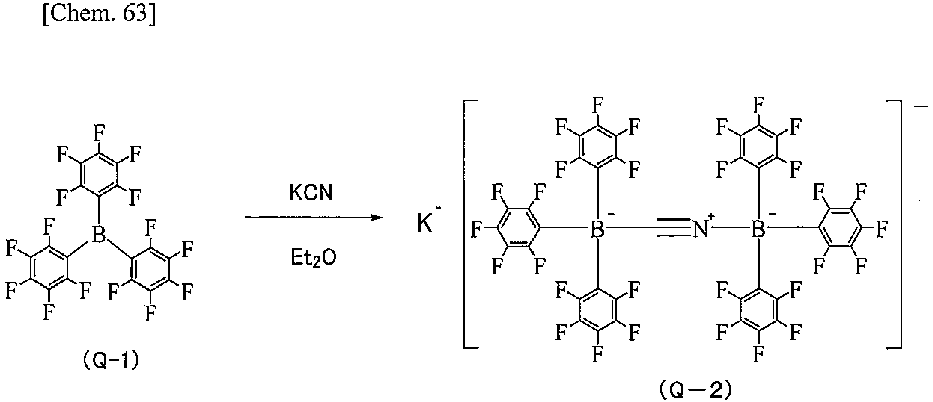

In a 300 mL three neck flask, 11.043 g of diphenyl[4-(phenylthio)phenyl]sulfonium trifluoromethane sulfonate, 22.000 g of the intermediate (Q-2), 110 mL of ion exchange water, and 110 mL of diethyl ether were charged, and reacted at 25°C for 16 hours. After the reaction, the content of the flask was transferred to a 300 mL separatory funnel and an aqueous layer was separated. An ether layer was cleaned five times with 100 mL of ion exchange water. The ether layer was concentrated at 40 to 45°C with an evaporator, and then dried under reduced pressure for 20 hours, thereby obtaining a target which is 24.000 g of a light yellow solid. The obtained target was identified by ¹H-NMR and LDI-MS.
¹H-NMR (300 MHz, DMSO-D6): δ 7.40 to 7.80 (19H, m)
LDI-MS m/Z found: 371.04 ([M]⁺ calcd: 371.09)
LDI-MS m/Z found: 1050.11 ([M]⁻ calcd: 1049.97)

### [Comparative Synthesis Example 1] Synthesis of P-4

P-4 represented by the following formula was synthesized according to the method described in WO 2006/025342.

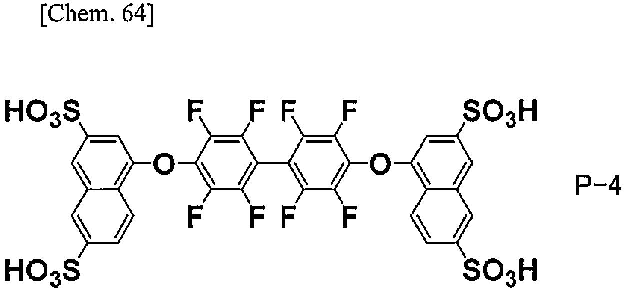

### [Comparative Synthesis Example 2] Synthesis of onium borate salt (P-1)

As P-1 represented by the following formula, one commercially available from Tokyo Chemical Industry Co., Ltd. was purchased and used.

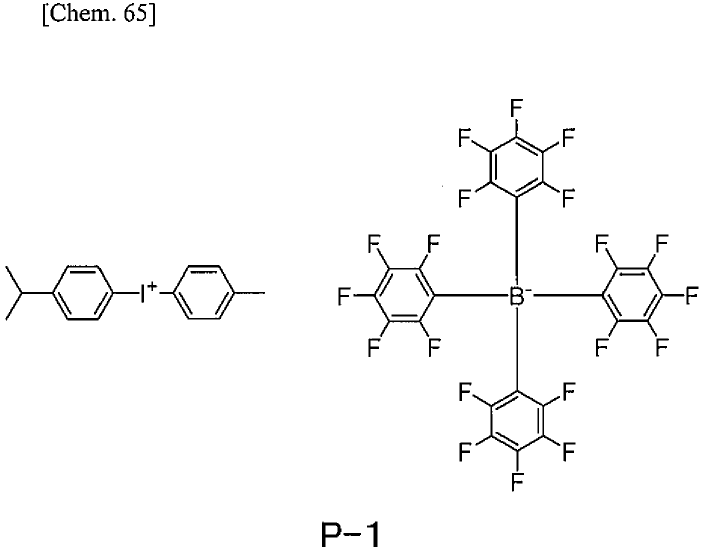

### [Comparative Synthesis Example 3] Synthesis of onium borate salt (P-2)

P-2 represented by the following formula was synthesized by the following method.

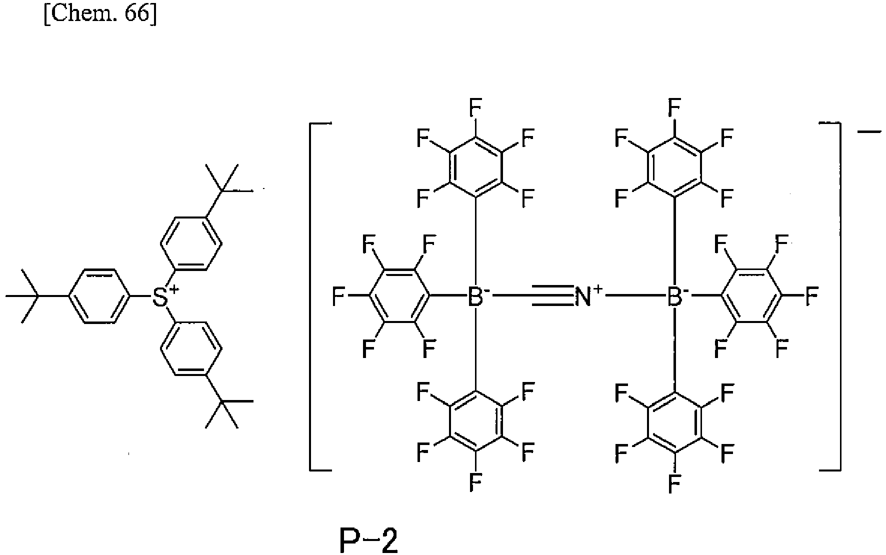

In a 2,000 mL four neck flask, 5.7 g of tri(4-t-butylphenylsulfonium)trifluoromethane sulfonate, 12.7 g of an intermediate (Q-2), 236.0 g of ion exchange water, and 704 mL of diethyl ether were charged, and reacted at 21 to 22°C for 16 hours. After the reaction, the content of the flask was transferred to a 1,000 mL separatory funnel and an aqueous layer was separated. Then, an ether layer was cleaned three times with 354.0 g of ion exchange water. Anhydrous sodium sulfate was added to the ether layer, moisture was removed, then filtered, the filtrate was concentrated under normal pressure at 50°C, and then dried under reduced pressure at 40°C for 18 hours, thereby obtaining a target represented by formula (P-2) which is 13.4 g of a white solid. The obtained target (P-2) was identified by ¹H-NMR.
¹H-NMR (300 MHz, DMSO-D6): δ 7.79 (12H, m), 1.32 (27H, s)

### [2] Preparation of charge-transporting varnish

### [Example 1-1]

To a mixture of 160 mg of T-1 represented by the following formula which was synthesized by the method described in Preparation Example 12 of WO 2015/050253 and 149 mg of P-3 obtained in Synthesis Example 1, 8 g of 3-phenoxytoluene and 2 g of 4-methoxytoluene were added, and the mixture was stirred and dissolved under ultrasonic irradiation at room temperature, and the resulting solution was filtered through a syringe filter having a pore size of 0.2 µm to obtain a charge-transporting varnish.

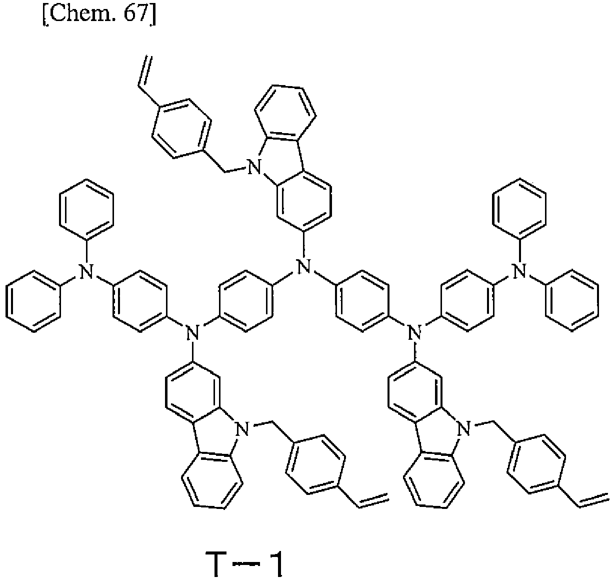

### [Example 1-2]

2.00 g of S-poly(3-MEET) (sulfonated poly(3-MEET)) which is a charge-transporting material synthesized according to the method described in Example 1 of US Patent No. 8,017,241 was dissolved in 100 mL of 28% ammonia water (manufactured by Junsei Chemical Co., Ltd.), and stirred at room temperature overnight. The reaction solution was reprecipitated in 1,500 mL of acetone and the precipitate was collected by filtration. The obtained precipitate was dissolved again in 20 mL of water and 7.59 g of triethylamine (manufactured by Tokyo Chemical Industry Co., Ltd.), and stirred at 60°C for 1 hour. After the reaction solution was cooled, the solution was reprecipitated in a mixed solvent of 1,000 mL of isopropyl alcohol and 500 mL of acetone, and the precipitate was collected by filtration. The obtained precipitate was dried under vacuum at 0 mmHg, 50°C for 1 hour, and treated with ammonia water to obtain 1.30 g of S-poly(3-MEET)-A.

0.125 g of the obtained S-poly(3-MEET)-A was dissolved in 2.28 g of ethylene glycol (manufactured by Kanto Chemical Co., Ltd.), 2.28 g of diethylene glycol (manufactured by Kanto Chemical Co., Ltd.), and 0.20 g of butylamine (manufactured by Tokyo Chemical Industry Co., Ltd.), and the mixture was stirred at 80°C for 1 hour using a hot stirrer. Then, 1.25 g of triethylene glycol dimethyl ether (manufactured by Tokyo Chemical Industry Co., Ltd.) was added thereto, and was stirred at 400 rpm, 80°C for 1 hour using a hot stirrer. Finally, 0.125 g of P-3 was added, stirred at 400 rpm, 40°C for 10 minutes using a hot stirrer, and the resulting solution was filtered through a PP syringe filter having a pore size of 0.2 µm to obtain 4% by weight of a charge-transporting varnish.

### [Comparative Example 1-1]

To a mixture of 0.208 g of T-2 represented by the following formula synthesized according to the method described in Example 4 and Examples 6 to 8 of JP 5839203 and 0.411 g of P-4 obtained in Comparative Synthesis Example 1,6.6 g of 1,3-Dimethyl-2-imidazolidinone (DMI), 8.0 g of (2,3-BD), and 5.4 g of dipropylene glycol monomethyl ether (DPM) were added, the mixture was stirred and dissolved at room temperature, and to the resulting solution, 0.021 g of trimethoxy(3,3,3-trifluoropropyl)silane and 0.041 g of trimethoxyphenylsilane were added, and filtered through a syringe filter having a pore size of 0.2 µm to obtain a charge-transporting varnish.

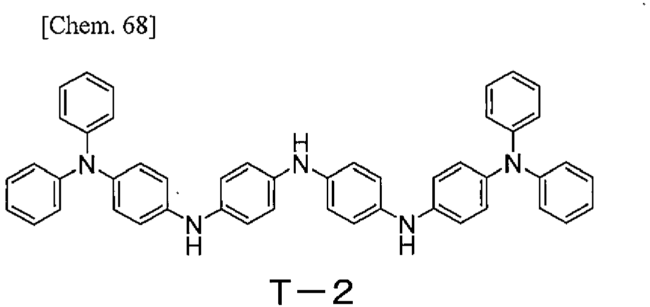

### [Comparative Example 1-2]

To a mixture of 158 mg of T-1 represented by the above formula and 105 mg of P-1 obtained in Comparative Synthesis Example 2, 4 g of 3-phenoxytoluene and 1 g of 4-methoxytoluene were added, and the mixture was stirred and dissolved under ultrasonic irradiation at room temperature, and the resulting solution was filtered through a syringe filter having a pore size of 0.2 µm to obtain a charge-transporting varnish.

### [Comparative Example 1-3]

To a mixture of 134 mg of T-1 represented by the above formula and 129 mg of P-2 obtained in Comparative Synthesis Example 3, 4 g of 3-phenoxytoluene and 1 g of 4-methoxytoluene were added, and the mixture was stirred and dissolved under ultrasonic irradiation at room temperature, and the resulting solution was filtered through a syringe filter having a pore size of 0.2 µm to obtain a charge-transporting varnish.

### [3] Manufacture and characteristic evaluation of organic EL device

### [Example 2-1]

The varnish obtained in Example 1-1 was applied to an ITO substrate using a spin coater, and then dried at 80°C for 1 minute under the air atmosphere. Then, the dried ITO substrate was inserted into a glove box and baked at 230°C for 30 minutes under a nitrogen atmosphere to form a thin film of 50 nm on the ITO substrate. As the ITO substrate, a glass substrate of 25 mm × 25 mm × 0.7 t in which indium tin oxide (ITO) is patterned with a thickness of 150 nm on the surface was used, and impurities on the surface were removed by an O₂ plasma cleaning device (150 W, for 30 seconds) before use.

Then, a deposition device (vacuum degree of 1.0×10⁻⁵ Pa) was used to form a film of α-NPD (N,N'-di(1-naphthyl)-N,N'-diphenylbenzidine) with 30 nm on the ITO substrate on which a thin film was formed, at 0.2 nm/sec. Then, a 10 nm thick film of an electron block material HTEB-01 manufactured by Kanto Chemical Co., Ltd. was formed. Then, a light-emitting layer host material NS60 manufactured by Nippon Steel Chemical & Material Co., Ltd. and a light-emitting layer dopant material Ir(PPy)₃ was co-deposited. In the co-deposition, a deposition rate was controlled so that the concentration of Ir(PPy)₃ was 6% and lamination was performed to 40 nm. Then, Alq3 lithium fluoride and an aluminum thin film were sequentially layered to obtain an organic EL device. At this time, the deposition rate was 0.2 nm/sec for Alq₃ and aluminum, and 0.02 nm/sec for lithium fluoride, and the film thicknesses were 20 nm, 0.5 nm, and 80 nm, respectively.

In addition, in order to prevent the characteristic degradation by the influence of oxygen in the air, water, and the like, after the organic EL device was sealed with a sealing substrate, the characteristics were evaluated. Sealing was performed in the following order. The organic EL device was stored between the sealing substrates in the nitrogen atmosphere at an oxygen concentration of 2 ppm or less and a dew point of -76°C or less, and the sealing substrate was bonded together by an adhesive (manufactured by MORESCO Corporation Mores Moisture Cut WB90US(P)). At this time, a water-collecting agent (manufactured by Dynic Corporation, HD-071010W-40) was stored in a sealing substrate with the organic EL device. After the bonded sealing substrate was irradiated with UV light (wavelength: 365 nm, irradiation amount: 6,000 mJ/cm²), the sealing substrate was subjected to annealing treatment at 80°C for 1 hour to cure the adhesive.

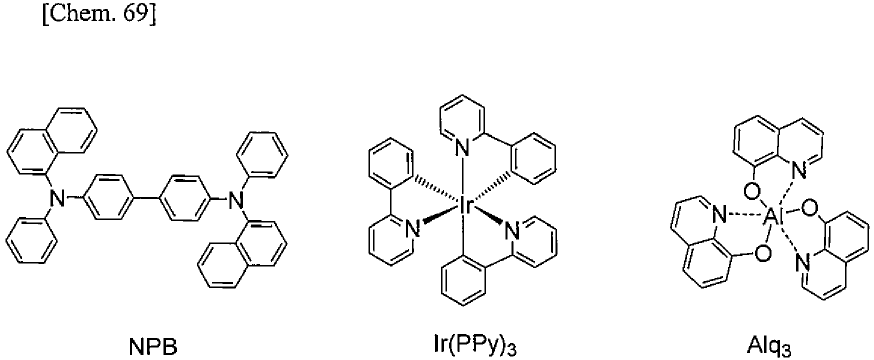

### [Example 2-2]

Each layer was formed in the same manner as in Example 2-1, except that the varnish obtained in Example 1-2 was used instead of the varnish obtained in Example 1-1 and applied on the ITO substrate using a spin coater, and then dried in a vacuum drier for 15 minutes, and an organic EL device was manufactured.

### [Comparative Examples 2-1 to 2-3]

Each layer was formed in the same manner as in Example 2-1, except that the varnish obtained in Comparative Examples 1-1 to 1-3 was used instead of the varnish obtained in Example 1-1, and an organic EL device was manufactured.

In Comparative Example 2-3, a film was not able to be formed using the varnish obtained in Comparative Example 1-3, and the device was not able to be obtained.

The characteristics of the devices of Examples 2-1 and 2-2, and Comparative Examples 2-1 and 2-2 were evaluated. A driving voltage, a current density, current efficiency, luminous efficiency, and external luminescence quantum efficiency (EQE) when the device emitted light at 10,000 cd/m² are shown in Table 19. A half-life of brightness of the device (initial brightness of 10,000 cd/m²) is shown in Table 20.

**[Table 19]**

| | Driving voltage (V) | Current density (mA/cm²) | Current efficiency (cd/A) | Luminous efficiency (lm/W) | EQE (%) |
|---|---|---|---|---|---|
| Example 2-1 | 5.7 | 9.7 | 51.4 | 28.4 | 14.3 |
| Example 2-2 | 6.2 | 23.5 | 42.6 | 21,5 | 12.2 |
| Comparative Example 2-1 | 5.6 | 10.8 | 46.2 | 26.0 | 13.0 |
| Comparative Example 2-2 | 5.7 | 9.8 | 51.0 | 28.3 | 14.3 |

**[Table 20]**

| | Half-life (hour) |
|---|---|
| Example 2-1 | 505 |
| Example 2-2 | 142 |
| Comparative Example 2-1 | 432 |
| Comparative Example 2-2 | 469 |

As shown in Tables 19 and 20, the EL device provided with the charge-transporting thin film of the present invention was suitably driven. Further, the life characteristics were also excellent.

## Claims

1. A charge-transporting varnish comprising: a charge-transporting material, an onium borate salt, and an organic solvent, wherein
the onium borate salt includes an onium borate salt including a monovalent or divalent anion represented by formula (a1) and a counter cation represented by formulae (c1) to (c5) (provided that the salt is an electrically neutral salt): 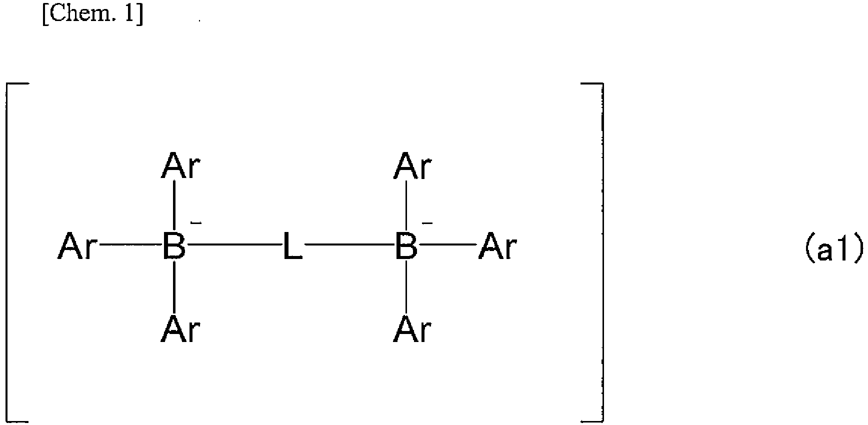 wherein Ar's, independently of each other, represent an aryl group which may have a substituent or a heteroaryl group which may have a substituent, and L represents an alkylene group, -NH-, an oxygen atom, a sulfur atom, or -CN⁺-,

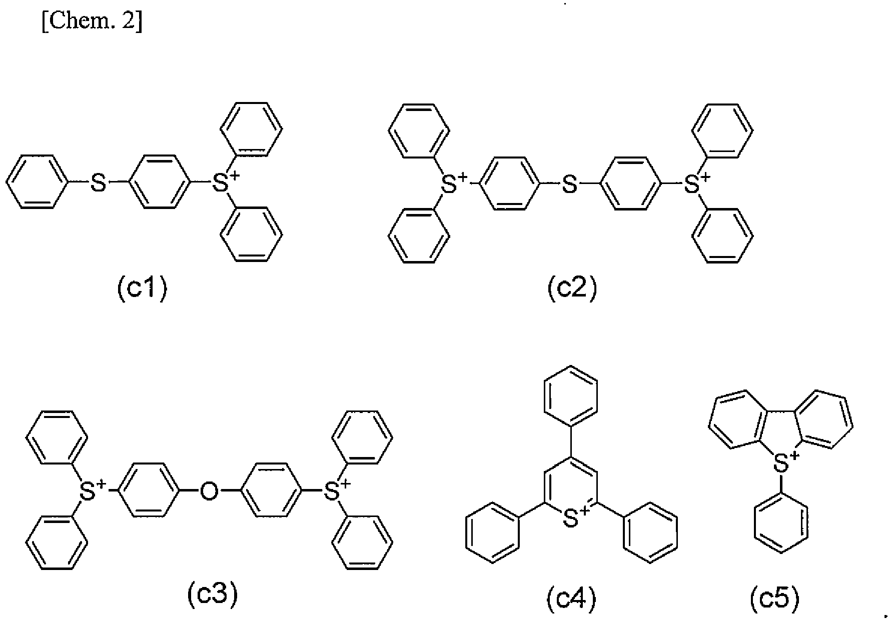

2. The charge-transporting varnish of claim 1, wherein Ar is an aryl group having one or two or more electron-withdrawing substituents.

3. The charge-transporting varnish of claim 2, wherein the electron-withdrawing substituent is a halogen atom.

4. The charge-transporting varnish of any one of claims 1 to 3, wherein the anion is represented by formula (a2):

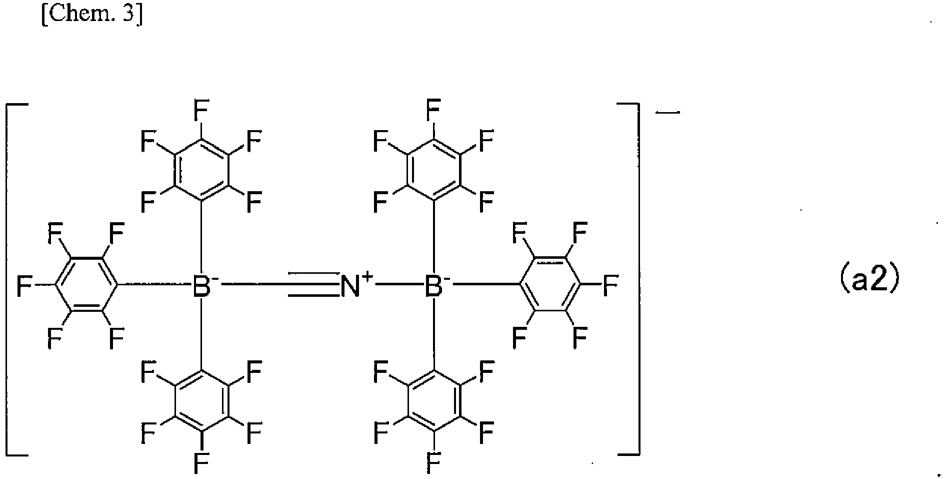

5. The charge-transporting varnish of claim 4, wherein the onium borate salt is represented by the following formula:

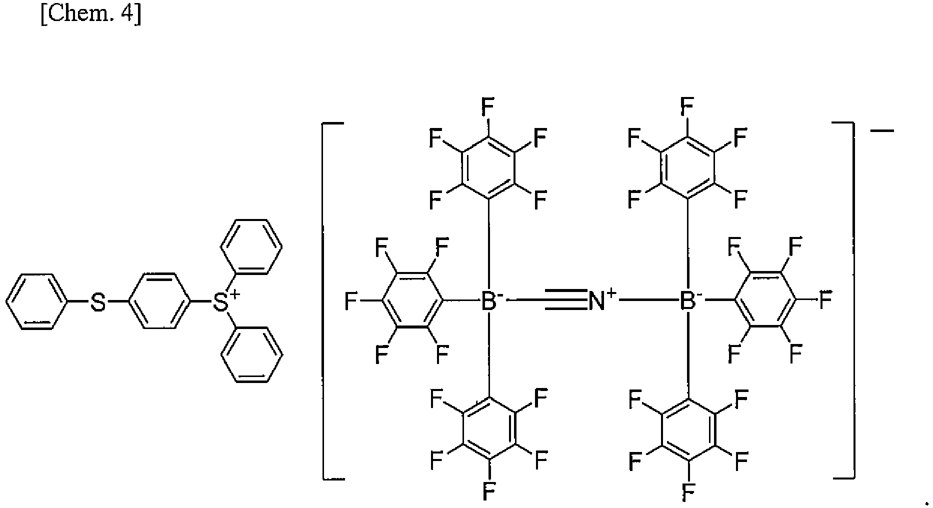

6. The charge-transporting varnish of any one of claims 1 to 5, wherein the charge-transporting material is at least one selected from an aniline derivative and a thiophene derivative.

7. The charge-transporting varnish of claim 6, wherein the charge-transporting material is the aniline derivative.

8. A charge-transporting thin film, obtained from the charge-transporting varnish of any one of claims 1 to 7.

9. An organic electroluminescent device, comprising the charge-transporting thin film of claim 8.

10. A method of producing a charge-transporting thin film, the method comprising applying the charge-transporting varnish of any one of claims 1 to 7 on a substrate, and evaporating a solvent.

11. An onium borate salt, included in any one of a hole injection layer, a hole transport layer, and a hole injection/transport layer in an organic electroluminescent device, the onium borate salt comprising a monovalent or divalent anion represented by formula (a1) and a counter cation represented by formulae (c1) to (c5) (provided that the salt is an electrically neutral salt): 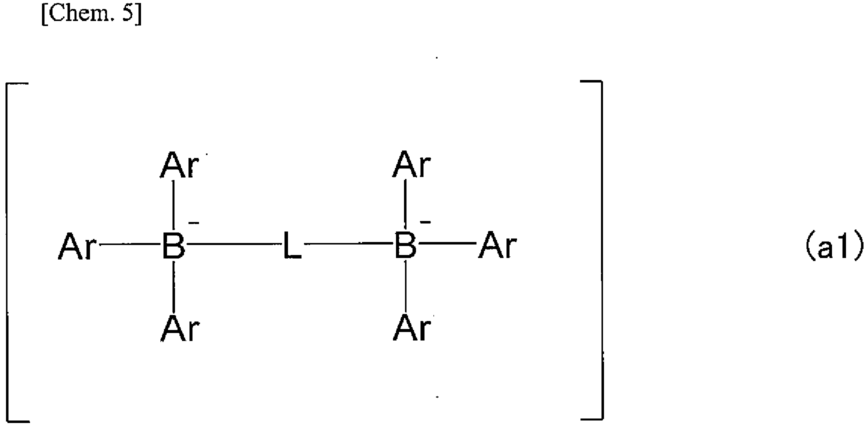 wherein Ar's, independently of each other, represent an aryl group which may have a substituent or a heteroaryl group which may have a substituent, and L represents an alkylene group, -NH-, an oxygen atom, a sulfur atom, or -CN⁺-,

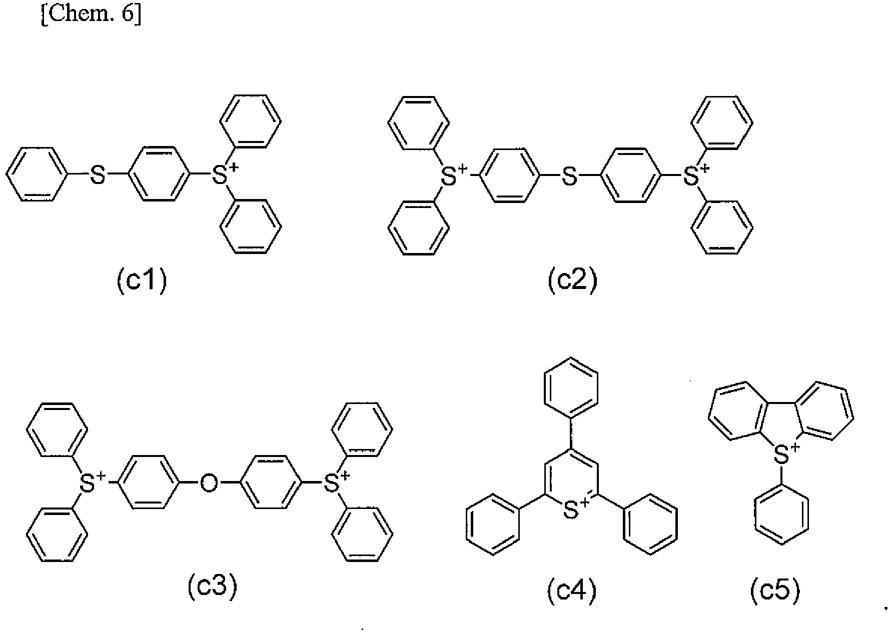

12. The onium borate salt of claim 11, wherein Ar is an aryl group having one or two or more electron-withdrawing substituents.

13. The onium borate salt of claim 12, wherein the electron-withdrawing substituent is a halogen atom.

14. The onium borate salt of any one of claims 11 to 13, wherein the anion is represented by formula (a2):

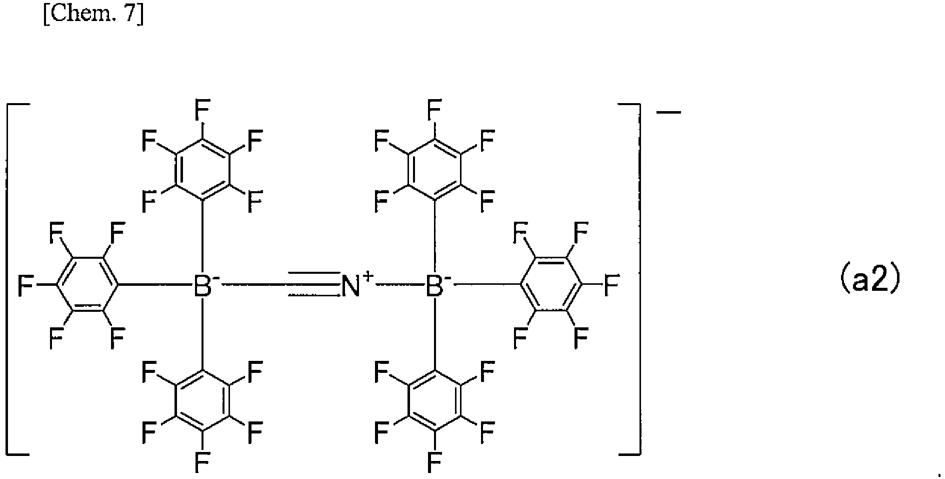

15. The onium borate salt of claim 14, wherein the onium borate salt is represented by the following formula:
